# FASCICULE DE BREVET EUROPEEN

(11) **EP 3 475 697 B1**
(45) Date de publication et mention de la délivrance du brevet: **14.10.2020**
(21) Numéro de dépôt: 17732887.9
(22) Date de dépôt: 23.06.2017
(51) Int. Cl.: G01N 33/50, C12Q 1/6883

(54) **MODÈLES DE LA DERMATITE ATOPIQUE JUVÉNILE**
MODELLE FÜR JUVENILE ATOPISCHE DERMATITIS
JUVENILE ATOPIC DERMATITIS MODELS

(30) Priorité: 23.06.2016 FR 1655850
(43) Date de publication de la demande: 01.05.2019
(73) Titulaire: Laboratoires Expanscience, 92048 Paris La Défense Cedex (FR)
(72) Inventeur: BAUDOUIN, Caroline, 78120 Rambouillet (FR); BREDIF, Stéphanie, 28210 Croisilles (FR)
(74) Mandataire: Regimbeau
(86) Numéro de dépôt international: PCT/EP2017/065497
(87) Numéro de publication internationale: WO 2017/220763

(56) Documents cités:
- WO-A1-2014/009566
- N. CASTEX-RIZZI ET AL: "In vitro approaches to pharmacological screening in the field of atopic dermatitis", BRITISH JOURNAL OF DERMATOLOGY, vol. 170, 16 juillet 2014 (2014-07-16), pages 12-18, XP055352641, UK ISSN: 0007-0963, DOI: 10.1111/bjd.13106
- M.A. BACHELOR ET AL: "Induction of an atopic dermatitis-like phenotype in a full-thickness in vitro human skin model", JOURNAL OF INVESTIGATIVE DERMATOLOGY, vol. 136, no. 5, 1 mai 2016 (2016-05-01), page S62, XP055353542, US ISSN: 0022-202X, DOI: 10.1016/j.jid.2016.02.388
- ILNYTSKA OLHA ET AL: "Colloidal Oatmeal (Avena Sativa) Improves Skin Barrier Through Multi-Therapy Activity", JOURNAL OF DRUGS IN DERMATOLOGY, STRATEGIC COMMUNICATION IN DERMATOLOGY, NEW YORK, NY, US, [Online] vol. 15, no. 6, 1 juin 2016 (2016-06-01), pages 684-690, XP008183625, ISSN: 1545-9616
- MATHES STEPHANIE H ET AL: "The use of skin models in drug development", ADVANCED DRUG DELIVERY REVIEWS, vol. 69, 27 décembre 2013 (2013-12-27), pages 81-102, XP028648699, ISSN: 0169-409X, DOI: 10.1016/J.ADDR.2013.12.006
- S. BREDIF ET AL: "In vitro models of infant skin to study molecular mechanisms involved in pediatric atopic dermatitis", JOURNAL OF INVESTIGATIVE DERMATOLOGY, vol. 136, no. 9, 7 septembre 2016 (2016-09-07), page S222, XP055353538, US ISSN: 0022-202X, DOI: 10.1016/j.jid.2016.06.378
- S. BREDIF ET AL: "Studying the role of S. aureus and its biofilm in atopic dermatitis pathogenesis using in vitro 3D models", JOURNAL OF INVESTIGATIVE DERMATOLOGY, vol. 136, no. 9, 7 septembre 2016 (2016-09-07), page S220, XP055353537, US ISSN: 0022-202X, DOI: 10.1016/j.jid.2016.06.368

## Description

### INTRODUCTION

La peau est un ensemble de cellules et de macromolécules regroupées sous forme d'un tissu résistant et souple, recouvrant la totalité du corps. Elle est formée de deux couches jointes : l'épiderme et le derme auxquelles on peut associer les tissus sous-cutanés.

La peau a pour fonction principale d'établir une barrière de protection contre les atteintes de l'environnement tout en permettant certains échanges entre le milieu intérieur et le milieu extérieur. La fonction de barrière est notamment particulièrement importante pour limiter les pertes d'eau de l'épiderme. Cette fonction est assurée avant tout par la couche cornée (*stratum corneum*), couche la plus superficielle de l'épiderme, composée de cellules aplaties et sans noyau, les cornéocytes. L'étanchéité de ce « mur de briques » est assuré par un ciment intercellulaire composé de lipides spécifiques (cholestérol, cholestérol sulfate, acides gras libres et céramides). La capacité régénérative de l'épiderme est conférée par des cellules souches adultes qui permettent le remplacement régulier des cellules différenciées éliminées lors de la kératinisation. Ce processus est en particulier crucial pour la maturation et le maintien de la fonction barrière.

L'adaptation à la vie extra-utérine est un processus qui commence dès la naissance et se poursuit tout au long de la première année de la vie. Les premiers mois de la vie postnatale constituent une période de réorganisation structurelle et fonctionnelle de la peau qui permet une adaptation physiologique à l'environnement extra-utérin. Par exemple, l'immaturité de la peau du nouveau-né est soulignée par la différence de structure et de composition moléculaire du *stratum corneum* par rapport à l'adulte. Celles-ci sont incomplètes et continuent ainsi de se développer durant les 12 premiers mois au moins après la naissance (Chiou et al., Skin Pharmacol Physiol, 17: 57-66, 2004; Nikolovski et al., J Invest Dermatol, 128: 1728-1736, 2008; Stamatas et al., Pediatr Dermatol, 27: 125-131, 2010; Telofski et al., Dermatol Res Pract, 2012 : 198789, 2012). Par ailleurs, les résultats de deux études cliniques récentes (Fluhr et al., Br J Dermatol, 166(3) : 483-90, 2012 et Fluhr et al., Br J Dermatol, 2014, 171(5) : 978-86) suggèrent que la peau des nourrissons présente une certaine immaturité quant à sa capacité à capter l'eau et à réguler les mécanismes qui y sont liés. De plus ces travaux ont montré que la barrière épidermique s'organise structurellement depuis la naissance jusqu'à l'âge de 2 ans et n'est donc pas complètement compétente pendant cette période. Ceci contribue à expliquer la fragilité de la peau des nourrissons et jeunes enfants et la susceptibilité de celle-ci aux agressions chimiques, physiques et microbiennes.

De plus, une maturation incomplète de la peau peut avoir des conséquences cliniques importantes. Il est donc important de permettre à la peau de se construire et de se développer correctement et harmonieusement, sans quoi son organisation fonctionnelle et structurelle pourrait en être obérée. À cet égard, il est crucial de préserver la fonction barrière et la capacité de renouvellement de l'épiderme.

Ainsi, l'immaturité de la barrière et des mécanismes de régulation de l'hydratation dans la peau du bébé contribue à rendre celle-ci encore plus vulnérable à des situations pathologiques telles que la dermatite atopique.

La dermatite atopique est une des maladies chroniques les plus répandues dans la population. Elle se caractérise par un ensemble de signes cliniques, dont les plus importants sont un prurit et des lésions eczémateuses qui peuvent être aiguës, subaiguës ou chroniques. Elle débute presque toujours chez le nourrisson ou l'enfant en bas âge, alors que la barrière est en train de s'organiser structurellement et fonctionnellement. La dermatite atopique commence généralement vers trois mois, mais parfois dès les premières semaines de la vie. Elle évolue en alternant poussées et phases de rémission. Selon les enfants et sa gravité, elle peut durer de plusieurs mois à plusieurs années. Un petit pourcentage d'entre elles peuvent persister à l'âge adulte.

La dermatite atopique est avant tout une maladie inflammatoire chronique dermatologique associant altération de la barrière cutanée et inflammation cutanée. Dans une première phase de sensibilisation, le déficit de la barrière cutanée favorise la pénétration des allergènes à travers la peau. Les allergènes qui pénètrent les couches superficielles de l'épiderme sont pris en charge (internalisés) par les cellules de Langerhans épidermiques et les cellules dendritiques dermiques. Les cellules de Langerhans sont des cellules présentatrices d'antigènes qui sont capables de capter des antigènes cutanés, de les apprêter et de les présenter aux lymphocytes T. Cette présentation entraine l'activation de la réponse Th2, ce qui aboutit à la production de cytokines inflammatoires comme IL-4, IL-5 ou IL-13 (voir par exemple Bieber, Ann Dermatol. 2010, 22(2) : 125-137).

Une fois l'individu sensibilisé par voie cutanée, les contacts ultérieurs avec l'allergène en cause peuvent induire des lésions d'eczéma. Cette réponse est aussi médiée par la réponse Th2. Les cellules de Langerhans notamment présentent les peptides aux lymphocytes T spécifiques qui, activés, produisent des cytokines de type Th2 (IL-4, IL-5). Les cytokines produites vont recruter de nouvelles cellules dont les éosinophiles qui jouent un rôle important dans la mise en place et la chronicité des lésions d'eczéma.

Dans toutes les périodes d'activité de la maladie, les surinfections cutanées bactériennes ou virales sont les complications les plus communes. La peau des patients de la dermatite atopique est hautement susceptible aux infections secondaires, lesquelles tendent ensuite à se généraliser. La bactérie *Staphylococcus aureus* est ainsi une cause majeure d'infections cutanées. Elle colonise habituellement la peau du patient atteint de dermatite atopique, alors qu'elle n'est présente que de façon transitoire sur la peau saine. La bactérie sécrète alors des facteurs de virulence qui réduisent encore la fonction barrière, ce qui exacerbe la maladie et contribue à la rendre chronique. Par ailleurs, *S. aureus* est habituellement retrouvé chez les patients atteints de dermatite atopique sous forme de biofilms homogènes, une forme résistante aux défenses de l'hôte et aux traitements.

A ce jour, il n'existe pas de cure de la dermatite atopique. Les traitements se fondent avant tout sur des soins locaux, dont le but est d'améliorer les symptômes et de contrôler l'évolution de la maladie (Eichenfield et al., J Am Acad Dermatol. 2014 ; 70(2): 338-351 ; Eichenfield et al., J Am Acad Dermatol. 2014; 71(1): 116-132). En particulier, l'utilisation quotidienne d'émollients est essentielle pour restaurer et protéger la barrière cutanée altérée. De nombreux émollients différents sont disponibles sur le marché. Cependant, les mécanismes précis par lesquels ils exercent leurs effets bénéfiques sont insuffisamment compris. Or la dermatite atopique se présente sous un grand nombre d'aspects. Il n'existe a priori de raison d'estimer qu'un émollient sera capable de restaurer la barrière dans chacune de ses situations pathologiques particulières. A ce sujet, il est important de noter que les données expérimentales permettant de sélectionner des préparations spécifiques sont rares. De fait, il existe très peu d'études comparatives de leur efficacité relative. N. Castex-Rizzi et al. décrivent des modèles *in vitro* de dermatite atopique pour le criblage pharmacologique (Castex-Rizzi et al., BJD. 2014; 170 (Suppl. s1): 12-18).

Il existe donc toujours un besoin de disposer de tests appropriés permettant de sélectionner des émollients efficaces et bien tolérés afin de traiter tous les aspects de la dermatite atopique.

### DESCRIPTION

La dermatite atopique est une pathologie affectant surtout les enfants et, notamment, les nourrissons. Elle est caractérisée principalement par une interaction entre une défectuosité de la barrière et une réaction inflammatoire. Elle se manifeste sous des aspects variés et nombreux dont il n'est généralement pas tenu compte lors de la mise au point de nouveaux actifs ou de nouveaux émollients. La physiopathologie de la dermatite atopique est complexe et met en jeu de multiples paramètres (fonction barrière, réponse immune, inflammation, microbiote...) ; il est difficile de reproduire in vitro, de façon simultanée, l'ensemble de ces facteurs. De fait, les modèles in vitro existants de la dermatite atopique sont limités par le fait qu'ils ne correspondent pas à l'image globale de la maladie mais représentent seulement une partie des facteurs impliqués dans la pathogénèse.

Les inventeurs ont développé des procédés d'évaluation de l'efficacité *in vitro* d'actifs cosmétiques, d'émollients et de formulations sur la prévention et le traitement de tous les aspects de la dermatite atopique affectant la peau d'enfant. De tels procédés n'avaient jamais été décrits jusqu'ici. Des peaux reconstruites spécifiquement capables de reproduire les caractéristiques de la peau des enfants, et en particulier celle des enfants très jeunes comme les nourrissons, ont été utilisées pour reproduire toutes les phases de la dermatite atopique. L'utilisation de cette variété de modèles, correspondant aux différentes phases de la pathologie, permet de sélectionner les actifs cosmétiques, les émollients et les formulations testées réellement efficaces contre la dermatite atopique. Les actifs cosmétiques, les émollients et formulations ainsi sélectionnés sont notamment efficaces sur l'ensemble des paramètres majeurs de la physiopathologie de la dermatite atopique (la fonction barrière, l'immunité, l'inflammation, le microbiote). L'invention permet ainsi de déterminer de façon précise quels actifs cosmétiques et quels émollients ont un effet avantageux sur la prévention ou le traitement des effets de la dermatite atopique. Les méthodes de l'invention sont aussi adaptées à l'évaluation de l'activité de formulations. Les inventeurs ont ainsi pu montrer que certaines formulations étaient plus efficaces que d'autres pour prévenir et/ou limiter les effets de la dermatite atopique, démontrant ainsi l'utilité de l'approche entreprise.

L'invention a donc pour objet une méthode d'évaluation de l'efficacité d'un actif cosmétique, d'un émollient ou d'une formulation pour traiter et/ou prévenir la dermatite atopique, comprenant l'évaluation de le l'efficacité dudit actif, dudit émollient ou de ladite formulation dans plusieurs modèles reproduisant chacun une phase spécifique ou une caractéristique de la physiopathologie de la dermatite atopique, telle que définie dans les revendications. Par « peau atopique », on entend ici une peau de patient souffrant d'une dermatite atopique ou une peau présentant les mêmes caractéristiques physiologiques et moléculaires qu'une peau de patient souffrant d'une dermatite atopique. Par « dermatite atopique » (ou eczéma constitutionnel), on entend ici une affection inflammatoire prurigineuse chronique commune chez l'enfant et l'adulte jeune, et caractérisée notamment par une atteinte épidermique prédominante avec un afflux de lymphocytes T (exocytose) et un oedème intercellulaire (spongiose) réalisant des vésicules microscopiques. La dermatite atopique est la manifestation cutanée de l'atopie, caractérisée par l'existence de manifestations d'hypersensibilité médiée par des IgE et par des lymphocytes T spécifiques.

La dermatite atopique est causée par une fragilisation de la barrière cutanée qui permet une réaction inflammatoire médiée par les cytokines Th2. Le terme « cytokine », tel qu'on l'entend ici, se rapporte à une famille de petites protéines sécrétées régulatrices ayant un rôle crucial dans les réponses immunitaires. Les cytokines sont impliquées dans la communication entre cellules et régulent de nombreuses fonctions cellulaires, comme par exemple la survie et la croissance des cellules, ainsi que l'induction de l'expression de nombreux gènes. Par « cytokines Th2 », on entend ici les cytokines que produisent les lymphocytes T CD4 Th2 (IL-4, IL-5, IL-10, IL-13, IL-22 et IL-31) ou qui sont produites par d'autres types cellulaires dans le même contexte signant une activation de la voie Th2 (TSLP et TNFα). Les cytokines peuvent être produites par de nombreux types cellulaires, notamment les phagocytes mononucléés résidents (macrophages et cellules dendritiques) et les mastocytes. Pour une revue des cytokines Th2 et de leur rôle dans la dermatite atopique, on pourra se référer par exemple à Brandt et Sivaprasad (J Clin Cell Immunol. 2011 ; 2(3) : 110).

Les présents inventeurs ont développé des modèles permettant d'étudier les différentes phases de la maladie et donc de sélectionner des actifs cosmétiques ou des émollients capables de restaurer la barrière cutanée et moduler les processus inflammatoires impliqués dans la pathologie dans chacune de ces phases. Les inventeurs ont ainsi mis au point des modèles de peau reconstruite à partir de d'échantillons provenant d'enfant et ont pu tester l'effet de la dermatite atopique sur ces modèles. Alors que la dermatite atopique dans les modèles de l'art antérieur était surtout liée à l'absence de la filaggrine (Mildner et al., J Invest Dermatol. 2010 ; 130(9) : 2286-2294 ; Pendaries et al., J Invest Dermatol. 2014; 134(12) : 2938-2946), les présents inventeurs ont utilisé plusieurs conditions différentes afin de reproduire *in vitro* les différentes étapes de la pathologie et les caractériser moléculairement.

Tout d'abord, les modèles de peau reconstruite utilisés ont été obtenus à partir d'échantillon de peau d'enfant. Contrairement à la peau d'adulte, la peau d'enfant n'est pas mature, tandis que l'organisation structurelle et fonctionnelle de la barrière n'est pas complètement compétente. Or la dermatite atopique affecte en priorité les enfants. D'autre part, la phase d'initiation de la dermatite atopique a été reproduite par l'addition sur des épidermes d'enfants reconstruits stimulés par un mélange de poly (I:C) et de cytokine IL1α (l'interleukine IL1α humaine possède une séquence protéique représentée par la séquence de référence NCBI : NP_000566, et est codée par le gène IL1A humain (référence NCBI : Gene ID: 3552), dont la séquence correspond à la référence NCBI : NM_000575.). Le stress induit par ce mélange de molécules aboutit à l'induction d'une réponse inflammatoire de type Th2 typique de l'initiation d'une réponse inflammatoire caractéristique de la dermatite atopique. Par ailleurs, la réponse inflammatoire établie a été reproduite dans un autre modèle par l'ajout d'un cocktail de cytokines Th2 aux épidermes d'enfants reconstruits. En effet, quand au moins deux cytokines choisies dans le groupe constitué par IL-4, IL-5, IL-10, IL-13, IL-22, IL-31, TSLP1 et TNFα, et préférentiellement quand un cocktail constitué de IL-4, IL-13, IL-22 et TNFα, sont mises en contact avec une peau d'enfant reconstruite, le modèle obtenu possède les mêmes caractéristiques physiologiques que les peaux atopiques *in vivo.* Notamment, ce modèle présente des profils d'expression de marqueurs spécifiques semblables à ceux d'une peau atopique.

Enfin, deux autres modèles ont été mis au point pour mimer les complications des dermatites atopiques, notamment les infections bactériennes.

Par « infection bactérienne », on entend ici l'établissement, la prolifération et le maintien de bactéries pathogènes à la surface de la peau, notamment sur une peau atopique. Une « bactérie pathogène » au sens de la présente demande est une bactérie qui peut causer une maladie chez un hôte, plus préférentiellement une maladie cutanée. Une bactérie pathogène peut être une bactérie pathogène opportuniste ou une bactérie pathogène stricte. Une « bactérie pathogène opportuniste » est une bactérie qui ne cause habituellement pas de maladie chez les sujets sains, mais qui peut devenir pathogène chez les sujets aux défenses altérées. Ces bactéries sont souvent des bactéries commensales qui vivent à la surface de la peau et des muqueuses de l'homme. Une « bactérie pathogène stricte » est une bactérie qui est pathogène quel que soit l'hôte. Les bactéries pathogènes responsables des infections cutanées les plus communes sont des staphylocoques tels que *Staphylococcus aureus* ou *Staphylococcus epidermidis,* des streptocoques, notamment *Streptococcus pyagenes* et *Streptococcus agalactiae,* des corynébactéries ou encore des bactéries propioniques. Préférentiellement, une bactérie pathogène est un staphylocoque ou un streptocoque ; plus préférentiellement, une bactérie pathogène est choisie parmi *S. aureus* et *S. pyagenes* ; encore plus préférentiellement, la bactérie pathogène est *S. aureus.*

Dans le premier modèle d'infection bactérienne de la peau atopique d'enfant, le modèle de peau reconstituée est cultivé en présence de monocytes THP-1. Cette lignée monocytaire humaine, qui est disponible notamment à l'American Type Culture Collection (ATCC, 10801 University Boulevard, Manassas, VA 20110 USA), est bien connue de l'homme du métier pour ses capacités à se différencier en macrophages et à synthétiser des cytokines. Le modèle de peau reconstituée cultivé en présence de monocytes THP-1 représente donc un modèle de réponse immuno-médiée particulièrement réaliste, qui permet d'étudier l'infection par une bactérie pathogène dans des conditions très proches de celles existant *in vivo.* Le second modèle permet de suivre l'évolution d'une population de bactéries pathogènes quand la colonisation de la peau atopique est déjà établie.

Les inventeurs ont pu observer que l'expression de certains marqueurs biologiques était altérée quand ces modèles de peau reconstruite d'enfant étaient cultivés dans des conditions variées de dermatite atopique. Certains marqueurs, comme les marqueurs de l'inflammation étaient ainsi plus fortement exprimés, tandis que l'expression d'autres, tels que les marqueurs des cellules souches ou ceux renforçant la fonction barrière était diminuée. En revanche, les variations d'expression de ces marqueurs étaient réduites, voire estompées, quand les modèles étaient traités avec des actifs cosmétiques, des émollients ou des formulations connues pour traiter ou prévenir la dermatite atopique. Ce résultat souligne la pertinence physiologique de ces marqueurs. L'importance de l'utilisation de modèles de peau reconstruite d'enfants et non pas d'adultes pour isoler de tels marqueurs en est d'ailleurs renforcée.

Ainsi, les présents inventeurs ont pu identifier des marqueurs biologiques dont l'expression est modifiée par la dermatite atopique dans la peau d'enfant. De tels marqueurs sont particulièrement avantageux parce qu'ils permettent de suivre la réponse de la peau durant le développement de la maladie.

Selon un premier aspect, ce document a pour objet une méthode d'évaluation de l'efficacité *in vitro* d'un actif cosmétique, d'un émollient ou d'une formulation pour prévenir ou traiter les effets de la dermatite atopique affectant la peau d'enfant, ladite méthode comprenant la détermination du niveau d'expression et/ou d'activation d'au moins un marqueur biologique.

Plus précisément, l'invention a pour objet une méthode d'évaluation de l'efficacité *in vitro* d'un actif cosmétique, d'un émollient ou d'une formulation pour prévenir ou traiter les effets de la dermatite atopique affectant la peau d'enfant, ladite méthode comprenant la détermination de l'efficacité dudit actif cosmétique, dudit émollient ou de ladite formulation dans chacun des quatre tests A, B, C et D, ladite méthode étant caractérisée en ce que :
- le test A comprend les étapes suivantes :
   a) obtenir un modèle de peau reconstruite à partir d'un échantillon cutané provenant d'un enfant ;
   b) mettre en contact ledit actif cosmétique, ledit émollient ou ladite formulation avec le modèle de peau reconstruite de l'étape a) ;
   c) mettre en contact le modèle de peau reconstruite de l'étape b) avec une solution comprenant de l'acide poly(déoxyinosinique-déoxycytidylique) (poly(dldC))et de l'interleukine 1 alpha (IL1α) ;
   d) mesurer le niveau d'expression d'au moins un marqueur biologique dans le modèle de peau de l'étape c) ; et
   e) évaluer l'efficacité dudit actif cosmétique, dudit émollient ou de ladite formulation en fonction du niveau de l'étape d) ;
- le test B comprend les étapes suivantes :
   a) obtenir un modèle de peau reconstruite à partir d'un échantillon cutané provenant d'un enfant ;
   b) mettre en contact ledit actif cosmétique, ledit émollient ou ladite formulation avec le modèle de peau reconstruite de l'étape a) ;
   c) mettre en contact le modèle de peau reconstruite de l'étape b) avec une solution comprenant au moins deux cytokines Th2 ;
   d) mesurer le niveau d'expression d'au moins un marqueur biologique dans le modèle de peau de l'étape c) ; et
   e) évaluer l'efficacité dudit actif cosmétique, dudit émollient ou de ladite formulation en fonction du niveau de l'étape d) ;
- le test C comprend les étapes suivantes :
   a) obtenir un modèle de peau reconstruite à partir d'un échantillon cutané provenant d'un enfant ;
   b) cultiver le modèle de peau reconstruite de l'étape a) en présence de monocytes THP-1 ;
   c) mettre en contact ledit actif cosmétique, ledit émollient ou ladite formulation avec le modèle de peau reconstruite de l'étape b) ;
   d) induire une altération de la fonction barrière dans le modèle de peau reconstruite de l'étape c) ;
   e) mettre en contact le modèle de peau reconstruite de l'étape d) avec au moins une bactérie pathogène ;
   f) mesurer le niveau d'expression et/ou d'activation d'au moins un marqueur biologique dans le modèle de peau de l'étape e) ; et
   g) évaluer l'efficacité dudit actif cosmétique, dudit émollient ou de ladite formulation en fonction du niveau de l'étape f) ; et
- le test D comprend les étapes suivantes :
   a) obtenir un modèle de peau reconstruite à partir d'un échantillon cutané provenant d'un enfant ;
   b) mettre en contact ledit actif cosmétique, ledit émollient ou ladite formulation avec le modèle de peau reconstruite de l'étape a) ;
   c) induire une altération de la fonction barrière dans le modèle de peau reconstruite de l'étape b) ;
   d) mettre en contact le modèle de peau reconstruite de l'étape c) avec au moins une bactérie pathogène ;
   e) mesurer le niveau d'expression et/ou d'activation d'au moins un marqueur biologique dans le modèle de peau de l'étape d) ; et
   f) évaluer l'efficacité dudit actif cosmétique, dudit émollient ou de ladite formulation en fonction du niveau de l'étape e),
dans laquelle ledit marqueur est choisi parmi :
- les marqueurs des activités inhibitrices de la physiologie bactérienne, dans lesquels l'activité inhibitrice de de la physiologie bactérienne est choisie parmi l'inhibition de la prolifération bactérienne et l'inhibition de la formation du biofilm ;
   les marqueurs de l'immunité, ledit marqueur de l'immunité étant HBD2 ou TLR2 ;
- les marqueurs de l'inflammation, ledit marqueur de l'inflammation étant CCL2, CXCL1, CCL7, IL6, IL18, CCL3, CCL5, CCL7, KLK5 ou TSLP ;
- les marqueurs de la fonction barrière, ledit marqueur de la fonction barrière étant FLG, KRT1, KRT10, SCEL, BARX2, LOR, IVL, TGM1 ,DSG1, CDSN, CLDN1, CASP14, SMPD1, GBA, LASS6, NMF ou les céramides ; et
- les marqueurs préférentiellement exprimés dans les cellules souches, ledit marqueur préférentiellement exprimé dans les cellules souches étant KRT15, KRT19, NOTCH1, BIRC5, ITGA6, ITGB1 ou ITGB4;
   et dans laquelle l'échantillon cutané provient d'un donneur choisi dans le groupe constitué des nouveau-nés, dont l'âge est compris entre 0 et 1 mois, des nourrissons, dont l'âge est compris entre 1 mois et 2 ans, et des enfants dont l'âge est compris entre 2 ans et 16 ans, et préférentiellement d'un donneur choisi dans le groupe constitué des nouveau-nés et des nourrissons.

Par « altération de la fonction barrière », on entend ici une altération de l'intégrité de la fonction barrière. La fonction barrière de la peau ou de la barrière cutanée (telles qu'entendues ici, les deux expressions sont synonymes) est intègre quand la barrière cutanée est pleinement fonctionnelle, c'est-à-dire que les échanges, et notamment la diffusion d'eau, sont limités. En revanche, une altération de la fonction barrière est une altération conduisant à une réduction de la fonction barrière du modèle de peau reconstruite utilisé et donc à une augmentation des échanges, et notamment la diffusion d'eau.

L'intégrité de la barrière cutanée peut être évaluée par la mesure d'un grand nombre de paramètres. En particulier, il est habituel de déterminer l'intégrité de la barrière cutanée par la mesure de la perte insensible en eau, aussi appelée perte en eau transépidermique. Les méthodes de mesure de la perte insensible en eau sont bien connues de l'homme de l'art et ne nécessitent pas d'être décrites ici en détail (voir par exemple H Tagami and K Kikuchi. « Diseases that affect barrier function ». In Elias PM and Feingold KR editors. Skin Barrier. New York : Taylor and Francis ; 2005. p. 447-468). Préférentiellement, pour mesurer la perte insensible en eau, on utilise un évaporimètre, qui est composé d'une sonde que l'on place à 3 ou 6 mm au-dessus de la peau.

L'intégrité de la barrière peut être affectée par un grand nombre de facteurs bien connus de l'homme du métier. Une altération de la barrière cutanée peut se produire suite à des agressions externes de type agents irritants (détergents, acides, bases, oxydants, réducteurs, solvants concentrés, gaz ou fumées toxiques), sollicitations mécaniques (frottements, chocs, abrasion, arrachement de la surface, projection de poussières, de particules, rasage ou épilation), déséquilibres thermiques ou climatiques (froid, sécheresse), xénobiotiques (micro-organismes indésirable, allergènes) ou à des agressions internes de type stress psychologique ou plus généralement au cours du vieillissement de la peau. Par exemple, une mutation inactivante dans le gène de la filaggrine conduit à une réduction de la fonction barrière et est une cause majeure de la dermatite atopique (voir par exemple, Peng & Novak, Clinical & Experimental Allergy, 2015, 45 : 566-574 ; Weidinger & Novak, Lancet. 2016, 387 : 1109-1122). De préférence, l'altération de la barrière cutanée résulte d'une sollicitation mécanique, comme, par exemple, d'une abrasion légère de la surface de la peau reconstituée.

Dans un premier mode de réalisation préféré, le modèle de peau reconstruite est mis en contact avec la solution de poly(dldC) et d'ILα dans l'étape c) du test A en présence de l'actif cosmétique, de l'émollient ou de la formulation. Selon un autre mode préféré de réalisation, l'actif cosmétique, l'émollient ou la formulation est éliminé préalablement à l'exposition dudit modèle de peau reconstruite à ladite solution lors de ladite étape c).

Selon un autre mode de réalisation préféré, le modèle de peau reconstruite est mis en contact avec les au moins deux cytokines Th2 dans l'étape c) du test B en présence de l'actif cosmétique, de l'émollient ou de la formulation. Selon un autre mode préféré de réalisation, l'actif cosmétique, l'émollient ou la formulation est éliminé préalablement à l'exposition dudit modèle de peau reconstruite aux au moins deux cytokines Th2 lors de ladite étape c).

Selon encore un autre mode de réalisation préféré, le modèle de peau reconstruite est mis en contact avec au moins une bactérie pathogène dans l'étape e) du test C en présence de l'actif cosmétique, de l'émollient ou de la formulation. Selon un autre mode préféré de réalisation, l'actif cosmétique, l'émollient ou la formulation est éliminé préalablement à l'exposition dudit modèle de peau reconstruite à au moins une bactérie pathogène lors de ladite étape e).

Selon encore un autre mode de réalisation préféré, le modèle de peau reconstruite est mis en contact avec au moins une bactérie pathogène dans l'étape d) du test D en présence de l'actif cosmétique, de l'émollient ou de la formulation. Selon un autre mode préféré de réalisation, l'actif cosmétique, l'émollient ou la formulation est éliminé préalablement à l'exposition dudit modèle de peau reconstruite à au moins une bactérie pathogène lors de ladite étape d).

L'homme du métier comprendra sans peine que ces différents modes de réalisation peuvent être combinés comme de besoin. Cependant, il est plus avantageux de suivre la même démarche dans chacun des quatre tests. Par exemple, si le modèle de peau reconstruite est mis en contact avec la solution de poly(dIdC) et d'ILα dans l'étape c) du test A en présence de l'actif cosmétique, de l'émollient ou de la formulation, il est préférable que le modèle de peau reconstruite soit mis en contact avec les au moins deux cytokines Th2 dans l'étape c) du test B en présence de l'actif cosmétique, de l'émollient ou de la formulation et que le modèle de peau reconstruite soit mis en contact avec au moins une bactérie pathogène dans l'étape e) du test C en présence de l'actif cosmétique, de l'émollient ou de la formulation et que le modèle de peau reconstruite soit mis en contact avec au moins une bactérie pathogène dans l'étape d) du test D en présence de l'actif cosmétique, de l'émollient ou de la formulation. En revanche, si l'actif cosmétique, l'émollient ou la formulation est éliminé préalablement à l'exposition dudit modèle de peau reconstruite à ladite solution lors de l'étape c) du test A, alors il est préférable que l'actif cosmétique, l'émollient ou la formulation soit éliminé préalablement à l'exposition dudit modèle de peau reconstruite aux au moins deux cytokines Th2 lors de l'étape c) du test B et que l'actif cosmétique, l'émollient ou la formulation soit éliminé préalablement à l'exposition dudit modèle de peau reconstruite à au moins une bactérie pathogène lors de l'étape e) du test C et que l'actif cosmétique, l'émollient ou la formulation soit éliminé préalablement à l'exposition dudit modèle de peau reconstruite à au moins une bactérie pathogène lors de l'étape d) du test D.

Selon un mode de réalisation préféré, le modèle de peau reconstruite peut être mis en contact avec la solution de poly(dldC) et d'ILα avant d'être exposé à l'actif cosmétique, à l'émollient ou à la formulation dans le test A. Selon un autre mode de réalisation préféré, le modèle de peau reconstruite peut être mis en contact avec les aux moins deux cytokines Th2 avant d'être exposé à l'actif cosmétique, à l'émollient ou à la formulation dans le test B. Selon encore un autre mode de réalisation préféré, le modèle de peau reconstruite peut être mis en contact avec au moins une bactérie pathogène avant d'être exposé à l'actif cosmétique, à l'émollient ou à la formulation dans le test C. Selon encore un autre mode de réalisation préféré, le modèle de peau reconstruite peut être mis en contact avec au moins une bactérie pathogène avant d'être exposé à l'actif cosmétique, à l'émollient ou à la formulation dans le test D. De tels modes de réalisation peuvent être particulièrement avantageux pour étudier les effets dudit actif cosmétique, dudit émollient ou de ladite formulation dans le traitement de la dermatite atopique.

Par ailleurs, l'invention a aussi pour objet une méthode d'évaluation de l'efficacité *in vitro* d'un actif cosmétique, d'un émollient ou d'une formulation dans la prévention ou la réduction des effets de la dermatite atopique de la peau d'enfant.

Ladite méthode comprend la détermination de l'efficacité dudit actif cosmétique, dudit émollient ou de ladite formulation dans chacun des quatre tests A, B, C et D, ladite méthode étant caractérisée en ce que :
- le test A comprend les étapes suivantes :
   a) obtenir un modèle de peau reconstruite à partir d'un échantillon cutané provenant d'un enfant ;
   b) mettre en contact le modèle de peau reconstruite de l'étape a) avec une solution comprenant de l'acide poly(déoxyinosinique-déoxycytidylique) (poly(dldC))et de l'interleukine 1 alpha (IL1α) ;
   c) mettre en contact ledit actif cosmétique, ledit émollient ou ladite formulation avec le modèle de peau reconstruite de l'étape b) ;
   d) mesurer le niveau d'expression d'au moins un marqueur biologique dans le modèle de peau de l'étape c) ; et
   e) évaluer l'efficacité dudit actif cosmétique, dudit émollient ou de ladite formulation en fonction du niveau de l'étape d) ;
- le test B comprend les étapes suivantes :
   a) obtenir un modèle de peau reconstruite à partir d'un échantillon cutané provenant d'un enfant ;
   b) mettre en contact le modèle de peau reconstruite de l'étape a) avec une solution comprenant au moins deux cytokines Th2 ;
   c) mettre en contact ledit actif cosmétique, ledit émollient ou ladite formulation avec le modèle de peau reconstruite de l'étape b) ;
   d) mesurer le niveau d'expression d'au moins un marqueur biologique dans le modèle de peau de l'étape c) ; et
   e) évaluer l'efficacité dudit actif cosmétique, dudit émollient ou de ladite formulation en fonction du niveau de l'étape d) ;
- le test C comprend les étapes suivantes :
   a) obtenir un modèle de peau reconstruite à partir d'un échantillon cutané provenant d'un enfant ;
   b) cultiver le modèle de peau reconstruite de l'étape a) en présence de monocytes THP-1 ;
   c) induire une altération de la fonction barrière dans le modèle de peau reconstruite de l'étape b) ;
   d) mettre en contact le modèle de peau reconstruite de l'étape c) avec au moins une bactérie pathogène ;
   e) mettre en contact ledit actif cosmétique, ledit émollient ou ladite formulation avec le modèle de peau reconstruite de l'étape d) ;
   f) mesurer le niveau d'expression et/ou d'activation d'au moins un marqueur biologique dans le modèle de peau de l'étape e) ; et
   g) évaluer l'efficacité dudit actif cosmétique, dudit émollient ou de ladite formulation en fonction du niveau de l'étape f) ; et
- le test D comprend les étapes suivantes :
   a) obtenir un modèle de peau reconstruite à partir d'un échantillon cutané provenant d'un enfant ;
   b) induire une altération de la fonction barrière dans le modèle de peau reconstruite de l'étape a) ;
   c) mettre en contact le modèle de peau reconstruite de l'étape b) avec au moins une bactérie pathogène ;
   d) mettre en contact ledit actif cosmétique, ledit émollient ou ladite formulation avec le modèle de peau reconstruite de l'étape c) ;
   e) mesurer le niveau d'expression et/ou d'activation d'au moins un marqueur biologique dans le modèle de peau de l'étape d) ; et
   f) évaluer l'efficacité dudit actif cosmétique, dudit émollient ou de ladite formulation en fonction du niveau de l'étape e),
dans laquelle ledit marqueur est choisi parmi :
- les marqueurs des activités inhibitrices de la physiologie bactérienne, dans lesquels l'activité inhibitrice de de la physiologie bactérienne est choisie parmi l'inhibition de la prolifération bactérienne et l'inhibition de la formation du biofilm ;
- les marqueurs de l'immunité, ledit marqueur de l'immunité étant HBD2 ou TLR2 ;
- les marqueurs de l'inflammation, ledit marqueur de l'inflammation étant CCL2, CXCL1, CCL7, IL6, IL18, CCL3, CCL5, CCL7, KLK5 ou TSLP ;
- les marqueurs de la fonction barrière, ledit marqueur de la fonction barrière étant FLG, KRT1, KRT10, SCEL, BARX2, LOR, IVL, TGM1 ,DSG1, CDSN, CLDN1, CASP14, SMPD1, GBA, LASS6, NMF ou les céramides ; et
- les marqueurs préférentiellement exprimés dans les cellules souches, ledit marqueur préférentiellement exprimé dans les cellules souches étant KRT15, KRT19, NOTCH1, BIRC5, ITGA6, ITGB1 ou ITGB4;
   et dans laquelle l'échantillon cutané provient d'un donneur choisi dans le groupe constitué des nouveau-nés, dont l'âge est compris entre 0 et 1 mois, des nourrissons, dont l'âge est compris entre 1 mois et 2 ans, et des enfants dont l'âge est compris entre 2 ans et 16 ans, et préférentiellement d'un donneur choisi dans le groupe constitué des nouveau-nés et des nourrissons.

L'homme du métier comprendra sans peine que les étapes b) et c) du test A peuvent être réalisées simultanément ou successivement, selon ses besoins. Autrement dit, le modèle de peau reconstruite peut être cultivé dans l'étape b) en présence de la solution de poly(dldC) et d'ILα et, en outre, en présence de l'actif cosmétique, de l'émollient ou de la formulation. Alternativement, le modèle de peau peut d'abord être cultivé dans des conditions où il est exposé à la solution de poly(dIdC) et d'ILα, puis mis en contact avec l'actif cosmétique, l'émollient ou la formulation.

De la même façon, les étapes b) et c) du test B peuvent être réalisées simultanément ou successivement, selon les besoins de l'homme du métier. Autrement dit, le modèle de peau reconstruite peut être cultivé dans l'étape b) en présence d'au moins deux cytokines Th2 et, en outre, en présence de l'actif cosmétique, de l'émollient ou de la formulation. Alternativement, le modèle de peau peut d'abord être cultivé dans des conditions où il est exposé à au moins deux cytokines Th2, puis mis en contact avec l'actif cosmétique, l'émollient ou la formulation.

Encore de la même façon, les étapes c), d) et e) du test C peuvent être réalisées simultanément ou successivement, selon les besoins de l'homme du métier. Autrement dit, le modèle de peau reconstruite peut être exposé dans l'étape c) à des conditions où la fonction barrière est altérée en présence d'au moins une bactérie pathogène et, en outre, en présence de l'actif cosmétique, de l'émollient ou de la formulation. Alternativement, le modèle de peau peut d'abord être exposé à des conditions où il subit une altération de sa fonction barrière, avant d'être exposé à au moins une bactérie pathogène, pour finalement être mis en contact avec l'actif cosmétique, l'émollient ou la formulation.

Toujours de la même façon, les étapes b), c) et d) du test D peuvent être réalisées simultanément ou successivement, selon les besoins de l'homme du métier. Autrement dit, le modèle de peau reconstruite peut être cultivé dans l'étape b) dans des conditions où la fonction barrière est altérée et en présence d'au moins une bactérie pathogène et, en outre, en présence de l'actif cosmétique, de l'émollient ou de la formulation. Alternativement, le modèle de peau peut d'abord être cultivé dans des conditions où il subit une altération de sa fonction barrière, avant d'être exposé à au moins une bactérie pathogène, pour finalement être mis en contact avec l'actif cosmétique, l'émollient ou la formulation.

L'homme du métier comprendra sans peine que ces différents modes de réalisation peuvent être combinés comme de besoin. Cependant, il est plus avantageux de suivre la même démarche dans chacun des quatre tests. Par exemple, si les étapes b) et c) du test A sont réalisées simultanément, il est préférable que les étapes b) et c) du test B soient aussi réalisées simultanément et que les étapes c), d) et e) du test C soient aussi réalisées simultanément et que les étapes b), c) et d) du test D soient aussi réalisées simultanément. En revanche, si les étapes b) et c) du test A sont réalisées successivement, il est préférable que les étapes b) et c) du test B soient aussi réalisées successivement et que les étapes c), d) et e) du test C soient aussi réalisées successivement et que les étapes b), c) et d) du test D soient aussi réalisées successivement.

Par « l'efficacité d'un actif cosmétique, d'un émollient ou d'une formulation dans la prévention ou la réduction des effets des de la dermatite atopique », on entend au sens de la présente demande la capacité de l'actif cosmétique, de l'émollient ou de la formulation à annuler ou diminuer lesdits effets de la dermatite atopique. La prévention s'entend dans le cas présent d'un traitement qui est administré avant le développement des effets de la pathologie, tandis que la réduction correspond à un traitement qui est administré une fois que les effets de la dermatite atopique sont apparus.

Par « enfant », on entend selon l'invention un individu dont l'âge est inférieur ou égal à 16 ans. Sont ainsi compris dans la catégorie des enfants selon l'invention, les nouveau-nés, dont l'âge est compris entre 0 et 1 mois, les nourrissons, qui ont entre 1 mois et 2 ans, et les enfants proprement dits, qui sont âgés d'au moins 2 ans. Un « nouveau-né », comme on l'entend ici, peut aussi bien être né à terme qu'être prématuré.

Pour lever toute ambiguïté, le terme « enfant » utilisé dans la présente demande sans autre précision doit être entendu dans son acception la plus générale, c'est-à-dire comme se référant à une personne de 16 ans ou moins. Un « adulte » au sens de la présente invention est une personne qui n'est pas un enfant, autrement dit une personne âgée de plus de 16 ans.

Selon un mode de réalisation préféré, le donneur de l'échantillon est plus particulièrement un donneur ayant un âge compris entre 0 et 1 mois, entre 1 mois et 2 ans ou entre 2 ans et 16 ans. Autrement dit, selon ce mode de réalisation, le donneur de l'échantillon est choisi dans le groupe constitué des nouveau-nés, dont l'âge est compris entre 0 et 1 mois, des nourrissons, dont l'âge est compris entre 1 mois et 2 ans, et des enfants dont l'âge est compris entre 2 ans et 16 ans. Plus préférentiellement, le donneur de l'échantillon est un nouveau-né ou un nourrisson.

Préférablement, la méthode de l'invention peut être utilisée quelle que soit l'origine ethnique ou géographique de la peau, ou encore le phototype de celle-ci. Elle peut ainsi être d'origine caucasienne, africaine, asiatique, sud-américaine, mélanésienne ou autre ; elle peut encore présenter un phototype I, II, III, IV, V ou VI, sans que cela n'affecte l'invention. Celle-ci a en effet pour objet l'identification de marqueurs biologiques caractérisant n'importe quel type de peau et ne dépendant que de l'âge du donneur.

Il est important de vérifier que les actifs cosmétiques, les émollients et les formulations sont bien tolérés. Par exemple, certains produits actuellement commercialisés pourraient entrainer des irritations s'ils sont employés régulièrement. Un tel effet ne peut qu'empirer une inflammation de la peau en développement ou déjà présente causée par la dermatite atopique.

Selon un autre aspect, l'invention a donc pour objet une méthode d'évaluation de la tolérance d'un actif cosmétique, d'un émollient ou d'une formulation par la peau atopique d'enfant ladite méthode comprenant la détermination de la tolérance dudit actif cosmétique, dudit émollient ou de ladite formulation par ladite peau atopique d'enfant dans chacun des quatre tests A, B, C et D, ladite méthode étant caractérisée en ce que :
- le test A comprend les étapes suivantes :
   a) obtenir un modèle de peau reconstruite à partir d'un échantillon cutané provenant d'un enfant ;
   b) mettre en contact ledit actif cosmétique, ledit émollient ou ladite formulation avec le modèle de peau reconstruite de l'étape a) ;
   c) mettre en contact le modèle de peau reconstruite de l'étape b) avec une solution comprenant de acide poly(déoxyinosinique-déoxycytidylique) et de l'interleukine 1 alpha (IL1α) ;
   d) mesurer le niveau d'expression d'au moins un marqueur biologique dans le modèle de peau de l'étape c) ; et
   e) déterminer si ledit actif cosmétique, ledit émollient ou ladite formulation est bien tolérée par la peau atopique d'enfant en fonction du niveau de l'étape d) ;
- le test B comprend les étapes suivantes :
   a) obtenir un modèle de peau reconstruite à partir d'un échantillon cutané provenant d'un enfant ;
   b) mettre en contact ledit actif cosmétique, ledit émollient ou ladite formulation avec le modèle de peau reconstruite de l'étape a) ;
   c) mettre en contact le modèle de peau reconstruite de l'étape b) avec une solution comprenant au moins deux cytokines Th2 ;
   d) mesurer le niveau d'expression d'au moins un marqueur biologique dans le modèle de peau de l'étape c) ; et
   e) déterminer si ledit actif cosmétique, ledit émollient ou ladite formulation est bien tolérée par la peau atopique d'enfant en fonction du niveau de l'étape d) ;
- le test C comprend les étapes suivantes :
   a) obtenir un modèle de peau reconstruite à partir d'un échantillon cutané provenant d'un enfant ;
   b) cultiver le modèle de peau reconstruite de l'étape a) en présence de monocytes THP-1 ;
   c) mettre en contact ledit actif cosmétique, ledit émollient ou ladite formulation avec le modèle de peau reconstruite de l'étape b) ;
   d) induire une altération de la fonction barrière dans le modèle de peau reconstruite de l'étape c) ;
   e) mettre en contact le modèle de peau reconstruite de l'étape d) avec au moins une bactérie pathogène ;
   f) mesurer le niveau d'expression et/ou d'activation d'au moins un marqueur biologique dans le modèle de peau de l'étape e) ; et
   g) déterminer si ledit actif cosmétique, ledit émollient ou ladite formulation est bien tolérée par la peau atopique d'enfant en fonction du niveau de l'étape f) ; et
- le test D comprend les étapes suivantes :
   a) obtenir un modèle de peau reconstruite à partir d'un échantillon cutané provenant d'un enfant ;
   b) mettre en contact ledit actif cosmétique, ledit émollient ou ladite formulation avec le modèle de peau reconstruite de l'étape a) ;
   c) induire une altération de la fonction barrière dans le modèle de peau reconstruite de l'étape b) ;
   d) mettre en contact le modèle de peau reconstruite de l'étape c) avec au moins une bactérie pathogène ;
   e) mesurer le niveau d'expression et/ou d'activation d'au moins un marqueur biologique dans le modèle de peau de l'étape d) ; et
   f) déterminer si ledit actif cosmétique, ledit émollient ou ladite formulation est bien tolérée par la peau atopique d'enfant en fonction du niveau de l'étape e),
dans laquelle ledit marqueur est choisi parmi :
- les marqueurs des activités inhibitrices de la physiologie bactérienne, dans lesquels l'activité inhibitrice de de la physiologie bactérienne est choisie parmi l'inhibition de la prolifération bactérienne et l'inhibition de la formation du biofilm ;
- les marqueurs de l'immunité, ledit marqueur de l'immunité étant HBD2 ou TLR2 ;
- les marqueurs de l'inflammation, ledit marqueur de l'inflammation étant CCL2, CXCL1, CCL7, IL6, IL18, CCL3, CCL5, CCL7, KLK5 ou TSLP ;
- les marqueurs de la fonction barrière, ledit marqueur de la fonction barrière étant FLG, KRT1, KRT10, SCEL, BARX2, LOR, IVL, TGM1 ,DSG1, CDSN, CLDN1, CASP14, SMPD1, GBA, LASS6, NMF ou les céramides ; et
- les marqueurs préférentiellement exprimés dans les cellules souches, ledit marqueur préférentiellement exprimé dans les cellules souches étant KRT15, KRT19, NOTCH1, BIRC5, ITGA6, ITGB1 ou ITGB4;
   et dans laquelle l'échantillon cutané provient d'un donneur choisi dans le groupe constitué des nouveau-nés, dont l'âge est compris entre 0 et 1 mois, des nourrissons, dont l'âge est compris entre 1 mois et 2 ans, et des enfants dont l'âge est compris entre 2 ans et 16 ans, et préférentiellement d'un donneur choisi dans le groupe constitué des nouveau-nés et des nourrissons.

La méthode de l'invention peut en outre comporter une comparaison de la viabilité cellulaire dans le modèle de peau reconstruite traité avec l'actif cosmétique, l'émollient ou la formulation et dans le modèle de peau reconstruite témoin, c'est-à-dire une peau d'enfant dans laquelle une dermatite atopique a été générée dans l'un au moins des quatre tests ci-dessus, mais qui n'a pas été traitée par l'actif cosmétique, l'émollient ou la formulation. Dans ce cas, l'actif cosmétique, l'émollient ou la formulation cosmétique est bien toléré par la peau d'enfant si la viabilité cellulaire du modèle de peau reconstruite n'est pas affectée par la présence de l'actif cosmétique, l'émollient ou de la formulation.

Selon un autre mode de réalisation préféré, la méthode de l'invention comprend donc une étape supplémentaire de détermination de la viabilité cellulaire dans le modèle de peau d'enfant atopique reconstruite traité avec l'actif cosmétique, l'émollient ou la formulation cosmétique, de détermination de la viabilité cellulaire du modèle de peau reconstruite témoin et de comparaison entre les deux. Cette étape supplémentaire peut être réalisée pour au moins un des tests A, B, C et D, ci-dessus ; de préférence, elle est réalisée pour chacun de ces tests.

De nombreux tests pour déterminer la viabilité cellulaire sont disponibles pour l'homme du métier et sont couramment utilisés en cosmétologie. On citera en particulier le test MTT, décrit par exemple dans Mosman et al. (J Immunol Methods, 65(1-2) : 55-63, 1983).

Dans un autre aspect, l'invention permet d'isoler des actifs cosmétiques, des émollients ou des formulations ayant un effet dans la prévention des effets de la dermatite atopique sur la peau d'enfant. Comme le montrent les exemples expérimentaux, l'invention permet en particulier de distinguer les actifs cosmétiques, les émollients ou les formulations selon leur activité pour prévenir les effets de la dermatite atopique affectant la peau d'enfant. L'invention est donc particulièrement adaptée pour identifier des actifs cosmétiques, des émollients ou des formulations appropriés à cette peau très spécifique.

L'invention a donc également pour objet une méthode d'identification d'un actif cosmétiques, d'un émollient ou d'une formulation pour la prévention des effets de la dermatite atopique de la peau d'enfant, ladite méthode comprenant la détermination de l'efficacité dudit actif cosmétique, dudit émollient ou de ladite formulation pour la prévention des effets de la dermatite atopique de la peau d'enfant dans chacun des quatre tests A, B, C et D, ladite méthode étant caractérisée en ce que :
- le test A comprend les étapes suivantes :
   a) obtenir un modèle de peau reconstruite à partir d'un échantillon cutané provenant d'un enfant ;
   b) mettre en contact ledit actif cosmétique, ledit émollient ou ladite formulation avec le modèle de peau reconstruite de l'étape a) ;
   c) mettre en contact le modèle de peau reconstruite de l'étape b) avec une solution comprenant de acide poly(déoxyinosinique-déoxycytidylique) et de l'interleukine 1 alpha (IL1α) ;
   d) mesurer le niveau d'expression d'au moins un marqueur biologique dans le modèle de peau de l'étape c) ; et
   e) déterminer si ledit actif cosmétique, ledit émollient ou ladite formulation candidat est un actif cosmétique, un émollient ou une formulation pour la prévention des effets de la dermatite atopique sur la peau d'enfant en fonction du niveau de l'étape d) ;
- le test B comprend les étapes suivantes :
   a) obtenir un modèle de peau reconstruite à partir d'un échantillon cutané provenant d'un enfant ;
   b) mettre en contact ledit actif cosmétique, ledit émollient ou ladite formulation avec le modèle de peau reconstruite de l'étape a) ;
   c) mettre en contact le modèle de peau reconstruite de l'étape b) avec une solution comprenant au moins deux cytokines Th2 ;
   d) mesurer le niveau d'expression d'au moins un marqueur biologique dans le modèle de peau de l'étape c) ; et
   e) déterminer si ledit actif cosmétique, ledit émollient ou ladite formulation candidat est un actif cosmétique, un émollient ou une formulation pour la prévention des effets de la dermatite atopique sur la peau d'enfant en fonction du niveau de l'étape d) ;
- le test C comprend les étapes suivantes :
   a) obtenir un modèle de peau reconstruite à partir d'un échantillon cutané provenant d'un enfant ;
   b) cultiver le modèle de peau reconstruite de l'étape a) en présence de monocytes THP-1 ;
   c) mettre en contact ledit actif cosmétique, ledit émollient ou ladite formulation avec le modèle de peau reconstruite de l'étape b) ;
   d) induire une altération de la fonction barrière dans le modèle de peau reconstruite de l'étape c) ;
   e) mettre en contact le modèle de peau reconstruite de l'étape d) avec au moins une bactérie pathogène ;
   f) mesurer le niveau d'expression et/ou d'activation d'au moins un marqueur biologique dans le modèle de peau de l'étape e) ; et
   g) déterminer si ledit actif cosmétique, ledit émollient ou ladite formulation candidat est un actif cosmétique, un émollient ou une formulation pour la prévention des effets de la dermatite atopique sur la peau d'enfant en fonction du niveau de l'étape f) ; et
- le test D comprend les étapes suivantes :
   a) obtenir un modèle de peau reconstruite à partir d'un échantillon cutané provenant d'un enfant ;
   b) mettre en contact ledit actif cosmétique, ledit émollient ou ladite formulation avec le modèle de peau reconstruite de l'étape a) ;
   c) induire une altération de la fonction barrière dans le modèle de peau reconstruite de l'étape b) ;
   d) mettre en contact le modèle de peau reconstruite de l'étape c) avec au moins une bactérie pathogène ;
   e) mesurer le niveau d'expression et/ou d'activation d'au moins un marqueur biologique dans le modèle de peau de l'étape d) ; et
   f) déterminer si ledit actif cosmétique, ledit émollient ou ladite formulation candidat est un actif cosmétique, un émollient ou une formulation pour la prévention des effets de la dermatite atopique sur la peau d'enfant en fonction du niveau de l'étape e),
dans laquelle ledit marqueur est choisi parmi :
- les marqueurs des activités inhibitrices de la physiologie bactérienne, dans lesquels l'activité inhibitrice de de la physiologie bactérienne est choisie parmi l'inhibition de la prolifération bactérienne et l'inhibition de la formation du biofilm ;
- les marqueurs de l'immunité, ledit marqueur de l'immunité étant HBD2 ou TLR2 ;
- les marqueurs de l'inflammation, ledit marqueur de l'inflammation étant CCL2, CXCL1, CCL7, IL6, IL18, CCL3, CCL5, CCL7, KLK5 ou TSLP ;
- les marqueurs de la fonction barrière, ledit marqueur de la fonction barrière étant FLG, KRT1, KRT10, SCEL, BARX2, LOR, IVL, TGM1 ,DSG1, CDSN, CLDN1, CASP14, SMPD1, GBA, LASS6, NMF ou les céramides ; et
- les marqueurs préférentiellement exprimés dans les cellules souches, ledit marqueur préférentiellement exprimé dans les cellules souches étant KRT15, KRT19, NOTCH1, BIRC5, ITGA6, ITGB1 ou ITGB4;
   et dans laquelle l'échantillon cutané provient d'un donneur choisi dans le groupe constitué des nouveau-nés, dont l'âge est compris entre 0 et 1 mois, des nourrissons, dont l'âge est compris entre 1 mois et 2 ans, et des enfants dont l'âge est compris entre 2 ans et 16 ans, et préférentiellement d'un donneur choisi dans le groupe constitué des nouveau-nés et des nourrissons.

Dans un premier mode de réalisation préféré, le modèle de peau reconstruite est mis en contact avec la solution de poly(dIdC) et d'ILα dans l'étape c) du test A en présence de l'actif cosmétique, l'émollient ou de la formulation. Selon un autre mode préféré de réalisation, l'actif cosmétique, l'émollient ou la formulation est éliminé préalablement à l'exposition dudit modèle de peau reconstruite à ladite solution lors de ladite étape c).

Selon un autre mode de réalisation préféré, le modèle de peau reconstruite est mis en contact avec les au moins deux cytokines Th2 dans l'étape c) du test B en présence de l'actif cosmétique, de l'émollient ou de la formulation. Selon un autre mode préféré de réalisation, l'actif cosmétique, l'émollient ou la formulation est éliminé préalablement à l'exposition dudit modèle de peau reconstruite aux au moins deux cytokines Th2 lors de ladite étape c).

Selon encore un autre mode de réalisation préféré, le modèle de peau reconstruite est mis en contact avec au moins une bactérie pathogène dans l'étape e) du test C en présence de l'actif cosmétique, de l'émollient ou de la formulation. Selon un autre mode préféré de réalisation, l'actif cosmétique, l'émollient ou la formulation est éliminé préalablement à l'exposition dudit modèle de peau reconstruite à au moins une bactérie pathogène lors de ladite étape e).

Selon encore un autre mode de réalisation préféré, le modèle de peau reconstruite est mis en contact avec au moins une bactérie pathogène dans l'étape d) du test D en présence de l'actif cosmétique, de l'émollient ou de la formulation. Selon un autre mode préféré de réalisation, l'actif cosmétique, l'émollient ou la formulation est éliminé préalablement à l'exposition dudit modèle de peau reconstruite à au moins une bactérie pathogène lors de ladite étape d).

L'homme du métier comprendra sans peine que ces différents modes de réalisation peuvent être combinés comme de besoin. Cependant, il est plus avantageux de suivre la même démarche dans chacun des quatre tests. Par exemple, si le modèle de peau reconstruite est mis en contact avec la solution de poly(dldC) et d'ILα dans l'étape c) du test A en présence de l'actif cosmétique, de l'émollient ou de la formulation, il est préférable que le modèle de peau reconstruite soit mis en contact avec les au moins deux cytokines Th2 dans l'étape c) du test B en présence de l'actif cosmétique, de l'émollient ou de la formulation et que le modèle de peau reconstruite soit mis en contact avec au moins une bactérie pathogène dans l'étape e) du test C en présence de l'actif cosmétique, de l'émollient ou de la formulation et que le modèle de peau reconstruite soit mis en contact avec au moins une bactérie pathogène dans l'étape d) du test D en présence de l'actif cosmétique, de l'émollient ou de la formulation. En revanche, si l'actif cosmétique, l'émollient ou la formulation est éliminé préalablement à l'exposition dudit modèle de peau reconstruite à ladite solution lors de l'étape c) du test A, alors il est préférable que l'actif cosmétique, l'émollient ou la formulation soit éliminé préalablement à l'exposition dudit modèle de peau reconstruite aux au moins deux cytokines Th2 lors de l'étape c) du test B et que l'actif cosmétique, l'émollient ou la formulation soit éliminé préalablement à l'exposition dudit modèle de peau reconstruite à au moins une bactérie pathogène lors de l'étape e) du test C et que l'actif cosmétique, l'émollient ou la formulation soit éliminé préalablement à l'exposition dudit modèle de peau reconstruite à au moins une bactérie pathogène lors de l'étape d) du test D.

Selon un mode de réalisation préféré, le modèle de peau reconstruite peut être mis en contact avec la solution de poly(dldC) et d'ILα avant d'être exposé à l'actif cosmétique, à l'émollient ou à la formulation dans le test A. Selon un autre mode de réalisation préféré, le modèle de peau reconstruite peut être mis en contact avec les aux moins deux cytokines Th2 avant d'être exposé à l'actif cosmétique, à l'émollient ou à la formulation dans le test B. Selon encore un autre mode de réalisation préféré, le modèle de peau reconstruite peut être mis en contact avec au moins une bactérie pathogène avant d'être exposé à l'actif cosmétique, à l'émollient ou à la formulation dans le test C. Selon encore un autre mode de réalisation préféré, le modèle de peau reconstruite peut être mis en contact avec au moins une bactérie pathogène avant d'être exposé à l'actif cosmétique, à l'émollient ou à la formulation dans le test D. De tels modes de réalisation peuvent être particulièrement avantageux pour étudier les effets dudit actif cosmétique, dudit émollient ou de ladite formulation dans le traitement de la dermatite atopique.

Par ailleurs, la méthode décrite dans ce document permet d'isoler des actifs cosmétiques, des émollients ou des formulations permettant de réduire les effets de la dermatite atopique affectant la peau d'enfant, ladite méthode comprenant la détermination de l'efficacité dudit actif cosmétique, dudit émollient ou de ladite formulation pour la réduction des effets de la dermatite atopique de la peau d'enfant dans chacun des quatre tests A, B, C et D, ladite méthode étant caractérisée en ce que :
- le test A comprend les étapes suivantes :
   a) obtenir un modèle de peau reconstruite à partir d'un échantillon cutané provenant d'un enfant ;
   b) mettre en contact le modèle de peau reconstruite de l'étape a) avec une solution comprenant de acide poly(déoxyinosinique-déoxycytidylique) et de l'interleukine 1 alpha (IL1α) ;
   c) mettre en contact ledit actif cosmétique, ledit émollient ou ladite formulation avec le modèle de peau reconstruite de l'étape b) ;
   d) mesurer le niveau d'expression d'au moins un marqueur biologique dans le modèle de peau de l'étape c) ; et
   e) déterminer si ledit actif cosmétique, ledit émollient ou ladite formulation candidat est un actif cosmétique, un émollient ou une formulation pour la réduction des effets de la dermatite atopique sur la peau d'enfant en fonction du niveau de l'étape d) ;
- le test B comprend les étapes suivantes :
   a) obtenir un modèle de peau reconstruite à partir d'un échantillon cutané provenant d'un enfant ;
   b) mettre en contact le modèle de peau reconstruite de l'étape a) avec une solution comprenant au moins deux cytokines Th2 ;
   c) mettre en contact ledit actif cosmétique, ledit émollient ou ladite formulation avec le modèle de peau reconstruite de l'étape b) ;
   d) mesurer le niveau d'expression d'au moins un marqueur biologique dans le modèle de peau de l'étape c) ; et
   e) déterminer si ledit actif cosmétique, ledit émollient ou ladite formulation candidat est un actif cosmétique, un émollient ou une formulation pour la réduction des effets de la dermatite atopique sur la peau d'enfant en fonction du niveau de l'étape d) ;
- le test C comprend les étapes suivantes :
   a) obtenir un modèle de peau reconstruite à partir d'un échantillon cutané provenant d'un enfant ;
   b) cultiver le modèle de peau reconstruite de l'étape a) en présence de monocytes THP-1 ;
   c) induire une altération de la fonction barrière dans le modèle de peau reconstruite de l'étape b) ;
   d) mettre en contact le modèle de peau reconstruite de l'étape c) avec au moins une bactérie pathogène ;
   e) mettre en contact ledit actif cosmétique, ledit émollient ou ladite formulation avec le modèle de peau reconstruite de l'étape d) ;
   f) mesurer le niveau d'expression et/ou d'activation d'au moins un marqueur biologique dans le modèle de peau de l'étape e) ; et
   g) déterminer si ledit actif cosmétique, ledit émollient ou ladite formulation candidat est un actif cosmétique, un émollient ou une formulation pour la réduction des effets de la dermatite atopique sur la peau d'enfant en fonction du niveau de l'étape f) ; et
- le test D comprend les étapes suivantes :
   a) obtenir un modèle de peau reconstruite à partir d'un échantillon cutané provenant d'un enfant ;
   b) induire une altération de la fonction barrière dans le modèle de peau reconstruite de l'étape a) ;
   c) mettre en contact le modèle de peau reconstruite de l'étape b) avec au moins une bactérie pathogène ;
   d) mettre en contact ledit actif cosmétique, ledit émollient ou ladite formulation avec le modèle de peau reconstruite de l'étape c) ;
   e) mesurer le niveau d'expression et/ou d'activation d'au moins un marqueur biologique dans le modèle de peau de l'étape d) ; et
   f) déterminer si ledit actif cosmétique, ledit émollient ou ladite formulation candidat est un actif cosmétique, un émollient ou une formulation pour la réduction des effets de la dermatite atopique sur la peau d'enfant en fonction du niveau de l'étape e).

L'homme du métier comprendra sans peine que les étapes b) et c) du test A peuvent être réalisées simultanément ou successivement, selon ses besoins. Autrement dit, le modèle de peau reconstruite peut être cultivé dans l'étape b) en présence de la solution de poly(dldC) et d'ILα et, en outre, en présence de l'actif cosmétique, de l'émollient ou de la formulation. Alternativement, le modèle de peau peut d'abord être cultivé dans des conditions où il est exposé à la solution de poly(dIdC) et d'ILα, puis mis en contact avec l'actif cosmétique, l'émollient ou la formulation.

De la même façon, les étapes b) et c) du test B peuvent être réalisées simultanément ou successivement, selon les besoins de l'homme du métier. Autrement dit, le modèle de peau reconstruite peut être cultivé dans l'étape b) en présence d'au moins deux cytokines Th2 et, en outre, en présence de l'actif cosmétique, de l'émollient ou de la formulation. Alternativement, le modèle de peau peut d'abord être cultivé dans des conditions où il est exposé à au moins deux cytokines Th2, puis mis en contact avec l'actif cosmétique, l'émollient ou la formulation.

Encore de la même façon, les étapes c), d) et e) du test C peuvent être réalisées simultanément ou successivement, selon les besoins de l'homme du métier. Autrement dit, le modèle de peau reconstruite peut être exposé dans l'étape c) à des conditions où la fonction barrière est altérée en présence d'au moins une bactérie pathogène et, en outre, en présence de l'actif cosmétique, de l'émollient ou de la formulation.

Alternativement, le modèle de peau peut d'abord être exposé à des conditions où il subit une altération de sa fonction barrière, avant d'être exposé à au moins une bactérie pathogène, pour finalement être mis en contact avec l'actif cosmétique, l'émollient ou la formulation.

Toujours de la même façon, les étapes b), c) et d) du test D peuvent être réalisées simultanément ou successivement, selon les besoins de l'homme du métier. Autrement dit, le modèle de peau reconstruite peut être cultivé dans l'étape b) dans des conditions où la fonction barrière est altérée et en présence d'au moins une bactérie pathogène et, en outre, en présence de l'actif cosmétique, de l'émollient ou de la formulation. Alternativement, le modèle de peau peut d'abord être cultivé dans des conditions où il subit une altération de sa fonction barrière, avant d'être exposé à au moins une bactérie pathogène, pour finalement être mis en contact avec l'actif cosmétique, l'émollient ou la formulation.

L'homme du métier comprendra sans peine que ces différents modes de réalisation peuvent être combinés comme de besoin. Cependant, il est plus avantageux de suivre la même démarche dans chacun des quatre tests. Par exemple, si les étapes b) et c) du test A sont réalisées simultanément, il est préférable que les étapes b) et c) du test B soient aussi réalisées simultanément et que les étapes c), d) et e) du test C soient aussi réalisées simultanément et que les étapes b), c) et d) du test D soient aussi réalisées simultanément. En revanche, si les étapes b) et c) du test A sont réalisées successivement, il est préférable que les étapes b) et c) du test B soient aussi réalisées successivement et que les étapes c), d) et e) du test C soient aussi réalisées successivement et que les étapes b), c) et d) du test D soient aussi réalisées successivement.

La formulation candidate est une formulation pour la prévention ou la réduction des effets de la dermatite atopique affectant la peau d'enfant, si ladite formulation candidate permet de moduler l'expression d'au moins un marqueur biologique utilisé dans l'invention. Cette modulation peut correspondre, selon les cas, et en particulier selon la nature du marqueur biologique, à une augmentation ou à une diminution de l'expression dudit marqueur. Par exemple, il peut être intéressant d'isoler des formulations minimisant les effets de la dermatite atopique sur les marqueurs préférentiellement exprimés dans les cellules souches, ces formulations permettant de préserver la capacité de renouvellement de l'épiderme fragile des enfants. De même, il serait avantageux d'identifier des formulations minimisant les effets de la dermatite atopique sur les marqueurs de la barrière chez les enfants, afin de maintenir l'intégrité de la barrière cutanée. Enfin, il pourrait être désirable d'isoler des formulations qui n'induiraient pas les marqueurs de l'inflammation, afin de ne pas accentuer les effets de la pathologie.

De la même façon, l'actif cosmétique ou l'émollient candidat est un actif cosmétique ou un émollient pour la prévention ou la réduction des effets de la dermatite atopique affectant la peau d'enfant, si ledit actif cosmétique ou émollient candidat permet de moduler l'expression d'au moins un marqueur biologique utilisé dans l'invention. Cette modulation peut correspondre, selon les cas, et en particulier selon la nature du marqueur biologique, à une augmentation ou à une diminution de l'expression dudit marqueur.

Dans un premier temps, l'actif cosmétique, l'émollient ou la formulation d'intérêt est mis en contact avec une culture de peau reconstruite obtenue à partir d'un échantillon provenant d'un enfant. Cette mise en contact de l'actif cosmétique ou de l'émollient d'intérêt avec le modèle de peau peut être faite directement. Alternativement, il pourra être avantageux de formuler l'actif cosmétique ou l'émollient d'intérêt, par exemple de façon à obtenir une composition liquide, afin de faciliter sa mise en contact avec le modèle de peau. Ainsi, selon un mode de réalisation de l'invention, le procédé comprend en outre une étape de formulation de l'actif cosmétique ou de l'émollient, notamment sous forme d'une solution liquide, en particulier aqueuse, préalablement à l'étape de mise en contact dudit actif cosmétique ou dudit émollient avec un modèle de peau.

Les inventeurs ont précédemment montré que les profils d'expression de catégories spécifiques de gènes (par exemple, gènes de la barrière, de l'inflammation, de défense, des cellules souches) évoluent en fonction de l'âge (demande WO 2014/009566). L'homme du métier peut ainsi aisément caractériser la peau au niveau moléculaire depuis la naissance jusqu'à l'âge adulte. Plus particulièrement l'homme du métier notera que les cellules de la peau d'enfant présentent un profil d'expression spécifique des gènes impliqués dans des processus physiologiques particuliers, notamment le métabolisme cellulaire, la réponse au stress, l'inflammation, l'immunité, l'apoptose, la croissance/prolifération et le cycle cellulaire, la signalisation cellulaire, la migration et la différenciation, la barrière épidermique, l'adhésion et les cellules souches pluripotentes de la peau.

Au sens de l'invention, le modèle de peau reconstruite obtenu à partir d'un échantillon cutané provenant d'un enfant peut être tout modèle tissulaire comprenant des cellules de la peau, notamment des kératinocytes, et dans lequel lesdites cellules cutanées ont été obtenues à partir d'un échantillon provenant d'un enfant.

Par « échantillon cutané », on entend au sens de l'invention tout échantillon contenant des cellules de la peau. Les échantillons cutanés selon l'invention comprennent donc aussi bien les explants de peau frais obtenus directement du patient, que les cultures de cellules cutanées en suspension, les cultures de cellules cutanées en monocouche, les cultures de cellules cutanées en bicouche et les modèles tissulaires, dont les cultures de peaux reconstruites et les cultures de muqueuses reconstruites. Comme il est souvent difficile de travailler sur des explants frais, il est particulièrement avantageux, dans le cadre de la présente invention, d'utiliser des cultures de cellules cutanées. Avantageusement, les cellules cutanées selon l'invention comprennent des cellules normales, saines ou pathologiques, ou des cellules issues de lignées. Par exemple, les cellules cutanées mises en culture peuvent être des cellules obtenues à partir d'explant de tissu cutané. Par « explant » ou « explant de peau », on entend ici un prélèvement de cellules ou de tissu cutané, lequel peut être réalisé dans un but chirurgical ou pour effectuer des analyses.

En particulier, un explant peut être obtenu lors d'une exérèse chirurgicale. Par « exérèse », on entend ici une intervention chirurgicale consistant à découper (exciser) une partie plus ou moins large ou profonde de la peau pour en traiter une anomalie ou une excroissance. On procède à une exérèse soit pour retirer une tumeur cancéreuse ou suspecte de l'être, soit pour traiter une anomalie bénigne de la peau qui est gênante, que ce soit pour des raisons fonctionnelles ou esthétiques. Une exérèse au sens de l'invention inclut par exemple les échantillons de peau obtenus après chirurgie plastique (plastie mammaire, abdominale, lifting, prélèvement préputial, otoplastie, c'est-à-dire recollement d'oreille, syndactylie ou doigt surnuméraire, etc.).

Un explant peut aussi être obtenu par biopsie. Par « biopsie », on entend ici un prélèvement de cellules ou tissu cutané réalisé à des fins d'analyse. Plusieurs types de procédures de biopsies sont connus et pratiqués dans le domaine. Les types les plus communs comprennent (1) la biopsie incisionnelle, dans laquelle seul un échantillon du tissu est prélevé ; (2) la biopsie excisionnelle (ou biopsie chirurgicale) qui consiste en l'ablation totale d'une masse tumorale, réalisant ainsi un geste thérapeutique et diagnostique, et (3) la biopsie à l'aiguille, dans laquelle un échantillon de tissu est prélevé avec une aiguille, celle-ci pouvant être large ou fine. D'autres types de biopsie existent, comme par exemple le frottis ou le curettage, et sont aussi englobés dans la présente invention.

Alternativement, lesdites cellules cutanées peuvent être obtenues par différentiation de cellules souches (Guenou et al., Lancet, 374(9703) : 1745-1753, 2009 ; Nissan et al., Proc. Natl. Acad. Sci., 108(36) : 14861-14866, 2011 ; Kraehenbuehl et al., Nature Methods, 8 : 731-736, 2011).

Les cellules cutanées selon l'invention, qu'elles proviennent d'une biopsie ou qu'elles soient obtenues par différentiation de cellules souches, comprennent au moins un type de cellules habituellement présentes dans l'hypoderme, le derme et/ou l'épiderme. Ces cellules comprennent ainsi, entre autres, des kératinocytes, des mélanocytes, des fibroblastes, des adipocytes, des cellules endothéliales, des mastocytes, des cellules de Langerhans et/ou des cellules de Merkel. De façon préférentielle, les cellules cutanées selon l'invention comprennent au moins des kératinocytes et/ou des fibroblastes. De façon plus préférentielle, les cellules cutanées selon l'invention comprennent des kératinocytes et/ou des fibroblastes.

De nombreuses méthodes de culture de cellules cutanées sont connues de l'homme du métier. N'importe laquelle de ces méthodes peut être utilisée pour cultiver les cellules cutanées utilisé dans l'invention. Avantageusement, les cellules cutanées sont cultivées et/ou conservées dans des conditions maintenant, au moins partiellement, un métabolisme cellulaire et/ou des fonctions cellulaires. La culture de cellules cutanées selon l'invention comprend donc aussi bien les cultures de cellules cutanées en suspension, les cultures de cellules cutanées en monocouche ou les cultures de cellules cutanées en bicouche que des modèles tissulaires, dont les cultures de peaux reconstruites et les cultures de muqueuses reconstruites.

Par exemple, les cultures de cellules cutanées en suspension sont pratiquées en routine dans un très grand nombre de laboratoires et ce, depuis plusieurs dizaines d'années. De même, les cultures de cellules cutanées en monocouche ou en bicouche sont connues et utilisées depuis très longtemps.

Par ailleurs, de nombreux modèles tissulaires, dont en particulier des modèles de peaux reconstruites et des modèles de muqueuses reconstruites (Rosdy et al., In Vitro Toxicol., 10(1) : 39-47, 1997 ; Ponec et al., J Invest Dermatol., 109(3) : 348-355, 1997 ; Ponec et al., Int J Pharm., 203(1-2) : 211-225, 2000; Schmalz et al., Eur J Oral Sci., 108(5) : 442-448, 2000 ; Black et al., Tissue Eng, 11(5-6): 723-733, 2005 ; Dongari-Batgtzoglou et Kashleva, Nat Protoc, 1(4) : 2012-2018, 2006 ; Bechtoille et al., Tissue Eng, 13(11) : 2667-2679, 2007 ; Vrana et al., Invest Ophthalmol Vis Sci, 49(12) : 5325-5331, 2008 ; Kinicoglu et al., Biomaterials, 30(32) : 6418-6425, 2009 ; Auxenfans et al., Eur J Dermatol, 19(2) : 107-113, 2009; Kinicoglu et al., Biomaterials, 32(25): 5756-5764, 2011 ; Costin et al., Altern Lab Anim, 39(4): 317-337, 2011 ; Auxenfans et al., J Tissue Eng Regen Med, 6(7) : 512-518, 2012 ; Lequeux et al., Skin Pharmacol Physiol, 25(1) : 47-55, 2012; EP 29 678 ; EP 285 471 ; EP 789 074 ; EP 1 451 302 B1 ; EP 1 878 790 B1 ; EP 1 974 718 ; US 2007/0148,771 ; US 2010/0,099,576 ; WO 02/070729 ; WO 2006/063864 ; WO 2006/0,63865 ; WO 2007/064305) sont à la disposition de l'homme du métier.

Avantageusement, le modèle tissulaire comprend les modèles de peau reconstruite et les modèles de muqueuse reconstruite. Préférablement, le modèle de peau reconstruite est sélectionné dans le groupe comprenant les modèles de derme, contenant principalement des cellules stromales, et plus particulièrement encore des fibroblastes, les modèles d'épiderme constitué principalement de kératinocytes, les modèles d'hypoderme, les modèles de peau comprenant un derme et un épiderme, et les modèles de peau comprenant un derme, un épiderme et un hypoderme. Les modèles comprenant au moins un derme, forment des tissus de type conjonctifs, tandis que les modèles comprenant au moins un épiderme forment des épithélia stratifiés comprenant les couches caractéristiques du tissu considéré. Par exemple, on peut identifier dans les modèles d'épiderme une couche basale (*stratum basalis*), une couche épineuse (*stratum spinosum*), une couche granuleuse (*stratum granulosum*), et une couche cornée (*stratum corneum*). D'autre part, de façon préférée, le modèle de muqueuse reconstruite selon l'invention est un modèle de muqueuse de la bouche, de la gencive, du vagin ou de la cornée.

Avantageusement, ledit modèle est un modèle tissulaire de type conjonctif de matrice de derme comprenant un support matriciel de préférence choisi parmi :
- un support inerte choisi parmi le groupe consistant d'une membrane synthétique semi-perméable, en particulier une membrane de nitrocellulose semi-perméable, une membrane de nylon semi- perméable, une membrane ou une éponge de téflon, une membrane de polycarbonate ou de polyéthylène, polypropylène, polyéthylène téréphtalate (PET) semi-perméable, une membrane inorganique Anopore semi-perméable, d'acétate ou d'ester de cellulose (HATF), une membrane Biopore-CM semi-perméable, une membrane de polyester semi-perméable, une membrane ou un film d'acide polyglycolique.

Dans ce groupe on trouve par exemple les modèles dermiques Skin²™ modèle ZK1100 et Dermagraft® et Transcyte® (Advanced Tissue Sciences) ;
- un plastique traité culture cellulaire (formation d'un feuillet dermique : Michel et al., In Vitro Cell. Dev Biol.-Animal, 35 : 318-326, 1999) ;
- un gel ou une membrane à base d'acide hyaluronique (Hyalograft® 3D - Fidia Advanced Biopolymers) et/ou de collagène (comme par exemple un derme équivalent ou des lattices de collagène) et/ou de fibronectine et/ou de fibrine ; dans ce groupe on trouve par exemple le modèle dermique Vitrix® (Organogenesis) ;
- une matrice poreuse, surfacée ou non surfacée (par exemple un derme équivalent), réalisée à partir de collagène pouvant contenir un ou plusieurs glycosaminoglycanes et/ou éventuellement du chitosane (EP0296078A1, WO 01/911821 et WO 01/92322).

Dans ce groupe on trouve par exemple le modèle dermique Mimederm® (Coletica).

Ces supports matriciels comprennent des cellules stromales, en particulier des fibroblastes.

Avantageusement, ledit modèle de peau est un modèle d'épiderme comprenant un support matriciel de préférence choisi parmi :
- un support inerte choisi parmi le groupe consistant d'une membrane synthétique semi-perméable, en particulier une membrane de nitrocellulose semi-perméable, une membrane de nylon semi- perméable, une membrane ou une éponge de téflon, une membrane de polycarbonate ou de polyéthylène, polypropylène, de polyéthylène téréphtalate (PET) semi-perméable, une membrane inorganique Anopore semi-perméable, d'acétate ou d'ester de cellulose (HATF), une membrane Biopore-CM semi-perméable, une membrane de polyester semi-perméable ;
   dans ce groupe on trouve les modèles Epiderme reconstruits (Skinethic®) ainsi que le modèle EpiDerm®, (Mattek Corporation) ;
- un film ou une membrane à base d'acide hyaluronique et/ou de collagène et/ou de fibronectine et/ou de fibrine.

Dans ce groupe on peut citer particulièrement les modèles : Laserskin® (Fidia Advanced Biopolymers), Episkin ® (L'Oréal).

Ces modèles peuvent être ensemencés par des fibroblastes dans la partie dermique.

Ces modèles, dans lesquels peuvent être intégrés ou non des fibroblastes, servent de support pour l'ensemencement des kératinocytes et la reconstitution de l'épiderme. Avantageusement, sont introduites, outre les kératinocytes, des cellules pigmentaires, des cellules immunocompétentes, des cellules nerveuses ; de préférence les cellules immunocompétentes sont des cellules de Langerhans.

Avantageusement, ledit modèle tissulaire est un modèle tissulaire de peau ou de muqueuse reconstruite comprenant un support matriciel dermique ou de chorion de préférence choisi parmi :
- un support inerte choisi parmi le groupe consistant d'une membrane synthétique semi-perméable, en particulier une membrane de nitrocellulose semi-perméable, une membrane de nylon semi- perméable, une membrane ou une éponge de téflon, une membrane de polycarbonate ou de polyéthylène, polypropylène, de polyéthylène téréphtalate (PET) semi-perméable, une membrane inorganique Anopore semi-perméable, d'acétate ou d'ester de cellulose (HATF), une membrane Biopore-CM semi-perméable, une membrane de polyester semi-perméable, ledit support inerte contenant ou non des cellules stromales, en particulier des fibroblastes,
- un gel à base de collagène et/ou acide hyaluronique et/ou fibronectine, et/ou de fibrine comprenant des cellules stromales en particulier des fibroblastes,
- une matrice poreuse, surfacée ou non surfacée, réalisée à partir de collagène pouvant contenir un ou plusieurs glycosaminoglycanes et/ou éventuellement du chitosane, ces matrices poreuses intégrant des cellules stromales, en particulier des fibroblastes,
- un derme desépidermisé ou derme mort, humain ou animal.

Dans ce groupe, on peut citer particulièrement les modèles Mimeskin (Coletica), EpidermFT™, EpiAirway™, EpiOccular™ EpiOral™, EpiGingival™, EpiVaginal™ (MatTek corporation), Human Corneal Epithelium (HCE), Human Oral Epithelium (HOE), Human Gingival Epithelium (HGE), Human Vaginal Epithelium (HVE) (Skinethic®), Phenion® Full Thickness Skin Model (Phenion) Apligraf® (Organogenesis), ATS-2000 (CellSystems® Biotechnologie Vertrieb) ainsi que Skin 2TM (ZK1200-1300-2000 Advanced Tissue Science).

Il existe par ailleurs des modèles dédiés à la thérapie tissulaire qui peuvent également être utilisés dans le cadre de la présente invention. On peut citer les modèles Epidex (Modex Thérapeutiques), Epibase® (Laboratoire Genévrier), Epicell™ (Genzyme), Autoderm™ et Transderm™ (Innogenetics).

Le support matriciel est ensuite ensemencé par des kératinocytes pour reconstruire l'épiderme et obtenir finalement une peau reconstruite.

Avantageusement, le modèle de peau utilisé comprend un modèle dans lequel a été incorporé au moins un type cellulaire complémentaire, comme des cellules endothéliales (CE) et/ou des cellules immunitaires telles que lymphocytes, macrophages, mastocytes, cellules dendritiques et/ou des cellules adipeuses et/ou des annexes cutanées, comme des poils, des cheveux, des glandes sébacées.

Après avoir exposé le modèle de peau reconstruite à au moins une cytokine, l'homme du métier pourra procéder à la mesure du niveau d'expression des marqueurs biologiques.

Par « marqueur biologique », on entend au sens de la présente demande une caractéristique qui est objectivement mesurée et évaluée comme indicateur de processus biologiques normaux, de processus pathogéniques, ou de réponses pharmacologiques à une intervention thérapeutique. Un marqueur biologique désigne donc toute une gamme de substances, d'activités et de paramètre divers. Par exemple, un marqueur biologique peut être une substance dont la détection indique un état pathologique particulier (par exemple la présence de la protéine C réactive en tant que marqueur d'une infection), ou au contraire une substance dont la détection indique un état physiologique spécifique. Alternativement, le marqueur biologique peut être un paramètre, lequel est par exemple caractéristique d'un état pathologique particulier. Un tel paramètre est par exemple l'activité de la peau d'inhibition de la prolifération des bactéries pathogènes ou de la formation d'un biofilm par ces bactéries. Le marqueur biologique est préférentiellement un gène, les produits d'un gène tels que ses transcrits, les peptides issus de ses transcrits, un lipide, un sucre ou un métabolite, ou une activité physiologique du modèle de peau.

Selon un mode de réalisation, le marqueur biologique est un gène, les produits d'un gène tels que des transcrits ou des peptides, un lipide, un sucre ou un métabolite ou une activité dont les changements d'expression et/ou d'activation, en particulier de niveau d'expression et/ou d'activation, corrèlent avec un état physiologique de la peau d'enfant.

L'homme du métier cherchant à déterminer à quelle classe appartient un marqueur génique ou protéique pourra facilement consulter la littérature scientifique pertinente ou se rapporter à des bases de données publiques telles que, par exemple, celles regroupées sur le site internet du National Center for Biotechnology Information (http://www.ncbi.nlm.nih.gov/guide/).

Les inventeurs ont particulièrement sélectionné des marqueurs dont la variation du niveau d'expression et/ou d'activation varie dans la peau atopique d'enfant de manière surprenante et inattendue chez les enfants. Les marqueurs sélectionnés présentent donc un intérêt particulier dans le cadre de la méthode de l'invention, dans la mesure où leur niveau d'expression et/ou d'activation est mesuré sur un modèle de peau reproduisant les caractéristiques de la peau d'enfant dans au moins une des phases de la dermatite atopique.

Les inventeurs ont notamment montré que les marqueurs de l'inflammation sont particulièrement exprimés après que la peau atopique d'enfant a été infectée par des bactéries pathogènes. L'inflammation est une réaction normale de défense de l'organisme, mais elle peut contribuer à la diminution de l'intégrité de la peau. En outre, les inventeurs ont montré que dans le même temps, l'expression et/ou l'activation des marqueurs conduisant à un renforcement de la barrière diminue. Par ailleurs, les marqueurs des cellules souches sont eux-aussi affectés et par conséquent la capacité de renouvellement de l'épiderme. En revanche, la mise en contact de la peau avec une formulation active contre les effets de la dermatite atopique sur la peau d'enfant permet de prévenir et corriger les variations d'expression et/ou d'activation desdits marqueurs, ce qui souligne la pertinence de ceux-ci. Enfin, la peau atopique infantile possédait une activité réduite d'inhibition de la prolifération des bactéries pathogènes. De même, l'activité inhibitrice de la formation de biofilms par ces mêmes bactéries est réduite chez des peaux d'enfants atteints de dermatite atopique.

Le marqueur biologique est donc avantageusement un marqueur sélectionné dans le groupe des activités inhibitrices de la physiologie bactérienne, des marqueurs de l'immunité, de l'inflammation cutanée, de la fonction barrière et des marqueurs préférentiellement exprimés dans les cellules souches.

Selon un premier mode de réalisation préféré, le marqueur biologique est une activité inhibitrice de la physiologie bactérienne. Plus particulièrement, ledit marqueur biologique est une activité d'inhibition de la prolifération des bactéries pathogènes. En effet, il est connu que lesdites bactéries pathogènes, notamment les staphylocoques et les streptocoques, ne sont pas, ou ne sont que peu capables de proliférer sur une peau saine, tandis qu'elles sont capables de s'établir et de se multiplier sur une peau atopique. Cette activité peut être mesurée simplement par un simple comptage bactérien. Par exemple, un nombre déterminé de bactéries peut être inoculé sur le modèle de peau reconstitué et le nombre restant de cellules bactériennes est déterminé après un temps spécifié ou à intervalles réguliers. Alternativement, le marqueur biologique est une activité d'inhibition de la formation d'un biofilm par les bactéries pathogènes. Par « biofilm », on entend ici un type d'organisation de microorganismes dans laquelle les cellules adhèrent à une surface. Le plus souvent, les biofilms sont caractérisés par la sécrétion d'une matrice adhésive et protectrice d'exopolymères. Dans un biofilm, les microorganismes et, en particulier, les bactéries, dont notamment les staphylocoques et les streptocoques, sont sous forme « sessile », c'est-à-dire attachés à la surface et vivant en communauté. De nombreux microorganismes, dont des bactéries pathogènes telles que celles décrites plus haut, forment des biofilms. Il est d'ailleurs considéré qu'il s'agit là du mode de vie naturel des microorganismes. La structure et la physiologie du biofilm donneraient aux microorganismes qui le constituent des conditions d'organisation sociale proches de celles qu'établissent entre elles les cellules eucaryotes au sein des tissus. Ainsi, les biofilms constituent des réservoirs pour plusieurs espèces de microorganismes et leur fournissent une protection vis-à-vis des agressions extérieures, telles que les agents désinfectants, les antibiotiques, les antiseptiques, via la matrice extracellulaire (Tremblay et al., Can J Vet Res. 2014 ; 78(2) : 110-116).

Un biofilm peut comprendre une ou plusieurs espèces de microorganismes. Préférentiellement, le biofilm comprend des cellules d'au moins une espèce de bactérie pathogène telles que décrites plus haut. Plus préférentiellement, le biofilm ne comprend que des cellules de bactéries pathogènes. Plus préférentiellement encore, le biofilm est constitué des cellules d'une seule espèce de bactérie pathogène.

L'activité inhibitrice du biofilm peut être mesurée par exemple en suivant au microscope, notamment au microscope électronique à balayage, la formation du biofilm à la surface du modèle de peau atopique de l'invention.

Cette approche a permis de différencier des formulations selon leur pouvoir restaurant l'un et/ou l'autre de ces activités inhibitrices, comme le montrent les exemples expérimentaux.

La méthode de l'invention est caractérisée en ce que l'activité inhibitrice de de la physiologie bactérienne est choisie parmi l'inhibition de la prolifération bactérienne et l'inhibition de la formation du biofilm.

Le marqueur biologique peut aussi être un marqueur de de l'immunité, de la fonction barrière, de l'inflammation ou des cellules souches.

Par « marqueur de l'immunité », on entend ici tous les marqueurs qui servent à déterminer l'identité de l'organisme et à défendre celui-ci contre l'extérieur. Ces marqueurs servent de première ligne de défense contre les infections bactériennes. Un marqueur de l'immunité selon l'invention est la bêta-défensine 2 ou le Toll-like receptor 2.

La bêta-défensine 2 (BD-2) qui est aussi appelée skin-antimicrobial peptide 1 (SAP1) est un peptide codé par le gène DEFB4 (référence NCBI : Gene ID: 1673). La séquence de la bêta-défensine 2 humaine est disponible sous le numéro d'accession NP_004933., tandis que celle du gène DEFB4 l'est sous le numéro NM_004942.

La protéine Toll-like receptor 2 ou TLR2 (séquence NP_003255.2) est un récepteur membranaire, qui est exprimé sur la surface de certaines cellules. Elle reconnaît les substances étrangères, notamment les lipoprotéines bactériennes. L'activation du TLR2 conduit à la synthèse de cytokines, notamment l'interleukine 6. TLR2 est codé par le gène TLR2 (référence NCBI : Gene ID : 7097 ; séquence : NM_003264).

Les présents inventeurs ont montré dans les modèles de dermatite atopique mis en place, une diminution de l'expression des marqueurs de l'immunité innée. Il est connu dans la littérature que la peau dans la dermatite atopique présente un déficit de l'immunité innée : les peptides anti-microbiens tels que la bêta-défensine 2 sont déficients, tout comme le récepteur TLR2 dont l'expression au sein de l'épiderme est diminuée dans la dermatite atopique. (The Etiology of Atopic Dermatitis, Edition: 1st, Chapter: Chapter Two: Microbiology of Atopic Dermatitis, Publisher: Springer, Editors: Herbert B. Allen). La diminution de ces marqueurs dans les modèles mis en place permet donc de valider les modèles au regard de la physiopathologie de la dermatite atopique.

Les présents inventeurs ont par ailleurs montré dans les modèles de dermatite atopique mis en place, une augmentation de de l'expression des marqueurs de l'inflammation. Cette augmentation permet donc de valider les modèles au regard de la physiopathologie de la dermatite atopique.

Par « marqueurs de l'inflammation », on entend au sens de l'invention les marqueurs dont la variation d'expression corrèle avec l'inflammation cutanée.

On entend par « inflammation » selon l'invention l'ensemble des mécanismes réactionnels de défense par lesquels l'organisme reconnaît, détruit et élimine toutes les substances qui lui sont étrangères. « L'inflammation cutanée » correspond plus particulièrement à une réaction du système immunitaire en réaction à une agression sur la peau, telle qu'une agression de l'environnement, occasionnant ou non une plaie, voire un dommage vasculaire le cas échéant. L'inflammation cutanée s'accompagne d'une variation de niveau d'expression ou de concentration de marqueur géniques ou protéiques bien connus de l'homme du métier, qui pourra se référer par exemple à Vahlquist (Acta Derm Venereol ; 80: 161 ; 2000).

Le déclenchement et la poursuite de l'inflammation, sa diffusion à partir du foyer initial font appel à des facteurs qui sont synthétisés localement ou qui sont à l'état de précurseur inactif dans la circulation. Selon le type de médiateurs synthétisés, on peut différencier des processus particuliers dans la réaction d'inflammation. Ainsi, l'inflammation cutanée comprend en particulier la production en réponse à une agression extérieure de médiateurs inflammatoires protéiques, tels que les cytokines IL-1, IL-2, IL-6, IL8, TNFα et TSLP, le système du complément, ou les protéines impliquées dans la coagulation, le cas échéant. Les médiateurs protéiques vont induire une cascade de réactions au sein de la peau impliquant d'autres cellules de l'inflammation, en particulier, des cellules immunitaires et vasculaires. Le résultat clinique se traduit notamment par des rougeurs, voire un oedème.

De préférence, le médiateur de l'inflammation est choisi dans le groupe constitué par CCL2, CXCL1, CCL7, IL6, IL18, CCL3, CCL5, CCL7, KLK5 et TSLP.

CCL2 (séquence: NP_002973.1), CCL3 (séquence: NP_002974.1), CCL5 (séquence: NP_002976.2) et CCL7 (séquence : NP_006264.2) sont des cytokines de type chimiokine et plus particulièrement des chimiokines à motif C-C. Elles sont codées respectivement par les gènes CCL2 (référence NCBI : Gene ID : 6347 ; séquence : NM_002982.3), CCL3 (référence NCBI : Gene ID: 6348 ; séquence : NM_002983), CCL5 (référence NCBI : Gene ID : 6352 ; séquence : NM_002985) et CCL7 (référence NCBI : Gene ID : 6354 ; séquence : NM_006273). Par ailleurs, CXCL1 (séquence : NP_001502.1) est aussi une chimiokine, mais à motif C-X-C. Elle est codée par le gène CXCL1 (référence NCBI : Gene ID : 2919 ; séquence : NM_001511).

Les interleukines IL-6 et IL-8 humaines sont des cytokines pro-inflammatoires. La séquence protéique de la première correspond à la séquence de référence NCBI : NP_000591.1. Cette protéine est codée par le gène IL6 humain (référence NCBI : Gene ID : 3569). La séquence de celui-ci est accessible sous la référence NCBI : NM_000600. La protéine IL-8, quant à elle, possède une séquence correspondant à une référence NCBI choisie parmi : NP_001230140.1, NP_001553.1 et NP_001230140.1. Cette protéine est codée par le gène IL8 humain (référence NCBI : Gene ID : 3606). La séquence de celui-ci est accessible sous l'une des références NCBI : NM_001562.3 et NM_001243211.1.

La cytokine Thymic stromal lymphopoietin ou TSLP (séquence : NP_149024.1 ou NP_612561.2). Cette protéine, codée par le gène TSLP (référence NCBI : Gene ID : 85480) de séquence NM_033035 ou NM_138551, a un rôle important dans la maturation des populations cellules T à travers l'activation des cellules présentatrices d'antigènes et, en étant impliqué dans l'orientation Th2 des cellules T, constitue un facteur clé dans la physiopathologie de la dermatite atopique.

La kallikrein-related peptidase 5, aussi appelée stratum corneum tryptic enzyme, de séquence NP_036559.1, est codée par le gène KLK5 (référence NCBI : Gene ID : 25818) qui a lui-même la séquence NM_012427. Cette sérine protéase régule la desquamation en dégradant des protéines qui forment le composant extracellulaire des jonctions cellulaires du stratum corneum. L'augmentation de la desquamation correspond à une réduction de l'efficacité de la barrière, les jonctions étant moins étanches. Le niveau d'expression de la KLK5 ainsi que son activité sont surexprimés dans la peau de dermatite atopique, ce qui contribue à exacerber l'altération de la fonction barrière (desquamation) et également contribue à promouvoir les processus inflammatoires et l'induction du prurit via l'activation du récepteur PAR2.

Les présents inventeurs ont par ailleurs montré dans les modèles de dermatite atopique mis en place, une réduction de l'expression ou de la quantité des marqueurs de la fonction barrière et des marqueurs préférentiellement exprimés dans les cellules souches. L'altération de la fonction barrière est une caractéristique bien connue de la physiopathologie de la dermatite atopique. Ainsi, l'induction d'une réduction des marqueurs de la fonction barrière valide les modèles comme étant représentatifs de la dermatite atopique. En revanche, il n'avait jamais été montré auparavant que l'expression des marqueurs préférentiellement exprimés dans les cellules souches est inhibée, que ce soit dans un modèle de dermatite atopique ou même dans la physiopathologie de la dermatite atopique. Ce qui constitue donc une découverte et une caractéristique spécifique de la peau de dermatite atopique chez le bébé.

Les « marqueurs de la barrière » comprennent les marqueurs qui sont spécifiquement exprimés dans les couches de l'épiderme les plus externes et qui participent de la fonction barrière.

Comme il est bien connu de l'homme du métier, la peau a pour fonction principale d'établir une barrière de protection contre les atteintes de l'environnement tout en permettant certains échanges entre le milieu intérieur et le milieu extérieur. Cette fonction barrière est principalement portée par le *stratum corneum* de l'épiderme. Les lipides et les cornéodesmosomes intercellulaires ainsi que l'enveloppe cornée des cornéocytes en sont les constituants clés.

Cependant, sous le *stratum corneum,* les jonctions serrées constituent une seconde ligne de fonction barrière. Ces jonctions constituent au niveau du *stratum granulosum* une barrière de diffusion paracellulaire sélective prévenant de la pénétration de molécules délétères. Les jonctions serrées sont composées de différentes protéines transmembranaires comme notamment les claudines, l'occludine et ZO1.

Les fonctions de barrière portées par le *stratum corneum* et les jonctions serrées sont étroitement liées. En effet, l'altération de l'une ou l'autre peut influencer la formation de l'autre.

Préférentiellement, les marqueurs de la fonction barrière sont des marqueurs exprimés ou présents dans le *stratum corneum* ou des marqueurs exprimés ou présents dans les jonctions serrées du *stratum granulosum.* Dans l'invention, ledit marqueur de la barrière épidermique est sélectionné dans le groupe constitué de la kératine 1 (KRT1), la desmogléine 1 (DSG), l'involucrine (IVL), la scielline (SCEL), la shingomyélinase (SMPD1), la caspase 14 (CASP14), la loricrine (LOR), la filaggrine (FLG), la transglutaminase 1 (TGM1), la claudine 1 (CLDN1), les facteurs naturels d'hydratation (ou NMF pour Natural Moisturizing Factors) et les céramides.

Le gène KRT1 (Gene ID : 3848 ; NM_006121) code la kératine 1 (NP_006112.3). Tout comme la kératine 10 (NP_000412.3), laquelle est codée par le gène KRT10 (Gene ID : 3858 ; NM_000421), la kératine 1 est un constituant du réseau intracellulaire des kératinocytes et, à ce titre, participe à la structure générale du *stratum corneum.* Le gène BARX2 (Gene ID : 8538 ; NM_003658.4) code le facteur transcription Barx2 (NP_003649.2) qui régule l'adhésion pendant la formation de l'épiderme et la différenciation de celui-ci.

Le cornéodesmosome est la seule structure jonctionnelle de la couche cornée, ce qui souligne l'importance de cette structure pour le maintien de l'intégrité de la couche cornée.

La cornéodesmosine (CDSN) (Simon et al., J Biol Chem, 272 : 31770-31776, 1997; Simon et al., J Biol Chem, 276: 20292-20299, 2001) est un marqueur spécifique des cornéodesmosomes, car c'est la seule protéine spécifiquement localisée dans la partie extracellulaire des cornéodesmosomes (Jonca et al., The Open Dermatology Journal, 4: 36-45, 2010; Jonca et al., Eur J Dermatol, 21(Suppl 2): 35-42, 2011). Par cornéodesmosine, on fait ici référence à la protéine humaine ayant une séquence représentée par NP_001255, et codée par le gène CDSN (Gene ID : 1041 ; NM_001264).

La desmogléine 1 est une protéine constitutive des cornéodesmosomes dont la séquence est disponible sous la référence NP_001933. Le gène DSG1 (Gene ID : 1828) code la desmogléine-1 et possède la séquence de référence NM_001942. L'involucrine, dont la séquence peptidique est la séquence de référence NCBI : NP_005538.2, est exprimée dans les couches épineuses-granuleuses. C'est le premier précurseur de l'enveloppe cornée qui représente 5 à 15 % de l'enveloppe cornée, et sert de lien également avec l'enveloppe cornée lipidique. Elle est codée par le gène IVL (Gene ID : 3713), dont la séquence a pour référence NCBI : NM_005547.2. La scielline, de séquence NP_001154178, et codée par le gène SC (Gene ID : 8796) qui a lui-même la séquence correspondant à la référence NCBI : NM_001160706 est un précurseur de l'enveloppe cornée. La loricrine est un composant protéique majeur de l'enveloppe cornée, dont elle représente environ 70 % en masse. Cette protéine a pour séquence NP_000418 et est codée par le gène LOR (Gene ID : 4014) de séquence correspondant à la référence NCBI : NM_000427. La formation des liaisons entre les composants protéiques de l'enveloppe cornée est assurée par l'enzyme transglutaminase 1. La séquence de cette enzyme correspond à celle qui peut être trouvée sous la référence NP_000350. Le gène TGM1 (référence NCBI : Gene ID : 7051) code la desmogléine-1 et possède la séquence de référence NM_000359.

La shingomyélinase (SMase) ou sphingomyéline diestérase est une hydrolase impliquée dans le métabolisme des sphingolipides. Elle clive les sphingomyélines en phosphocholine et céramides 2 et 5, lesquels font partie de la matrice lipidique intercellulaire qui assure l'étanchéité de la couche cornée. La shingomyélinase est une protéine dont la séquence est représentée par la référence NCBI : NP_000534. Le gène codant cette enzyme est le gène SMPD (référence NCBI : Gene ID : 6609) de séquence correspondant à la référence NM_000543. La glucosylcéramidase (NP_000148.2), encore appelée β-glucosidase acide, β-glucocérébrosidase et D-glucosyl-N-acylsphingosine glucohydrolase, est une autre hydrolase impliquée dans le métabolisme des sphingolipides. Elle catalyse le clivage de la liaison entre l'unité céramide et le résidu de glucose des glucocérébrosides, participant ainsi à la synthèse des céramides. Cette enzyme est codée par le gène GBA (Gene ID : 2629), dont la séquence correspond à la référence NM_000157. Le gène LASS6 (Gene ID : 253782), aussi appelé CERS6, code la céramide synthétase 6 (NP_001243055.1) qui est, elle aussi, impliquée dans la synthèse des céramides. Le gène LASS6 a pour séquence la séquence représentée par NM_001256126.

Les jonctions serrées représentent un des modes d'adhésion cellulaires, dans les tissus épithéliaux, Elles bloquent la circulation de fluides entre les cellules et assurent ainsi l'étanchéité entre deux compartiments tissulaires. Elles sont localisées à l'apex des cellules épithéliales où elles forment une bande continue tout autour qui assure l'étanchéité. Le gène CLDN 1 (référence NCBI : Gene ID : 9076) code la protéine claudine 1 qui est un est un des composants les plus importants des jonctions serrées. Cette protéine a une séquence correspondant à celle dont la référence NCBI est NP_066924. La séquence du gène CLDN1 est accessible sous la référence NM_021101.

Les marqueurs de la barrière comprennent aussi des marqueurs du métabolisme des NMF et des lipides intercornéocytaires, tels que les céramides. Ces composés permettent de retenir l'eau dans l'épiderme au cours de son ascension vers la couche cornée. Dans la couche cornée, les lipides sont disposés en plan lamellaires dans les espaces entre les cornéocytes, formant ainsi un ciment qui participe à la protection de la peau contre les agressions extérieures et au maintien d'un bon niveau d'eau intraépidermique. Ces lipides sont des phospholipides, cholestérol et glucosylcéramides, qui sont modifiés dans les espaces intercornéocytaires, par des enzymes spécialisées, en céramides, cholestérol, sulfate de cholestérol et acides gras libres. (Jungerstend et al., Contact Dermitis, 58(5) : 255-262, 2008). Ainsi, les enzymes sphingomyélinase acide et beta glucocérébrosidase transforment les lipides intercornéocytaires en céramides 2 et 5 (pour la sphingomyélinase) et en céramides 1, 3, 4, 6, 7, 8 et 9 (pour la glucocérébrosidase).

Le facteur naturel d'hydratation, aussi appelé NMF (Natural Moisturizing Factor) est issu de la protéolyse de la filaggrine selon une cascade de réactions impliquant des enzymes dont notamment la caspase 14 et la peptidylarginine deiminase (PAD1). Le NMF est un mélange de substances hygroscopiques ayant la propriété de retenir l'eau (Fluhr et al., Exp Dermatol., 19(6) : 483-492, 2010). Parmi celles-ci, le sel de sodium de l'acide pyrrolidone carboxylique ou PCA Na (provenant de la cyclisation de l'acide glutamique libéré par la décomposition de la profilaggrine) et les lactates sont les substances les plus hygroscopiques. Le NMF comprend en outre des acides aminés libres (sérine, citrulline...), des citrates et des formiates, de l'urée, des ions, de l'azote, de l'acide urique, de la glycosamine, de la créatinine, des phosphates, ainsi que des composés non encore identifiés. La quantité de NMF peut être mesurée par toutes les méthodes connues de l'homme du métier, notamment par microspectroscopie de Raman.

La filaggrine (NCBI : NP_002007.1) est codée par le gène FLG humain (référence NCBI : Gene ID : 2312). La filaggrine s'agrège aux fibres de kératine du cytosquelette, réduisant ainsi les cornéocytes en disques aplatis, ce réseau intracellulaire confère résistance et protection au *stratum corneum.* D'autre part, sa dégradation conduit à la formation des constituants du NMF. La caspase-14 est un membre de la famille des caspases qui est nécessaire pour la dégradation de la filaggrine en NMF (Hoste et al., J Invest Dermatol. 131(11): 2233-2241, 2011). Cette protéine, de séquence NP_036246, est codée par le gène CASP14 (référence NCBI : Gene ID : 23581) dont la séquence se trouve sous la référence NM_012114.

La filaggrine et ses dérivés NMF sont particulièrement bien connus pour être déficients dans la peau de dermatite atopique et constituent un facteur majeur de la physiopathologie de la dermatite atopique, ce qui confirme que les modèles mis en place par les inventeurs reproduisent fidèlement la physiopathologie de la dermatite atopique.

Les « marqueurs préférentiellement exprimés dans les cellules souches » recouvrent les marqueurs, et plus spécifiquement les gènes et les protéines, qui sont spécifiquement présents dans les cellules souches épidermiques. Par « cellule souche de l'épiderme » ou « cellule souche épidermique », on entend une cellule de l'épiderme capable de renouvellement à long terme. Les cellules souches épidermiques comprennent, entre autres, les cellules souches folliculaires, les cellules souches sébacées et les cellules souches basales, ces dernières étant aussi appelées cellules souches épidermiques interfolliculaires. On entend par « cellules souches folliculaires », « cellules souches sébacées » et « cellules souches basales » les cellules souches situées respectivement dans la région du bulge/renflement du follicule pileux, dans les glandes sébacées et dans la couche basale de l'épiderme. Dans un mode préférentiel, les cellules souches épidermiques de l'invention sont des cellules souches basales.

Plus précisément, on entend par cellule souche épidermique une cellule dotée d'un potentiel élevé de renouvellement à long terme. Par « potentiel de renouvellement », on entend ici la capacité de subir au moins un cycle de division cellulaire. Un « potentiel élevé de renouvellement à long terme » représente donc la capacité d'une cellule d'entrer dans plusieurs cycles de division cellulaire successifs. Il est bien connu que les cellules différenciées de la peau ne sont pas capables d'effectuer plusieurs divisions successives (Fortunel et Martin, J Soc Biol, 202(1) : 55-65, 2008). Il est entendu ici que « successif » ne signifie pas « consécutif » et qu'il peut exister des périodes pendant lesquelles une cellule souche selon l'invention reste quiescente sans toutefois perdre son potentiel élevé de renouvellement à long terme.

La conservation d'un potentiel élevé de renouvellement à long terme s'exprime par une division asymétrique produisant deux cellules différentes. La première cellule fille est une cellule souche identique à la cellule souche mère, tandis que la seconde est une cellule d'amplification transitoire qui se divise de façon limitée sur une courte période puis entre dans le processus de différenciation. Avantageusement, les cellules souches épidermiques sont donc en outre capables de générer au moins un type de cellule épidermique par différenciation. Autrement dit, la cellule d'amplification transitoire est capable de donner au moins un type de cellule épidermique par différentiation. Préférentiellement, ladite cellule épidermique est un kératinocyte. Plus préférentiellement, la cellule d'amplification transitoire est capable de donner tous les types de cellules épidermiques par différentiation.

Préférentiellement, les marqueurs exprimés dans les cellules souches sont des marqueurs qui participent aux fonctions et à la protection des cellules souches. On citera par exemple les marqueurs ΔNp63, BIRC5 (survivine), FN1 (fibronectin 1), MCSP (Melanoma-associated Chondroitin Sulfate Proteoglycan), LRIG1 (Leucine-rich repeats and immunoglobulin-like domains protein 1), GJA1 (connexin 43), NID1 (nidogen 1), KRT15 (keratin 15), KRT19 (keratin 19), EGFR (epidermal growth factor receptor), CD71 (transferrin receptor), DSG3 (desmoglein 3), ITGB1BP1 (integrin beta1 binding protein), ITGA6 (integrin alpha 6), ITGB1 (integrin beta1) et ITGB4 (integrin beta 4) ou encore des marqueurs impliqués dans la signalisation et la régulation de l'activité des cellules souches comme Wnt/beta catenin, sonic hedgehog (SHH), NOTCH1 (Notch homolog 1, translocation-associated). Les marqueurs ΔNp63 et survivine sont des marqueurs de résistance à l'apoptose, ayant donc un rôle dans la survie des cellules souches. Les cytokératines 15 et 19 sont des marqueurs positifs des cellules souches, la cytokératine 15 étant un marqueur de survie de celles-ci. Le MCSP colocalise avec les intégrines dans les cellules qui ne se divisent pas, tandis que les intégrine beta1 (marqueur d'adhésion à la matrice extracellulaire de la membrane basale) et intégrine alpha 6 (constituant des hémidesmosomes marqueur de liaison des kératinocytes entre eux) sont des protéines de surface qui participent à la communication intercellulaire, régulent les processus de différenciation/prolifération, ainsi que l'interaction avec la niche. Le récepteur à la transférine CD71 est un marqueur de surface connu des cellules souches, qui est utilisé pour isoler, dans une population de cellules integrine-alpha6 positives, les cellules à haut pouvoir clonogéniques. Enfin, Lrig1 est un antagoniste du récepteur à l'Epidermal Growth Factor (EGFR), maintenant ainsi les cellules souches quiescentes, alors que le récepteur EGFR, qui est un marqueur dont l'absence caractérise les cellules souches, entraine au contraire les cellules dans la voie de la prolifération.

Dans l'invention, le marqueur préférentiellement exprimé dans les cellules souches de l'invention est choisi dans le groupe constitué des marqueurs KRT15 (kératine 15), KRT19 (kératine 19), NOTCH1 (Notch Homolog 1), BIRC5 (survivine), ITGA6 (intégrine alpha 6), ITGB1 (intégrine beta1) et ITGB4 (intégrine beta 4). Ces marqueurs sont bien connus de l'homme du métier. Les gènes KRT15 (référence NCBI : Gene ID : 3866), KRT19 (référence NCBI : Gene ID : 3880), NOTCH1 (référence NCBI : Gene ID : 4851), BIRC5 (référence NCBI : Gene ID : 332), ITGA6 (référence NCBI : Gene ID : 3655) et ITGB1 (référence NCBI : Gene ID : 3688) correspondent ainsi aux séquences représentées par les numéros d'accession Genbank suivants : NM_002276, NM_001012270, NM_017617, NM_015541, NM_000210, NM_002211 et NM_000213, respectivement. Les protéines kératine 15, kératine 19, Notch Homolog 1, survivine, intégrine alpha 6 et intégrine beta1 correspondent quant à elles aux séquences représentées par les numéros d'accession Genbank suivants : NP_002266, NP_002267, NP_060087, NP_001012270, NP_000201 et NP_002202, respectivement.

La méthode de l'invention est caractérisée en ce que :
- le marqueur de l'immunité est HBD2 ou TLR2,
- le marqueur de l'inflammation est CCL2, CXCL1, CCL7, IL6, IL18, CCL3, CCL5, CCL7, KLK5 ou TSLP,
- le marqueur de la fonction barrière est FLG, KRT1, KRT10, SCEL, BARX2, LOR, IVL, TGM1, DSG1, CDSN, CLDN1, CASP14, SMPD1, GBA, LASS6, NMF ou les céramides, ou
- le marqueur préférentiellement exprimé dans les cellules souches est KRT15, KRT19, NOTCH1, BIRC5, ITGA6, ITGB1 ou ITGB4.

Il sera par ailleurs évident à l'homme du métier que la méthode de l'invention permettra une évaluation de l'efficacité de la formulation ou de l'actif d'autant plus complète qu'un grand nombre de marqueurs de types différents seront utilisés.

Selon un mode de réalisation préféré, la méthode de l'invention comprend une étape de mesure du niveau d'expression d'une combinaison de marqueurs biologiques, dans l'étape d) des tests A et B, l'étape f) du test C, et l'étape e) du test D comprennent. Ladite combinaison selon l'invention comprend au moins deux marqueurs, lesdits marqueurs étant choisis dans au moins deux catégories différentes de marqueurs décrits-ci-dessus : marqueurs des activités inhibitrices de la physiologie bactérienne, de l'immunité, de l'inflammation cutanée, de la fonction barrière et des marqueurs préférentiellement exprimés dans les cellules souches. Selon un mode de réalisation préférée, ladite combinaison comprend plus de deux marqueurs. Selon un mode de réalisation plus préféré, chacun des marqueurs appartient à une catégorie distincte de marqueurs décrites ci-dessus. Il est aussi possible d'utiliser des combinaisons de marqueur telles que définies ci-dessus, dans lesquelles certaines classes de marqueurs sont représentées par plus d'un marqueur.

L'utilisation de combinaisons de marqueurs comprenant au moins un marqueur de chacun des différents types indiqués plus haut est particulièrement avantageuse.

Pour chacun des marqueurs biologiques décrits plus haut, le terme « niveau d'expression » se réfère à la concentration cellulaire dudit marqueur. Ainsi, le niveau d'expression des lipides, tels que les céramides, correspond à la concentration desdits lipides dans la cellule. De la même façon, le niveau d'expression d'une protéine correspond à la concentration de ladite protéine dans la cellule. Si le marqueur est un gène, le « niveau d'expression » au sens de l'invention correspond à la concentration cellulaire d'au moins un des produits du gène dudit marqueur. Plus précisément, le niveau d'expression dudit marqueur biologique correspond à la quantité ou à la concentration cellulaire du transcrit dudit gène ou de la protéine issue dudit transcrit. Selon un mode de réalisation préféré, le niveau d'expression dudit marqueur biologique correspond à la quantité ou à la concentration cellulaire du transcrit dudit gène. Selon un autre mode de réalisation, le niveau d'expression dudit marqueur biologique correspond à la quantité ou à la concentration cellulaire de la protéine issue dudit transcrit. Selon un autre mode de réalisation, le niveau d'expression dudit marqueur biologique correspond à la quantité ou à la concentration cellulaire du lipide.

Par la « mesure du niveau d'expression d'une combinaison de marqueurs biologiques », on entend au sens de la présente demande la mesure du niveau d'expression de chacun des marqueurs de la combinaison. L'expression d'un gène peut être mesurée par exemple au niveau nucléotidique, en mesurant la quantité de transcrits dudit gène, et peut aussi être mesurée par exemple au niveau peptidique, en mesurant par exemple la quantité des protéines issus desdits transcrits. Ainsi, par « mesure du niveau d'expression dudit gène » on entend au sens de l'invention la mesure de la quantité de produit du gène sous sa forme peptidique ou sous sa forme nucléotidique.

De façon générale, l'expression du marqueur biologique selon l'invention sera détectée *in vitro* à partir du modèle de peau reconstruite.

Dans un mode de réalisation particulier, la méthode de l'invention peut comprendre une ou plusieurs étapes intermédiaires entre l'obtention du modèle de peau reconstruite et la mesure de l'expression du marqueur biologique, lesdites étapes correspondant à l'extraction à partir dudit modèle de peau reconstruite d'un échantillon lipidique, d'un échantillon de NMF, d'un échantillon d'ARNm (ou de l'ADNc correspondant) ou d'un échantillon de protéine. Celui-ci peut ensuite être directement utilisé pour mesurer l'expression du marqueur. La préparation ou l'extraction d'ARNm (ainsi que la rétrotranscription de celui-ci en ADNc), de protéines, de lipides ou de NMF à partir d'un échantillon de cellules de peau ne sont que des procédures de routine bien connues de l'homme du métier.

Une fois qu'un échantillon d'ARNm (ou d'ADNc correspondant) ou de protéine est obtenu, l'expression du marqueur, au niveau soit des ARNm (c'est-à-dire dans l'ensemble des ARNm ou des ADNc présents dans l'échantillon), soit des protéines (c'est-à-dire dans l'ensemble des protéines présentes dans l'échantillon), peut être mesurée. La méthode utilisée pour ce faire dépend alors du type de transformation (ARNm, ADNc ou protéine) et du type d'échantillon disponible.

Quand l'expression du marqueur est mesurée au niveau de l'ARNm (ou d'ADNc correspondant), n'importe quelle technologie habituellement utilisée par l'homme du métier peut être mise en oeuvre. Ces technologies d'analyse du niveau d'expression des gènes, comme par exemple l'analyse du transcriptome, incluent des méthodes bien connues telles que la PCR (Polymerase Chain Reaction, si on part d'ADN), la RT-PCR (Reverse Transcription-PCR, si on part d'ARN) ou la RT-PCR quantitative ou encore les puces d'acides nucléiques (dont les puces à ADN et les puces à oligonucléotides) pour un plus haut débit.

Par « puces d'acides nucléiques », on entend ici plusieurs sondes d'acides nucléiques différentes qui sont attachées à un substrat, lequel peut être une micropuce, une lame de verre, ou une bille de la taille d'une microsphère. La micropuce peut être constituée de polymères, de plastiques, de résines, de polysaccharides, de silice ou d'un matériau à base de silice, de carbone, de métaux, de verre inorganique, ou de nitrocellulose.

Les sondes peuvent être des acides nucléiques tels que les ADNc (« puce à ADNc »), les ARNm (« puce à ARNm ») ou des oligonucléotides (« puce à oligonucléotides »), lesdits oligonucléotides pouvant typiquement avoir une longueur comprise entre environ 25 et 60 nucléotides.

Pour déterminer le profil d'expression d'un gène particulier, un acide nucléique correspondant à tout ou partie dudit gène est marqué, puis mis en contact avec la puce dans des conditions d'hybridation, conduisant à la formation de complexes entre ledit acide nucléique cible marqué et les sondes attachées à la surface de la puce qui sont complémentaires de cet acide nucléique. La présence de complexes hybridés marqués est ensuite détectée.

Ces technologies permettent de suivre le niveau d'expression d'un gène en particulier ou de plusieurs gènes voire même de tous les gènes du génome (full genome ou full transcriptome) dans un échantillon biologique (cellules, tissus...). Ces technologies sont utilisées en routine par l'homme du métier et il n'est donc pas besoin de les détailler ici. Des exemples de mises en oeuvre de l'invention basées sur l'analyse d'expression génique (puces à ADNc) et sur la PCR quantitative sont décrits dans la section expérimentale.

Alternativement, il est possible d'utiliser toute technologie actuelle ou future permettant de déterminer l'expression des gènes sur la base de la quantité d'ARNm dans l'échantillon. Par exemple, l'homme du métier peut mesurer l'expression d'un gène par hybridation avec une sonde d'acide nucléique marquée, comme par exemple par Northern blot (pour l'ARNm) ou par Southern blot (pour l'ADNc), mais aussi par des techniques telles que la méthode d'analyse sérielle de l'expression des gènes (SAGE) et ses dérivés, tels que LongSAGE, SuperSAGE, DeepSAGE, etc. Il est aussi possible d'utiliser des puces à tissu (aussi connues en tant que TMAs : « tissue microarrays »). Les tests habituellement employés avec les puces à tissu comprennent l'immunohistochimie et l'hybridation fluorescente in situ. Pour l'analyse au niveau de l'ARNm, les puces à tissu peuvent être couplées avec l'hybridation fluorescente in situ. Enfin, il est possible d'utiliser le séquençage massif en parallèle pour obtenir déterminer la quantité d'ARNm dans l'échantillon (RNA-Seq ou « Whole Transcriptome Shotgun Sequencing »). À cet effet, plusieurs méthodes de séquençage massif en parallèle sont disponibles. De telles méthodes sont décrites dans, par exemple, US 4,882,127 ; U.S. 4,849,077 ; U.S. 7,556,922 ; U.S. 6,723,513 ; WO 03/066896 ; WO 2007/111924 ; US 2008/0020392 ; WO 2006/084132 ; US 2009/0186349 ; US 2009/0181860 ; US 2009/0181385 ; US 2006/0275782 ; EP-B1-1141399 ; Shendure Et Ji, Nat Biotechnol., 26(10): 1135-45. 2008 ; Pihlak et al., Nat Biotechnol., 26(6) : 676-684, 2008 ; Fuller et al., Nature Biotechnol., 27(11): 1013-1023, 2009 ; Mardis, Genome Med., 1(4): 40, 2009 ; Metzker, Nature Rev. Genet., 11(1) : 31-46, 2010.

Quand l'expression du marqueur est mesurée au niveau protéique, il est possible d'employer des anticorps spécifiques, en particulier dans des technologies bien connues telles que l'immunoprécipitation, l'immunohistologie, le western blot, le dot blot, l'ELISA ou l'ELISPOT, les puces à protéines, les puces à anticorps, ou les puces à tissu couplées à l'immunohistochimie. Parmi les autres techniques qui peuvent être utilisées sont comprises les techniques de FRET ou de BRET, les méthodes de microscopie ou d'histochimie, dont notamment les méthodes de microscopie confocale et de microscopie électronique, les méthodes basées sur l'utilisation d'une ou plusieurs longueurs d'onde d'excitation et d'une méthode optique adaptée, comme une méthode électrochimique (les techniques voltammétrie et d'ampérometrie), le microscope à force atomique, et les méthodes de radiofréquence, comme la spectroscopie résonance multipolaire, confocale et non-confocale, détection de fluorescence, luminescence, chemiluminescence, absorbance, réflectance, transmittance, and biréfringence ou index de réfraction (par exemple, par résonance des plasmons de surface, ou « surface plasmon resonance » en anglais, par ellipsometry, par méthode de miroir résonnant, tec.), cytométrie de flux, imagerie par résonance radioisotopique ou magnétique, analyse par électrophorèse en gel de polyacrylamide (SDS-PAGE); par spectrophotométrie HPLC-Mass, par chromatographie liquide/spectrophotométrie de masse/spectrométrie de masse (LC-MS/MS). Toutes ces techniques sont bien connues de l'homme du métier et il n'est pas nécessaire de les détailler ici.

Si le marqueur biologique est un lipide, notamment un céramide, l'homme du métier pourra utiliser toutes les méthodes à sa disposition pour mesurer la teneur en lipide dans un échantillon de cellules cutanées. Ces méthodes comprennent, entre autres, la chromatographie liquide (HPLC, voir par exemple Sullivan et al., Arch Ophthalmol., 120(12) : 1689-99, 2002), par exemple couplée à un détecteur évaporatif à diffraction de la lumière (HPLC-ESD, voir Nordbäck et al., J. High Resolut. Chromatogr., 22 : 483-486, 1999 ; Torres et al., J. Chromatogr. A., 1078 : 28-34, 2005) ; la chromatographie en couche mince (TLC, par exemple Downing et al., J Invest Dermatol., 77(4) : 358-360, 1981; Nordstrom et al., J Invest Dermatol., 87(2) : 260-263, 1986) ; la résonance magnétique nucléaire (NMR, voir par exemple Robosky et al., J Lipid Res., 49(3) : 686-692, 2008) ; la microspectroscopie confocale *in vivo* de Raman ; la spectrométrie de masse, la chromatographie en phase gazeuse couplée à la spectrométrie de masse (GC-MS, voir O'Neill et al., J Chromatogr Sci., 14(1) : 28-36, 1976) ; la chromatographie en phase gazeuse couplée à un détecteur à ionisation de flamme ; la chromatographie en phase liquide couplée à la spectrométrie de masse (voir par exemple van Smeden et al., J Lipid Res, 52(6):1211-1221, 2011) ; la chromatographie en phase liquide ultra-performante (UPLC, voir Rainville et al., J Proteome Res., 6(2):552-558, 2007 ; Castro-Perez et al., J Proteome Res., 10(9) : 4281-4290, 2011). On peut également analyser l'organisation de ces lipides dans la peau et plus particulièrement dans le *stratum corneum* (ou couche cornée), organisation lamellaire ou latérale, par des techniques par exemple de diffraction aux rayons X (Bouwstra et al., J Invest Dermatol., 97(6): 1005-1012, 1991 ; van Smeden et al., J Lipid Res., 52(6):1211-1221, 1991) ou par spectroscopie infra-rouge à transformée de Fourier (Gorcea et al., Int J Pharm. Nov 10, 2011.) ou par une analyse morphométrique en microscopie électronique (Daehnhardt-Pfeiffer et al., Skin Pharmacol Physiol., 25(3): 155-161, 2012) ou par une analyse en microscopie électronique de section vitreuse de peau combinée à une analyse moléculaire (Iwai et al., J Invest Dermatol., Apr 26, 2012).

Le dosage du NMF est une procédure bien connue de l'homme du métier. Il est en particulier possible de doser le NMF par l'utilisation de microspectroscopie confocale *in vivo* de Raman. C'est une procédure couramment utilisée dans le domaine depuis au moins 15 ans. A titre d'exemple, on citera entre autres les publications de Caspers et al. (J Invest Dermatol., 116(3) : 434-442, 2001), Vyumvuhore et al. (J Biomed Opt., 19(11) : 111603, 2014), ou encore Falcone et al. (Skin Pharmacol Physiol, 28 : 307-317, 2015). Il est aussi possible de doser les NMF par la chromatographie en phase liquide couplée à la spectrométrie de masse. On se référera par exemple à Piraud et al. (Rapid Commun Mass Spectrom, 19(12):1587-602, 2005), Petritis et al. (Journal of Chromatography A, 833(2): 147-155, 1999), Henriksen et al. (J Am Soc Mass Spectrom, 16(4): 446-455, 2005) ou encore Yang (Application of biophysics and bioengineering to the assessment of skin barrier function. Thesis (Doctor of Philosophy (PhD)). University of Bath, UK, 2011).

Dans un mode de réalisation particulier, le niveau d'expression dudit marqueur biologique de l'étape d) du test A est comparé à un niveau d'expression de référence. Selon un autre mode de réalisation particulier, le niveau d'expression dudit marqueur biologique de l'étape d) du test B est comparé à un niveau d'expression de référence. Selon encore un autre mode de réalisation particulier, le niveau d'expression dudit marqueur biologique de l'étape f) du test C est comparé à un niveau d'expression de référence. Selon toujours un mode de réalisation particulier, le niveau d'expression dudit marqueur biologique de l'étape e) du test D est comparé à un niveau d'expression de référence.

Par « un niveau d'expression de référence d'un marqueur biologique », on entend au sens de la présente demande tout niveau d'expression dudit marqueur utilisé à titre de référence. Par exemple, un niveau d'expression de référence peut être obtenu en mesurant le niveau d'expression du marqueur d'intérêt dans un modèle de peau d'enfant, dans des conditions particulières. L'homme du métier saura choisir ces conditions particulières en fonction de son objectif lors de la mise en oeuvre de l'invention.

Selon un autre mode de réalisation, le niveau d'expression de référence d'un marqueur biologique est le niveau d'expression dudit marqueur obtenu dans un modèle de peau d'enfant, mis en contact avec une formulation ou l'actif de référence, et exposé à des conditions de dermatite atopique selon l'un au moins des tests A, B, C ou D décrits ci-dessus.

Lorsque le niveau d'expression de référence est un niveau d'expression obtenu dans un modèle de peau exposé à des conditions de dermatite atopique selon l'un au moins des tests A, B, C ou D, l'homme du métier comprendra aisément que les conditions de dermatite atopique du modèle de peau utilisé dans la méthode de l'invention et du modèle utilisé pour obtenir un niveau d'expression de référence sont préférentiellement identiques. Ainsi, préférentiellement, les conditions utilisées pour générer la dermatite atopique, par exemple la bactérie pathogène responsable de l'infection dans les tests C et D, ainsi que la durée de l'exposition utilisée dans la méthode de l'invention et du modèle utilisé pour obtenir un niveau d'expression de référence sont préférentiellement identiques.

Par exemple, l'homme du métier pourra utiliser comme formulation de référence toute formulation connue de l'art antérieur pour son effet dans la prévention ou le traitement de la dermatite atopique de la peau d'enfant.

### PRODUIT 1 : Emulsion huile/eau (H/E) type BAUME

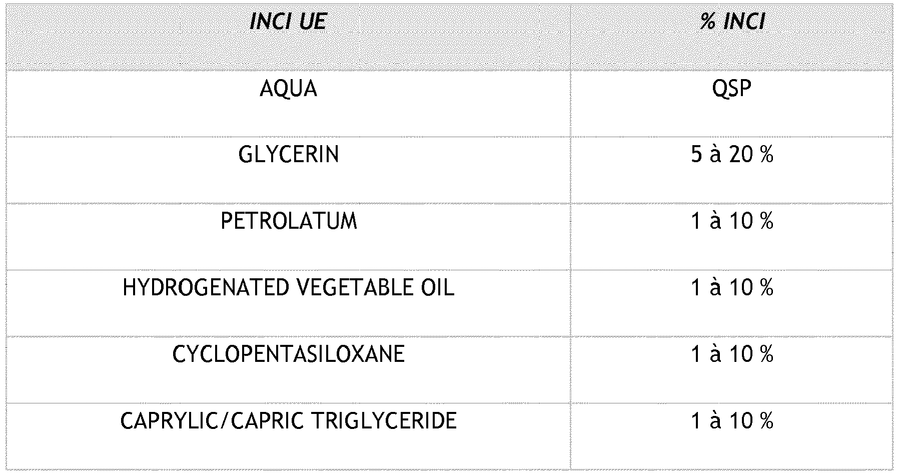

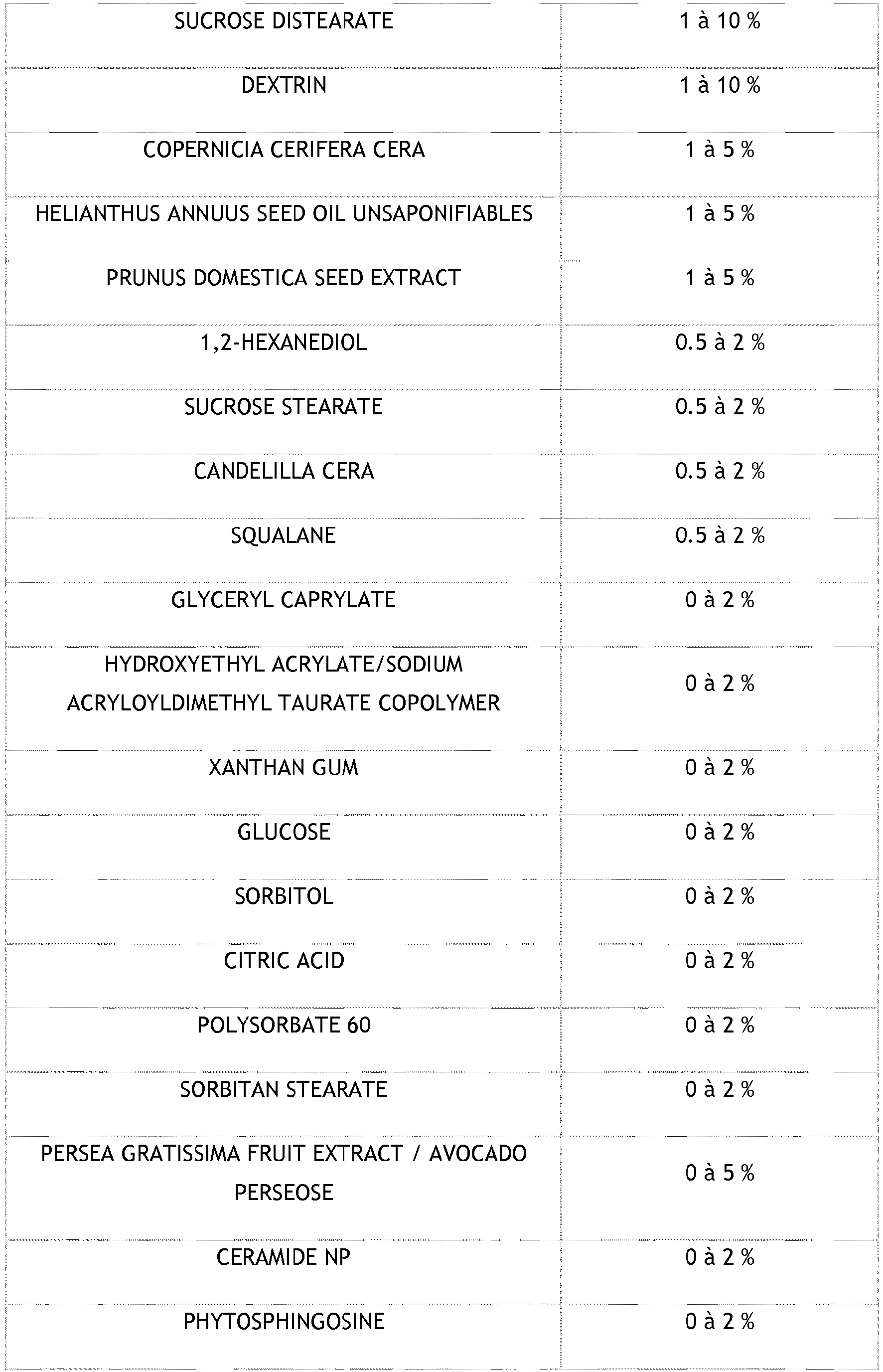

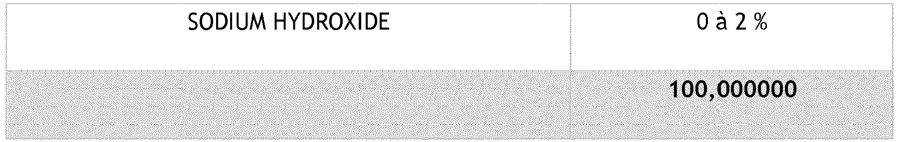

### PRODUIT 2 Emulsion H/E type Baume

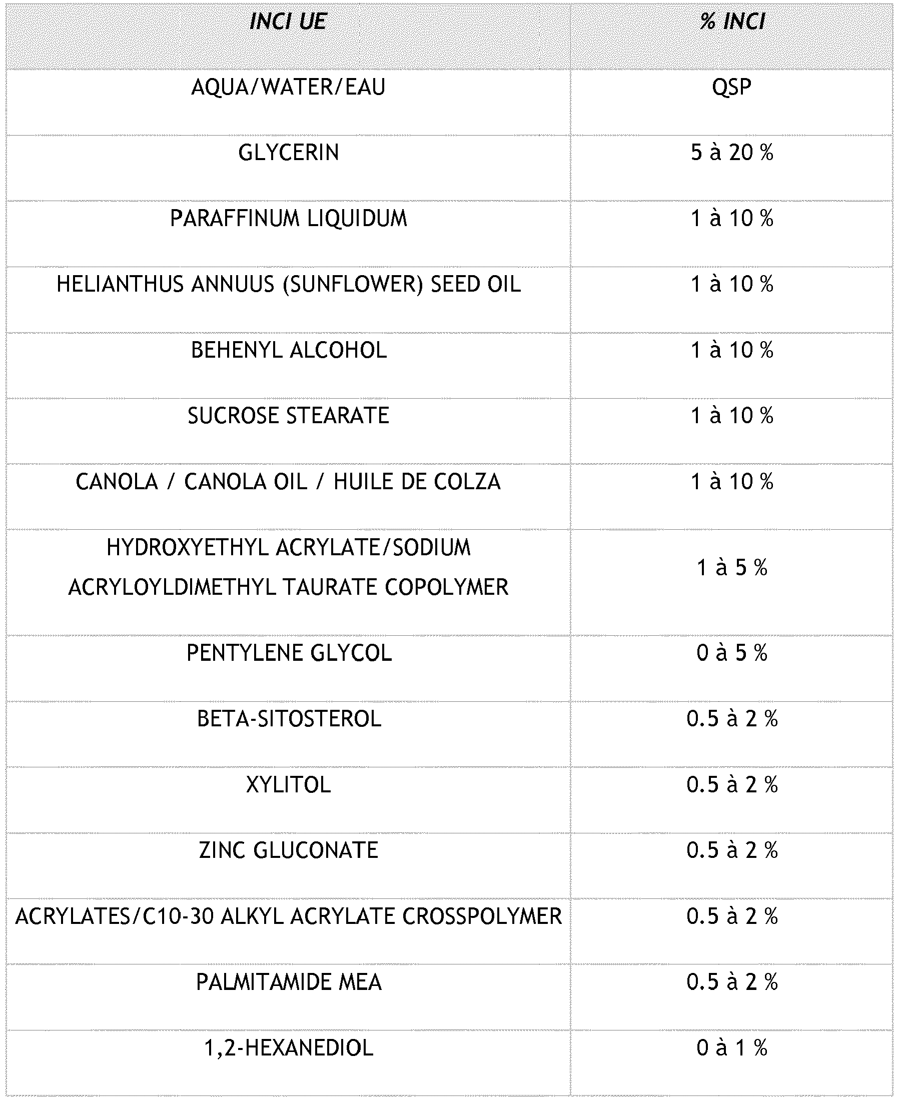

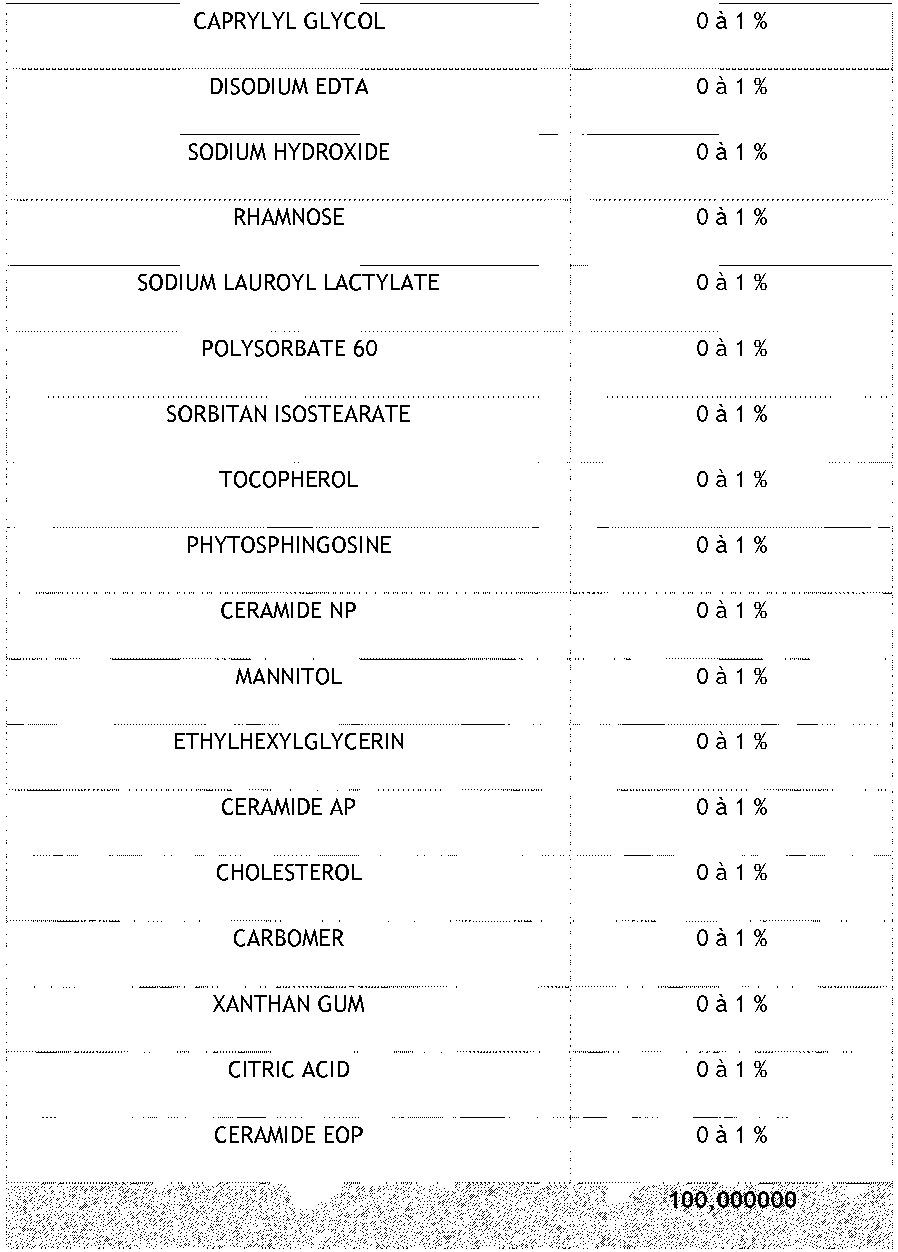

### PRODUIT 3 Emulsion H/E type Baume

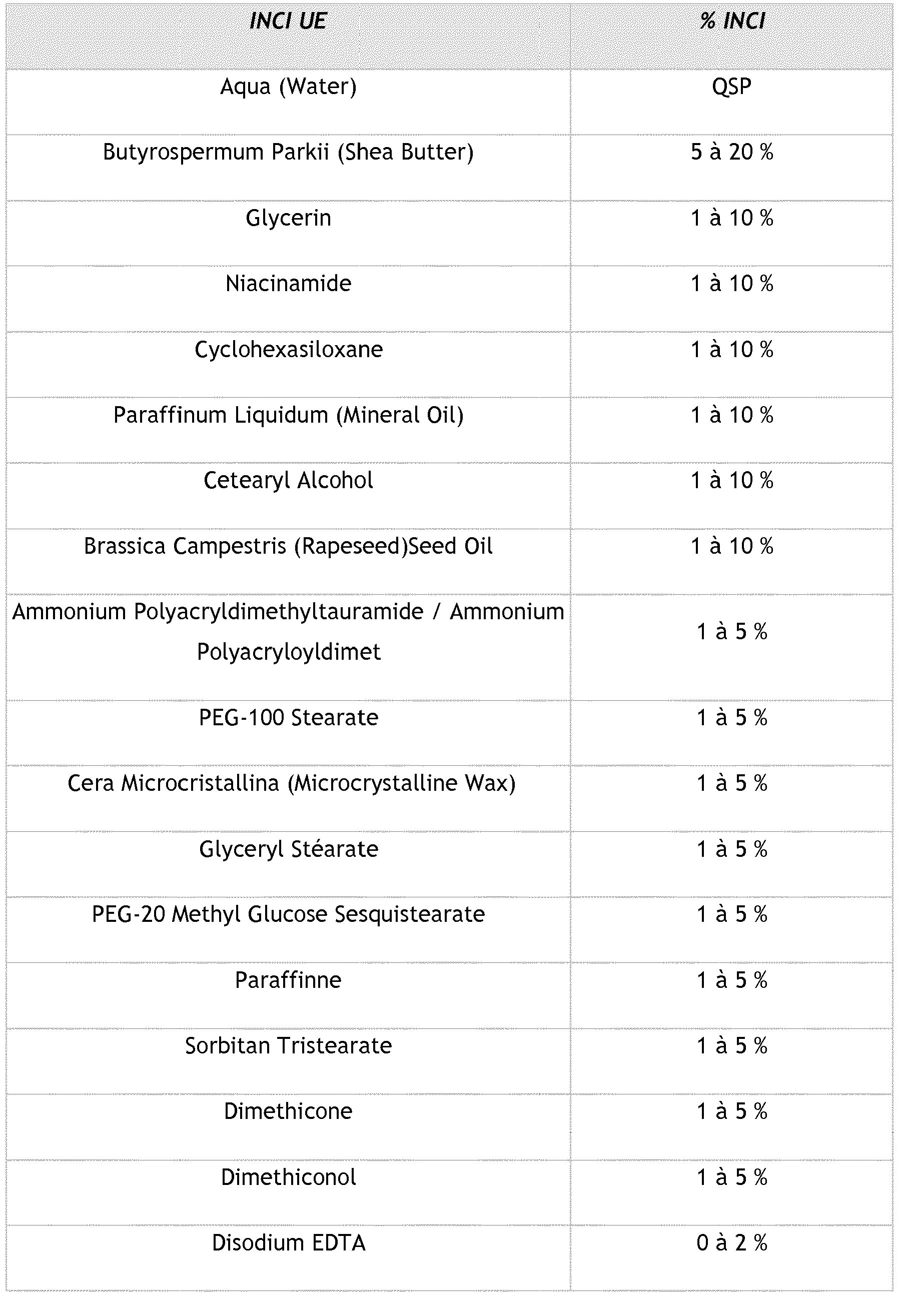

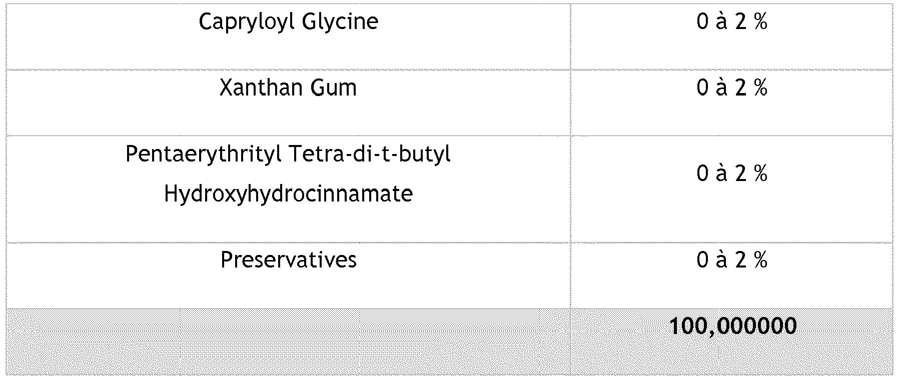

### PRODUIT 4 Emulsion H/E type baume

| Aqua | QSP |
|---|---|
| Mineal Oil (Paraffinum Liquidum) | 5 à 20 % |
| Butyrospermum Parkii (Shea Butter) (Butyrospermum Parkii Butter) | 1 à 10 % |
| Glycerin | 1 à 10 % |
| Polysorbate 60 | 1 à 10 % |
| Cetearyl Alcohol | 1 à 10 % |
| Dimethicone | 1 à 10 % |
| PEG-12 | 1 à 10 % |
| Oenothera Biennis (Evening Primerose) Oil (Oenothera Biennis Oil) | 1 à 5 % |
| Butylene Glycol | 1 à 5 % |
| Squalane | 1 à 5 % |
| Niacinamide | 0.5 à 2 % |
| 10-Hydroxydecenoid Acid | 0.5 à 2 % |
| Avena Sativa (Oat) Leaf/Stem Extract (Avena Sativa Leaf/Stem Extract) | 0.5 à 2 % |
| BHT | 0 à 2 % |
| C 13-14 Isoparaffin | 0 à 2 % |
| Cetearyl Glucoside | 0 à 2 % |
| Preservatives | 0 à 2 % |
| Disodium EDTA | 0 à 2 % |
| Laureth-7 | 0 à 2 % |
| Maltodextrin | 0 à 2 % |
| Polyacrylamide | 0 à 2 % |
| Sodium Acetate | 0 à 2 % |
| Tocopherol | 0 à 2 % |

### PRODUIT 5 : EMULSION H/E type Baume

| ***MP*** | % |
|---|---|
| AQUA | QSP |
| Eau thermale | 5 à 10 % |
| Huile de paraffine / synthèse | 1 à 10 % |
| Glycerine | 1 à 10 % |
| Glyceryl Stearate | 1 à 10 % |
| PEG-100 Stearate | 1 à 10 % |
| Alcool cetylique | 1 à 10 % |
| Cires minérales | 1 à 10 % |
| Huile végétale | 1 à 10 % |
| Ester | 1 à 10 % |
| Polysorbate 20 | 0.5 à 5 % |
| Parfum | 0.5 à 2 % |
| Gélifiant synthèse | 0.5 à 2 % |
| Ajusteur pH | 0 à 2 % |
| Antioxydant | 0 à 2 % |
| PRESERVATIVES | 0 % |
| ACTI FS | 0 à 5 % |

### PRODUIT 6 : Emulsion H/E type Baume

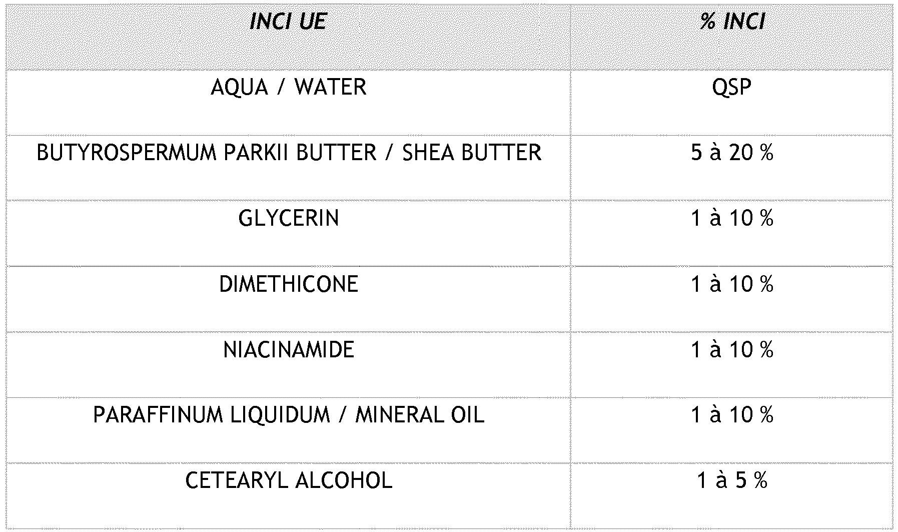

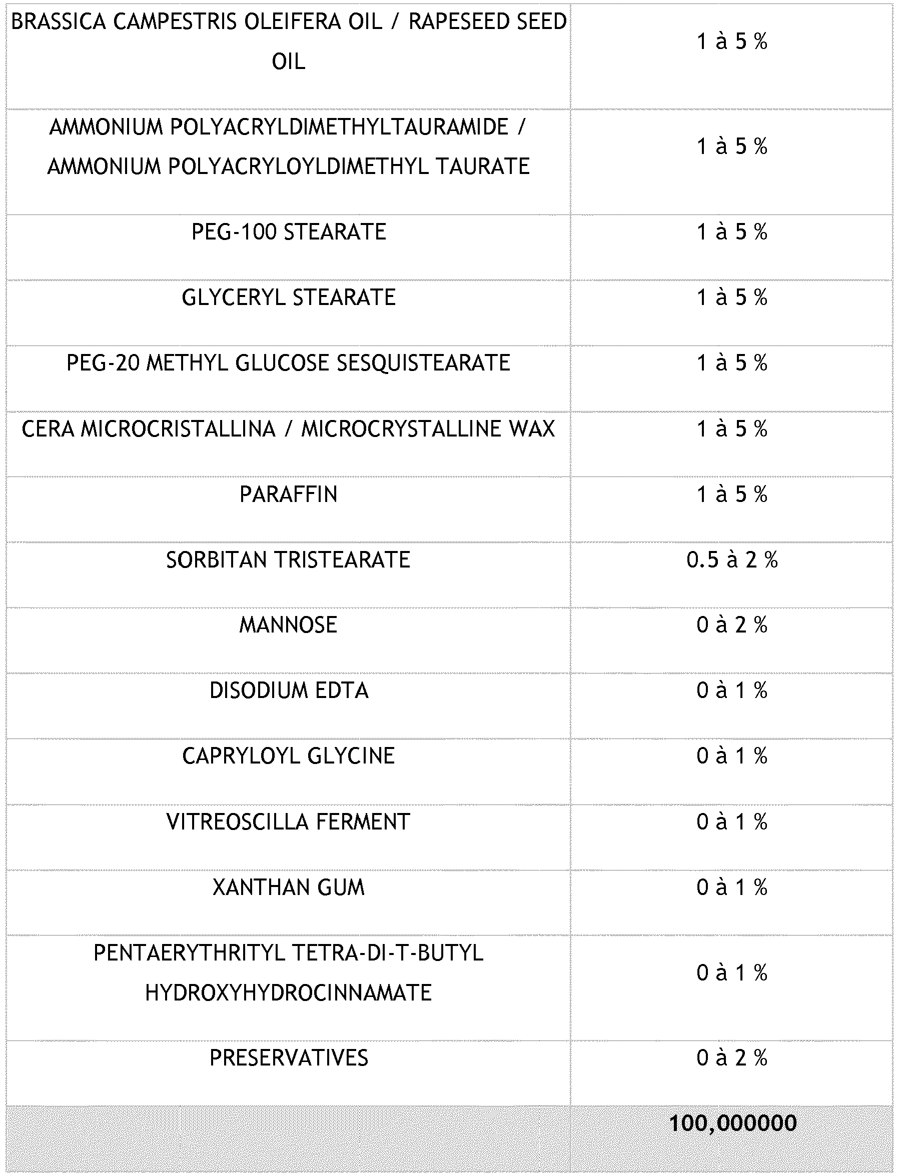

### PRODUIT 7 : Lait Atopie H/E

| INCI UE | % INCI |
|---|---|
| AQUA | QSP |
| PROPANEDIOL DICAPRYLATE | 5 à 20 % |
| CAPRYLIC/CAPRIC TRIGLYCERIDE | 1 à 10 % |
| C10-18 TRIGLYCERIDES | 1 à 10 % |
| GLYCERIN | 1 à 10 % |
| BUTYROSPERMUM PARKII | 1 à 10 % |
| EMULSIONNANT | 1 à 10 % |
| 1,2-HEXANEDIOL | 1 à 10 % |
| ACTIF | 0 à 5 % |
| HYDROXYETHYL ACRYLATE/SODIUM ACRYLOYLDIMETHYL TAURATE COPOLYMER | 0 à 2 % |
| JOJOBA ESTERS | 0 à 2 % |
| CAPRYLYL GLYCOL | 0 à 2 % |
| CETYL ALCOHOL | 0 à 2 % |
| CITRIC ACID | 0 à 2 % |
| TOCOPHERYL ACETATE | 0 à 2 % |
| CO-EMULSIONNANT | 0 à 2 % |

### PRODUIT 8 : Emulsion H/E type Crème émolliente

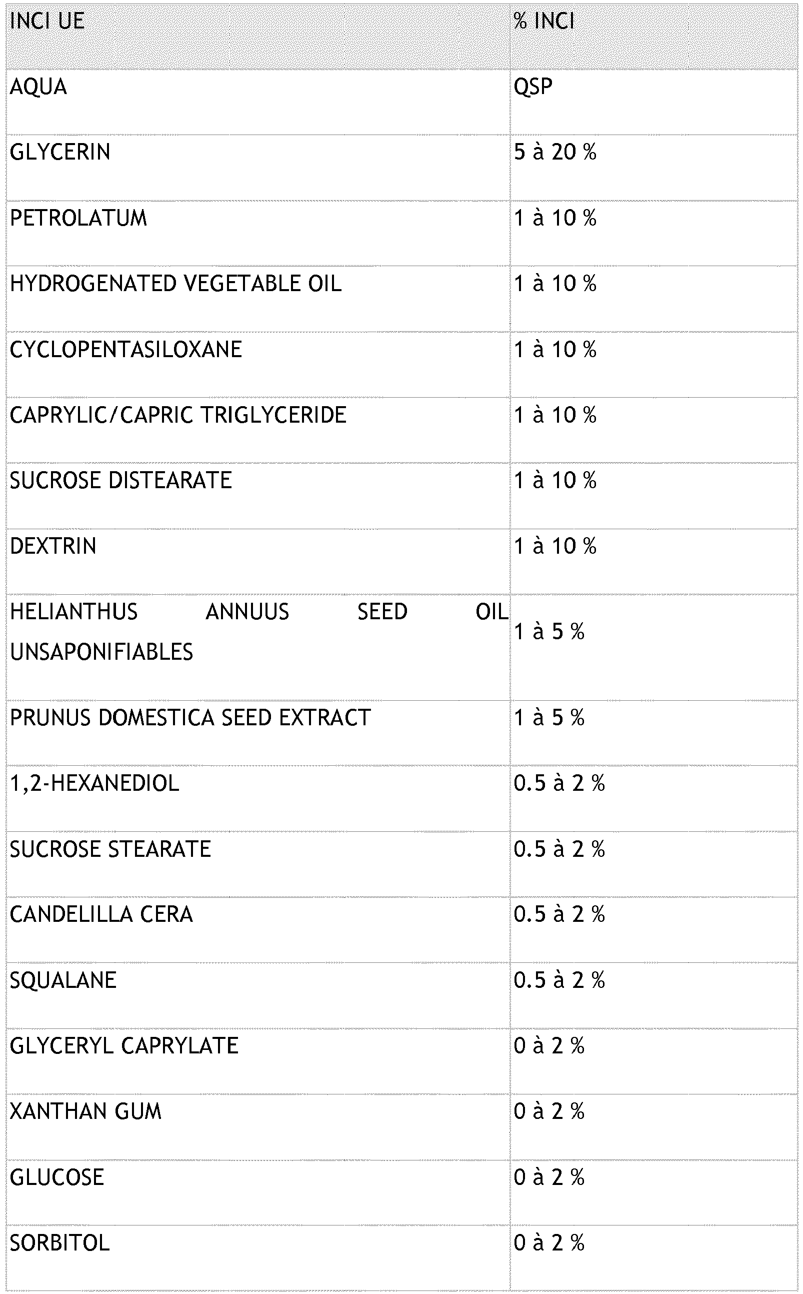

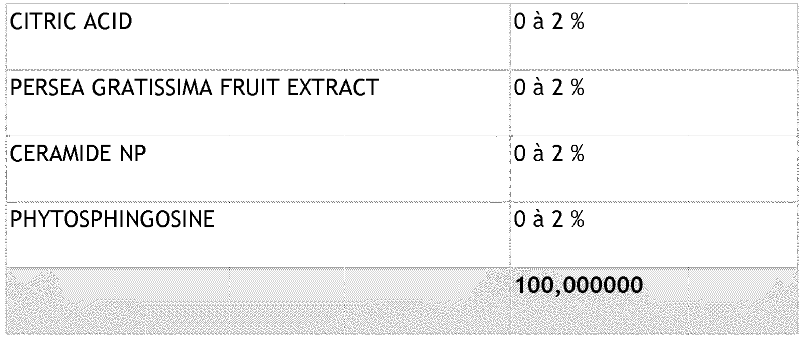

Selon un autre mode de réalisation préféré, le niveau d'expression de référence d'un marqueur biologique est le niveau d'expression dudit marqueur obtenu dans un modèle de peau obtenu à partir d'un échantillon cutané provenant d'un enfant, ledit modèle étant non traité avec la formulation ou l'actif d'intérêt, et n'étant ni exposé à des conditions entrainant une dermatite atopique selon l'un au moins des tests A, B, C et D.

Selon un autre mode de réalisation préféré, le niveau d'expression de référence d'un marqueur biologique est le niveau d'expression dudit marqueur obtenu dans un modèle de peau obtenu à partir d'un échantillon cutané provenant d'un enfant, ledit modèle étant non traité avec la formulation ou l'actif d'intérêt, mais ayant été exposé à des conditions entrainant une dermatite atopique selon l'un au moins des tests A, B, C et D.

Selon un autre mode de réalisation préféré, le niveau d'expression de référence d'un marqueur biologique est le niveau d'expression dudit marqueur obtenu dans un modèle de peau obtenu à partir d'un échantillon cutané provenant d'un enfant, ledit modèle étant non traité avec la formulation ou l'actif d'intérêt, mais ayant été exposé à des conditions entrainant une dermatite atopique selon l'un au moins des tests A, B, C et D.

Selon un autre mode de réalisation, le niveau d'expression de référence d'un marqueur biologique est le niveau d'expression dudit marqueur obtenu dans un modèle de peau obtenu à partir d'un échantillon cutané provenant d'un enfant, traité avec la formulation ou l'actif d'intérêt, et n'étant pas exposé à des conditions entrainant une dermatite atopique selon l'un au moins des tests A, B, C et D.

L'homme du métier comprendra en outre aisément que la comparaison de l'étape e) du test A, de l'étape e) du test B, de l'étape g) du test C ou de l'étape f) du test D est de préférence effectuée entre des mesures de niveaux d'expression obtenus pour des modèles de peau obtenus à partir d'échantillons cutanés provenant d'enfants, de structures histologiques similaires, voire identiques. Par « structures histologiques similaires », on entend au sens de la présente demande que les proportions relatives des types cellulaires compris dans les modèles de peau comparés sont similaires. Ainsi, il est préférable que les proportions relatives des types cellulaires compris dans le modèle de peau de l'étape a) de chacun de ces tests ne diffèrent pas de plus de 5 % des proportions relatives des types cellulaires compris dans le modèle de peau utilisé pour l'obtention du niveau d'expression de référence de l'étape e) du test A, de l'étape e) du test B, de l'étape g) du test C ou de l'étape f) du test D. Par « proportion relative d'un type cellulaire » on entend au sens de la présente demande le rapport du nombre de cellules correspondant à ce type cellulaire sur le nombre de cellules totales comprises dans le modèle de peau. Ainsi, il est par exemple préférable que la proportion de kératinocytes sur le nombre de cellules totales dans le modèle de peau de l'étape a) de chacun de ces tests ne diffère pas de plus de 5 % de la proportion de kératinocytes sur le nombre de cellules totales dans le modèle de peau utilisé pour l'obtention du niveau d'expression de référence de l'étape e) du test A, de l'étape e) du test B, de l'étape g) du test C ou de l'étape f) du test D. Par « structures histologiques identiques », on entend au sens de la présente demande que les proportions relatives des types cellulaires compris dans les modèles de peau comparés sont identiques. Au sens de la présente invention, les proportions relatives des types cellulaires compris dans le modèle de peau de mamelon de l'étape a) de chacun de ces tests sont identiques aux proportions relatives des types cellulaires compris dans le modèle de peau utilisé pour l'obtention du niveau d'expression de référence de l'étape e) du test A, de l'étape e) du test B, de l'étape g) du test C ou de l'étape f) du test D lorsqu'elles ne diffèrent pas de plus de 0,1 %. Avantageusement, la proportion de kératinocytes sur le nombre de cellules totales dans le modèle de peau de l'étape a) de chacun de ces tests ne diffère pas de plus de 0,1 % de la proportion de kératinocytes sur le nombre de cellules totales dans le modèle de peau utilisé pour l'obtention du niveau d'expression de référence de l'étape e) du test A, de l'étape e) du test B, de l'étape g) du test C ou de l'étape f) du test D.

L'homme du métier comprendra tout aussi aisément que la comparaison de l'étape e) du test A, de l'étape e) du test B, de l'étape g) du test C ou de l'étape f) du test D est de préférence effectuée entre des mesures de niveaux d'expression obtenus pour des modèles de peau qui sont de taille, de volume ou de poids similaires, voire identiques. Ainsi, il est préférable que la taille, le volume, ou le poids du modèle de peau de l'étape a) de chacun de ces tests ne diffère pas de plus de 5 % de la taille, du volume, ou du poids du modèle de peau utilisé pour l'obtention du niveau d'expression de référence de l'étape e) du test A, de l'étape e) du test B, de l'étape g) du test C ou de l'étape f) du test D. Plus préférentiellement, la taille, et le volume et le poids du modèle de peau de l'étape a) de chacun de ces tests ne diffèrent pas de plus de 5 % de la taille, du volume et du poids du modèle de peau utilisé pour l'obtention du niveau d'expression de référence de l'étape e) du test A, de l'étape e) du test B, de l'étape g) du test C ou de l'étape f) du test D. Encore plus préférentiellement, la taille, et le volume et le poids du modèle de peau de l'étape a) de chacun de ces tests ne diffèrent pas de plus de 0,1 % de la taille, du volume et du poids du modèle de peau utilisé pour l'obtention du niveau d'expression de référence de l'étape e) du test A, de l'étape e) du test B, de l'étape g) du test C ou de l'étape f) du test D.

Alternativement, si les modèles de peau diffèrent de plus de 5 % de par la taille, le volume et le poids, l'homme du métier pourra normaliser le niveau obtenu à l'étape c) et le niveau de référence de l'étape e) du test A, de l'étape e) du test B, de l'étape g) du test C ou de l'étape f) du test D.

Ce facteur de normalisation pourra par exemple être un marqueur physique directement accessible tel que la masse de cellules de l'échantillon, ou la masse d'un constituant cellulaire, comme la masse d'ADN cellulaire ou la masse de protéines cellulaires.

Il peut aussi être avantageux d'utiliser comme facteur de normalisation le niveau d'expression d'un gène qui est exprimé au même niveau dans toutes les cellules, ou presque, de l'organisme. En d'autres termes, selon un mode de réalisation particulier de la présente invention, on utilise comme facteur de normalisation le niveau d'expression d'un gène de ménage. Selon un autre mode de réalisation, le niveau obtenu à l'étape d) du test A, à l'étape d) du test B, à l'étape f) du test C ou à l'étape e) du test D, et le niveau de référence sont normalisés en utilisant le niveau d'expression, non pas des gènes de ménage, mais des protéines codées par ceux-ci. Un gène de ménage est un gène exprimé dans tous les types cellulaires, qui code une protéine ayant une fonction de base nécessaire pour la survie de tous les types cellulaires. Une liste de gènes de ménage humains peut être trouvée dans Eisenberg et al. (Trends in Genet, 19: 362-365, 2003). Les gènes de ménage selon l'invention incluent par exemple les gènes RPS28, GAPDH, B2M, TFRC, YWHAZ, RPLO, 18S, GUSB, UBC, TBP, GAPDH, PPIA, POLR2A, ACTB, PGK1, HPRT1, IP08 et HMBS. De préférence, le gène de ménage est le gène RPS28 (gene ID : 6234 ; NM_001031) code la protéine ribosomique S28 (NP_001022.1) ou le gène GAPDH (référence NCBI : Gene ID : 2597), de séquence NM_002046, qui code la glycéraldéhyde 3-phosphate déshydrogénase (NP_002037.2).

L'homme du métier pourra ainsi aisément évaluer l'efficacité de la formulation d'intérêt en fonction de la comparaison de l'étape e) du test A, de l'étape e) du test B, de l'étape g) du test C ou de l'étape f) du test D.

Les exemples suivants sont fournis ici à titre d'illustration et ne sont pas, sauf indication contraire, destinés à être limitatifs.

### LEGENDES DES FIGURES :

Figure 1 : Effet du produit P8 sur la formation du biofilm de *S*. *aureus* à la surface des épidermes, observé après 6h par microscopie électronique à balayage. A : Contrôle non traité ; Grossissement X 750. B : Contrôle non traité ; Grossissement X 10000. C : Produit P8 ; Grossissement X 750.
Figure 2 : Effet du produit P8 sur la formation du biofilm de *S*. *aureus* à la surface des épidermes, observé après 24h par microscopie électronique à balayage. A : Contrôle non traité ; Grossissement X 750. B : Contrôle non traité ; Grossissement X 10000. C : Produit P8 ; Grossissement X 750. D : Produit P8 ; Grossissement X 10000.
Figure 3 : Effet des produits P1 et P5 et du Xylitol sur la formation du biofilm de S. *aureus* à la surface des épidermes, observé après 24h par microscopie électronique à balayage à un grossissement X 10000 (A) ou X 20000 (B).

### EXEMPLES

### I. Modèle d'inflammation TH2

L'efficacité biologique de 6 produits (P1, P2, P3, P4, P5 et P8 : voir Produits 1 à 5 et 8 plus haut) a été évaluée dans un modèle de dermatite atopique induite par un mix de cytokines Th2 sur des épidermes de 6 mois.

### A. Matériel et méthodes

Les épidermes reconstruits ont été réalisés avec les kératinocytes d'un donneur de 6 mois.

La reconstruction des épidermes a été réalisée selon le modèle dérivé de la méthode de Poumay et al (Arch Dermatol Res 2004 ; 296: 203-11). Les épidermes ont été traités ou non (contrôle) en topique par les produits à l'essai à raison de 5 mg/cm². Après une pré-incubation de 24 heures, les épidermes ont de nouveau été traités par les produits à raison de 2 mg/cm² et stimulés, ou non, par le mélange de cytokines Th2 IL4/IL13/IL22/TNFα à 3 ng/ml.

Après 24 heures d'incubation, l'expression génique des marqueurs de la fonction barrière, des cellules souches et de l'inflammation listés dans le tableau 1 a été évaluée par qRT-PCR (PCR quantitative en temps réel).

**Tableau 1 : Classification et nom des gènes étudiés**

| Nom du cluster | Abréviation | Nom du gène |
|---|---|---|
| Housekeeping | RPS28 | Ribosomal protein 28S |
| | GADPH | Glyceraldehyde-3-phosphate dehydrogenase |
| Cellules souches | ITGB1 | Intégrine beta 1 |
| | KRT15 | Keratin15 |
| | KRT19 | Keratin 19 |
| | BIRC5 | Baculoviral IAP repeat-containing 5 or surviving (=Survivine) |
| | NOTCH1 | Notch Homolog 1 |
| Différenciation épiderme, Fonction barrière, Hydratation | FLG | Filaggrine |
| | KRT1 | Kératine 1 |
| | KRT10 | Kératine 10 |
| | SCEL | Scielline |
| | BARX2 | Barx Homeobox 2 |
| | LOR | Loricrine |
| | IVL | Involucrine |
| | TGM1 | Transglutaminase 1 |
| | DSG1 | Desmogléine 1 |
| | CDSN | Cornéodesmosine |
| | CLDN1 | Claudine 1 |
| | CASP14 | Caspase 14 |
| | SMPD1 | Sphingomyélinase |
| | GBA | Glucocerebrosidase |
| | LASS6 | Ceramide synthase |
| Inflammation | CCL2 | Chemokine (C-C motif) ligand 2 ou monocyte chimoattractant protein 1 (MCP1) |
| | CXCL1 | C-X-C motif chemokine ligand 1 |
| | CCL7 | Chemokine (C-C motif) ligand 7 ou Monocyte-specific Chemokine 3 (MCP3) |

Après 48 heures d'incubation, les quantités de facteurs naturels d'hydratation (ou NMF pour Natural Moisturizing Factors) et de céramides produits par les épidermes ont été évalués :

### Analyse des céramides :

Les lipides épidermiques ont été extraits par agitation des épidermes à partir d'un mélange de solvants organiques durant 2h à température ambiante. Un traitement par extraction de type solide/liquide a ensuite été réalisé afin d'isoler les céramides des autres lipides constitutifs des épidermes.

La présence de céramides présentant une base sphingoïde de type shingosine [S], dihydrosphingosine [DS] et phytosphingosine [P] avec une longueur de chaine paire allant de 16 à 22 atomes de carbone a été recherchée par une méthode LC/MS.

La teneur en céramides a été normalisée par la quantité de protéines totales (dosage BCA).

### Analyse des éléments du NMF :

Les épidermes reconstruits ont été extraits sous agitation 2h à température ambiante à partir d'un mélange aqueux en présence d'un tensioactif non ionique pour favoriser l'extraction des marqueurs d'intérêt.

Les catabolites de la filaggrine ont été dosés par une méthode LC/UV permettant de cribler l'acide urocanique (UCA) sous ses deux isomères (cis et trans) ainsi que l'acide L-pyrrolidone carboxylique (PCA).

La teneur en NMF a été normalisée par la quantité de protéines totales (dosage BCA).

### B. Résultats

### 1. Analyse de l'expression des gènes marqueurs de la barrière

Le déficit de la fonction barrière est l'un des paramètres clés de la physiopathologie de la dermatite atopique. Nous avons évalué le niveau d'expression de différents marqueurs de différenciation kératinocytaire et de la fonction barrière épidermique dans le modèle d'épidermes de nourrisson soumis à un stress Th2.

L'incubation des épidermes reconstruits de 6 mois en présence du mélange de cytokines Th2 a conduit à une forte inhibition de l'ensemble des marqueurs de la barrière étudiés (en moyenne, 61% d'inhibition ; tableau 2).

Ceci est en corrélation avec les données de la littérature et valide le modèle comme mimant l'altération de la fonction barrière induite par l'environnement inflammatoire Th2 dans le cadre de la physiopathologie de la dermatite atopique.

**Tableau 2 : Niveau d'expression génique des marqueurs de la barrière dans les épidermes de 6 mois traités par le mix de cytokines Th2**

| *Expression relative en % par rapport au Contrôle* | | |
|---|---|---|
| | Epidermes 6 mois contrôles | Epidermes 6 mois Th2 |
| FLG | 100 | 11 |
| KRT1 | 100 | 25 |
| KRT10 | 100 | 26 |
| SCEL | 100 | 29 |
| BARX2 | 100 | 36 |
| LOR | 100 | 25 |
| IVL | 100 | 31 |
| DSG1 | 100 | 30 |
| CDSN | 100 | 79 |
| CLDN1 | 100 | 63 |
| CASP14 | 100 | 26 |
| SMPD1 | 100 | 40 |
| GBA | 100 | 81 |
| LASS6 | 100 | 40 |
| Moyenne d'expression des marqueurs barrière (%) | 100 | 39 |

*La **filaggrine** (FLG), les **kératines 1 et 10** (KRT1, KRT10) et **BARX2** sont des marqueurs de la différenciation épidermique. D'autre part, la filaggrine, précurseur des NMF, est un marqueur clé impliqué dans la physiopathologie de la dermatite atopique.*

*La **loricrine** (LOR) et **l'involucrine** (IVL) sont des protéines constitutives de l'enveloppe cornée. La **scielline** (SCL) des est un précurseur de l'enveloppe cornée.*

*La **desmogléine 1** (DSG1) et la **cornéodesmosine** (CDSN) sont des protéines constitutives des cornéodesmosomes qui assurent la cohésivité des cornéocytes au sein de la couche cornée.*

*La **claudine** 1 (CLDN1) est une protéine constitutive des jonctions serrées qui jouent un rôle dans la fonction barrière de l'épiderme et sont décrites comme une seconde barrière contre les pertes en eau après le stratum corneum.*

*La **caspase 14** (CASP14) est une enzyme impliquée dans le processing de la filaggrine pour obtenir le NMF.*

*La **sphingomyélinase** (SMPD1), la **glucocerebrosidase** (GBA) et la **céramide synthase** (LASS6) sont des enzymes impliquées dans la synthèse des lipides épidermiques, notamment les céramides, clés pour assurer l'étanchéité de la barrière.*

Les produits testés ont induit une augmentation de l'expression génique des marqueurs clés de la barrière dans les épidermes de 6 mois incubés en condition de stress Th2 (tableau 3).

L'efficacité des produits sur l'expression des marqueurs de la barrière peut être classée comme suit : P1 >P5>P3>P2>P4.

**Tableau 3 : Niveau d'expression génique des marqueurs de la barrière dans les épidermes de 6 mois traités par le mix de cytokines Th2 - Effet des produits**

| *Expression relative en* % *par rapport au Contrôle Th2* | | | | | |
|---|---|---|---|---|---|
| | Claudine 1 | SMPD1 | Involucrine | TGM | *Moyenne* |
| Th2 | 100 | 100 | 100 | 100 | *100* |
| P1 | 639 | 346 | 1484 | 916 | *846* |
| P2 | 180 | 105 | 210 | 142 | 159 |
| P3 | 141 | 171 | 107 | 233 | *163* |
| P4 | 115 | 76 | 144 | 96 | *108* |
| P5 | 555 | 221 | 1767 | 635 | 795 |

### 2. Analyse de l'expression des gènes marqueurs de l'inflammation

L'incubation des épidermes de 6 mois en présence du mélange de cytokines Th2 a induit une importante surexpression des chimiokines CXCL1 (Chimiokine ligand 1), CCL2 (Chimiokine ligand 2) et CCL7 (Chimiokine ligand 7) (tableau 4).

Ces chimiokines, responsables du recrutement des cellules de l'inflammation dans la peau, jouent un rôle important dans l'amplification de la réponse inflammatoire dans le cadre de la dermatite atopique. Les chimiokines CXCL1 et CCL2 ont notamment été décrites comme étant surexprimées dans la peau atopique. Ainsi, ce modèle représente l'amplification de la réponse inflammatoire liée à l'environnement Th2 dans la dermatite atopique.

L'application topique des produits à l'essai a permis de moduler la surexpression des chimiokines induite par le stress Th2, l'efficacité des produits peut être classée comme suit : P5≥P1>P2>P3=P4.

**Tableau 4 : Niveau d'expression génique des marqueurs de l'inflammation dans les épidermes de 6 mois traités par le mix de cytokines Th2 - Effet des produits**

| *Expression relative en* % *par rapport au Contrôle* | | | | | | |
|---|---|---|---|---|---|---|
| | CCL2 | | CXCL1 | | CCL7 | |
| | *Niveau d'expression* | *% Inhibi tion* | *Niveau d'expression* | *% Inhibi tion* | *Niveau d'expression* | *% Inhibi tion* |
| Epidermes 6 mois contrôles | 100 | | 100 | | 100 | |
| Epidermes 6 mois Th2 | 9863 | | 3688 | | 2583 | |
| P1 | 99 | *-99%* | 2250 | *-39%* | 103 | -96% |
| P2 | 4636 | *-53%* | 1549 | *-58%* | 1653 | -36% |
| P3 | 3255 | *-67%* | 3688 | *0* | 1343 | -48% |
| P4 | 3057 | *-69%* | 3835 | *+4%* | 1188 | -54% |
| P5 | 0 | *-100%* | 996 | *-73%* | 129 | -95% |

| | | | | | | |
|---|---|---|---|---|---|---|
| ***CXCL1** (Chemokine ligand 1) : Chimiokine attractive pour les neutrophiles, surexprimée dans la dermatite atopique.* ***CCL2** ou MCP1 (Chemokine ligand 2) : Chimiokine attractive pour les monocytes et basophiles ; surexprimée dans la dermatite atopique, où elle est responsable du recrutement des précurseurs de cellules dendritiques.* ***CCL7** ou MCP3 (Chemokine ligand 7) : Chimiokine attractive pour les monocytes*/*macrophages.* | | | | | | |

### 3. Analyse de l'expression des gènes marqueurs de cellules souches

Les cellules souches de tissus en renouvellement permanent sont classiquement définies comme étant des cellules rares et relativement quiescentes. Elles possèdent une capacité unique d'auto-renouvellement et de régénération tissulaire leur permettant d'assurer l'homéostasie et l'intégrité du tissu dans lequel elles résident.

Parmi les cellules souches de l'épiderme, les cellules souches interfolliculaires situées dans la couche basale constituent le réservoir épidermique principal en cellules souches. Ces cellules résident dans un microenvironnement anatomique et fonctionnel, la niche, qui contribue à maintenir leurs caractéristiques, notamment quand les conditions physiologiques changent. Les cellules souches interfolliculaires et leurs niches sont impliquées dans le maintien de l'intégrité et la régénération de l'épiderme. Les cellules souches ne sont identifiables qu'en suivant plusieurs marqueurs. Nous avons ainsi évalué le niveau d'expression de différents marqueurs géniques caractéristiques des cellules souches dans le modèle d'inflammation Th2.

L'incubation des épidermes de 6 mois en présence du mélange de cytokines Th2 a induit une diminution importante (-39%) du niveau d'expression moyen du pool de marqueurs de cellules souches étudié (tableau 5).

C'est, à notre connaissance, la première fois qu'un impact de l'inflammation Th2 est observé sur les marqueurs des cellules souches. Ces observations tendent à confirmer la forte vulnérabilité du capital cellulaire dans les épidermes de nourrisson vis-à-vis d'une agression extérieure modélisée ici par un stress Th2 typique de la pathogénèse de la dermatite atopique.

**Tableau 5 : Niveau d'expression génique des marqueurs de cellules souches dans les épidermes de 6 mois traités par le mix de cytokines Th2**

| *Expression relative en* % *par rapport au Contrôle* | | |
|---|---|---|
| | Epiderme s 6 mois contrôles | Epiderme s 6 mois Th2 |
| ITGB1 | 100 | 49 |
| KRT15 | 100 | 86 |
| KRT19 | 100 | 89 |
| BIRC5 (SURVIVIN) | 100 | 47 |
| NOTCH1 | 100 | 35 |
| Moyenne d'expression du pool de marqueurs de Cellules Souches (%) | 100 | 61 |

L'application du produit P1, et plus modérément du produit P5, a permis de restaurer le niveau d'expression du pool de marqueurs de cellules souches à un niveau identique à celui du contrôle non traité (tableau 6). Les produits P2, P3 et P4 n'ont montré aucune efficacité protectrice du pool de marqueurs de cellules souches.

**Tableau 6 : Niveau d'expression génique du pool de marqueurs de cellules souches dans les épidermes de 6 mois traités par le mix de cytokines Th2 - Effet des produits**

| *Expression relative en % par rapport au Contrôle* | | | | | | | |
|---|---|---|---|---|---|---|---|
| | ITGB1 | KRT15 | KRT19 | BIRC5 | TP63 | NOTCH1 | *Moyenne Pool marqueurs* |
| Contrôle | 100 | 100 | 100 | 100 | 100 | 100 | *100* |
| Th2 | **49** | **86** | **89** | **47** | **91** | **35** | **66** |
| P1 | 141 | 34 | 185 | 43 | 48 | 157 | *101* |
| P2 | 61 | 29 | 105 | 22 | 109 | 54 | *63* |
| P3 | 53 | 55 | 56 | 10 | 56 | 47 | 46 |
| P4 | 57 | 15 | 91 | 39 | 65 | 37 | 51 |
| P5 | 86 | 23 | 230 | 58 | 51 | 109 | 93 |

### 4. Analyse des quantités de NMFs et de céramides

L'incubation des épidermes de 6 mois en présence du mélange de cytokines Th2 a induit une diminution significative de la quantité de NMFs (-52 %, p<0,001) et de céramides (-41 %, p<0,001 ; tableaux 7 et 8, respectivement).

Ceci est en corrélation avec les données de la littérature et confirme la validité du modèle pour mimer l'altération de la fonction barrière et des mécanismes de régulation de l'hydratation induite par l'environnement inflammatoire Th2 dans le cadre de la physiopathologie de la dermatite atopique.

Dans ces conditions de stress Th2, les produits P1 et P8 induisent une augmentation significative de la production de NMFs et de céramides par les épidermes reconstruits, avec un effet plus important du produit P1 (Tableaux 7 et 8).

**Tableau 7 : Quantification de la teneur en NMFs dans les épidermes reconstruits de 6 mois en condition de stress Th2 - Statistiques : Analyse de variance à un facteur suivie d'un test de Tukey**

| | NMF (∑ UCA et PCA en µg/mg de protéines) (moyenne ± déviation standard) | Evolution (%) |
|---|---|---|
| Epidermes 6 mois contrôles | 22,65 ± 1,878 | |
| Epidermes 6 mois Th2 | 10,84 ± 1,005 | - 52% p<0,001 |
| P1 | 16,5 ± 1,718 | +52% p<0,001 |
| P8 | 12,88 ± 0,582 | +19% p<0,05 |

**Tableau 8 : Quantification de la teneur en céramides dans les épidermes reconstruits de 6 mois en condition de stress Th2 - Statistiques : Analyse de variance à un facteur suivie d'un test de Tukey**

| | Céramides (UA/mg de protéines) (moyenne ± déviation standard) | Evolution (%) |
|---|---|---|
| Epidermes 6 mois contrôles | 148,4 ± 8,045 | |
| Epidermes 6 mois Th2 | 88,27 ± 7,125 | - 41% p<0,001 |
| P1 | 110,7 ± 7,322 | +25% p<0,05 |
| P8 | 100,6 ± 8,985 | +14% p<0,1 |

### C. Conclusion

Le traitement des épidermes reconstruits par un mélange de cytokines Th2 reproduit les caractéristiques physiopathologiques de la dermatite atopique liées à l'environnement inflammatoire Th2 : altération de la barrière cutanée, amplification de l'inflammation locale.

Appliqué à des épidermes obtenus à partir de kératinocytes d'un donneur de 6 mois, ce modèle constitue, à notre connaissance le premier modèle spécifique bébé mimant l'environnement inflammatoire de la dermatite atopique.

D'autre part, ce modèle a permis de mettre en évidence, pour la première fois l'altération possible du capital en cellules souches par l'environnement inflammatoire Th2.

Ce modèle permet en outre d'étudier de façon comparative l'activité biologique de produits topiques sur la protection des marqueurs de la barrière et des cellules souches ainsi que sur la modulation de la réponse inflammatoire.

### II. Modèle d'initiation de la dermatite atopique

L'efficacité biologique de 4 produits (P1, P4, P5, P6) a été évaluée dans un modèle reproduisant la phase d'initiation de la dermatite atopique sur des épidermes reconstruits issus d'un donneur de 6 mois stimulés par un mélange Poly (I:C) + IL1α.

Le Poly I:C est un agoniste du récepteur TLR3, l'IL1α est une cytokine pro-inflammatoire. Le stress induit par ce mélange de molécules mime la cascade de réactions induite par une agression bactérienne et aboutissant à l'induction d'une réponse inflammatoire de type Th2. Il s'agit donc d'un modèle reproduisant l'initiation d'une réponse inflammatoire caractéristique de la dermatite atopique.

### A. Matériel et méthodes

Des épidermes reconstruits ont été réalisés, comme décrit plus haut, avec les kératinocytes d'un donneur de 6 mois. Les épidermes ont été traités ou non en topique (à raison de 5 mg/cm²) par les produits à l'essai et pré-incubés pendant 24h. Les épidermes ont à nouveau été traités ou non en topique (à raison de 2 mg/cm²) par les produits et stimulés ou non par le mélange d'inducteurs : Poly(I:C) à 10 µg/ml + IL1α à 10 ng/ml.

Après 4h d'incubation, l'expression génique des marqueurs inflammatoires a été évaluée par qRT-PCR (PCR quantitative en temps réel) en n=2.

Le tableau 9 liste les gènes qui ont été étudiés.

**Tableau 9 : Classification et nom des gènes étudiés**

| Nom du cluster | Abréviation | Nom du gène |
|---|---|---|
| Housekeeping | RPS28 | Ribosomal protein 28S |
| | GADPH | Glyceraldehyde-3-phosphate dehydrogenase |
| Inflammation/Prurit | IL6 | Interleukine 6 |
| | IL18 | Interleukine 18 |
| | CCL3 | Chemokine (C-C motif) ligand 3 ou Macrophage Inflammatory Protein 1alpha (MIP1α) |
| | CCL5 | Chemokine (C-C motif) ligand 5 ou regulated on activation, normal T cell expressed and secreted (RANTES) |
| | CCL7 | Chemokine (C-C motif) ligand 7 ou monocyte-specific chemokine 3 (MCP3) |
| | KLK5 | Kallikréine 5 ou Stratum Corneum Trypsin-like Enzyme (SCTE) |

### B. Résultats : Analyse de l'expression des gènes marqueurs de l'inflammation et du prurit

La stimulation des épidermes de 6 mois par le stress Poly(I:C)+IL1α a induit une surexpression très importante des chimiokines CCL3, CCL5 et CCL7, impliquées dans le recrutement et l'activation des cellules inflammatoires (tableau 10). En particulier, les chimiokines CCL3 (ou MIP1α) et CCL5 (ou RANTES) sont décrites comme étant surexprimées dans la peau atopique et responsables de l'activation et du recrutement des cellules Th2. Ainsi, ce modèle mime effectivement la phase d'initiation de la cascade inflammatoire qui conduit à l'installation de l'environnement inflammatoire Th2 spécifique de la dermatite atopique.

D'autre part, le stress Poly(I:C)+IL1α a également induit une surexpression de la kallikréine 5 .Cette protéase, dont l'activité est accrue dans la dermatite atopique, joue un rôle dans le contrôle de la desquamation, de l'inflammation et du prurit (*via* activation du PAR2) ; contribuant ainsi à la pathogénèse de la dermatite atopique liée à l'altération de la barrière et l'induction de l'inflammation et du prurit.

Les différents produits testés ont entraîné une diminution plus ou moins nette de l'expression génique des marqueurs de l'inflammation et du prurit ; le potentiel d'activité des produits peut être classé comme suit : P1>P5>P4≥P6. Seul le produit P1 a montré une efficacité inhibitrice sur l'ensemble des marqueurs étudiés.

**Tableau 10: Niveau d'expression génique des marqueurs de l'inflammation et du prurit**

| *Expression relative en %* | | | | | | |
|---|---|---|---|---|---|---|
| | IL6 | IL18 | CCL3 | CCL5 | CCL7 | KLK5 |
| Contrôle | nd | nd | 100 | 100 | 100 | 100 |
| Polyl:C+IL1 | **100** | **100** | **5532** | **9280** | **8462** | **136** |
| P1 | **12** | **54** | **664** | **3248** | **508** | **95** |
| P4 | 246 | 95 | **2213** | 9373 | **6770** | 140 |
| P5 | 188 | **65** | **2600** | **7610** | **4400** | 167 |
| P6 | 114 | 138 | **3872** | 12806 | **6600** | 155 |

| | | | | | | |
|---|---|---|---|---|---|---|
| ***IL6** (Interleukine 6) : cytokine pro-inflammatoire* ***IL18** (Interleukine 18) : cytokine surexprimée dans la peau atopique, impliquée dans l'induction de la réponse Th2* ***CCL3** ou MIP1α (Chemokine ligand 3) : Chimiokine attractive pour les cellules inflammatoires ; surexprimée dans la dermatite atopique.* ***CCL5** ou PANTES (Chemokine ligand 5) : Chimiokine attractive pour les cellules inflammatoires ; surexprimée dans la dermatite atopique.* ***CCL7** ou MCP3 (Chemokine ligand 7) : Chimiokine attractive pour les monocytes*/*macrophages.* ***KLK5** ou SCTE (Kallikréine 5) : protéase surexprimée dans la dermatite atopique, impliquée dans la desquamation, l'inflammation et le prurit.* | | | | | | |

### C. Conclusion

Le traitement des épidermes de 6 mois par le mélange poly(I:C) / IL1α permet effectivement de modéliser la phase d'induction de la réponse Th2 dans la dermatite atopique. En effet, ce stress a entraîné une surexpression des chimiokines et messagers moléculaires impliqués dans le recrutement et l'activation des cellules Th2.

Ce modèle d'initiation de la phase inflammatoire de la dermatite atopique a permis d'évaluer et de classer l'efficacité biologique de différents produits topiques sur les marqueurs de l'inflammation et du prurit.

### III. Modèle de réponse au Staphylococcus aureus sur épidermes immunocompétents

L'efficacité biologique des produits P1 et P5 a été évaluée dans un modèle mimant la dermatite atopique induite par *S*. *aureus* sur des épidermes reconstruits de 1 an rendus immunocompétents par co-culture avec des cellules immunitaires (lignée de monocytes THP1). La co-culture avec les cellules immunes permet d'établir une réponse au *S*. *aureus* adaptative et protectrice immunomédiée par induction d'une réponse de type Th2.

### A. Matériel et méthodes

Des épidermes reconstruits obtenus avec des kératinocytes provenant d'un donneur de 1 an ont été infectés par une souche Methicillin-Resistant *S*. *aureus* (MRSA ; ATCC 33591) à raison de 2.10⁶ cfu/épiderme pendant 4 heures, après avoir subi une légère abrasion de leur surface. Les bactéries non adhérentes ont alors été éliminées par rinçage et les épidermes ont été placés en co-culture avec les cellules THP1 (10⁵ cellules/ml). Dans le même temps, les produits à l'essai ont été appliqués en surface des épidermes (à raison de 20µl/épiderme). Après 16h de co-culture (T1), l'expression génique de différents marqueurs a été évaluée par qRT-PCR (PCR quantitative en temps réel) en n=3.

Sur une seconde série d'épidermes, les produits ont été réappliqués et les épidermes incubés pendant 24h en l'absence des cellules THP1. A la fin de cette période d'incubation, l'expression génique de différents marqueurs a été évaluée par qRT-PCR (PCR quantitative en temps réel) en n=3 (T2).

Le tableau 11 liste les gènes qui ont été étudiés.

**Tableau 11 : Classification et nom des gènes étudiés**

| | | |
|---|---|---|
| Nom du cluster | Abréviation | Nom du gène |
| Housekeeping | GADPH | Glyceraldehyde-3-phosphate dehydrogenase |
| Immunité Innée | HBD2 | Human Beta-Defensin 2 |
| | TLR2 | Toll-Like Receptor 2 |
| Inflammation TH2 | TSLP | Thymic Stromal Lymphopoietin |
| Barrière/Prurit | KLK5 | Kallikréine 5 ou Stratum Corneum Trypsin-like Enzyme (SCTE) |
| Barrière | CLDN1 | Claudin-1 |
| Cellules souches | K19 | Keratin 19 |
| | ITGA6 | Integrin alpha 6 |
| | ITGB1 | Integrin beta 1 |

### B. Résultats

### 1. Evaluation de la réponse des épidermes

### - T1 : Phase d'induction de la Dermatite Atopique

Après 16 heures de co-culture (T1) avec les cellules immunitaires, l'analyse de l'expression des gènes dans les épidermes de 1 an a montré une surexpression de la kallikréine-5, de la β-défensine 2 et de la TSLP, ainsi qu'une diminution du TLR2 (Tableau 12).

La stimulation de la kallikréine-5 serait le signe d'une réponse précoce de l'épiderme en faveur de l'induction de la phase inflammatoire Th2 (via la TSLP), de l'altération de la barrière et du prurit.

La TSLP est, elle-même, modérément augmentée, témoignant d'un début d'induction de l'inflammation Th2.

La stimulation de la β-défensine 2, peptide anti-microbien altéré dans la dermatite atopique, montre que dans cette phase précoce, l'épiderme met en place des mécanismes de défense pour empêcher la colonisation par le *S*. *aureus.*

Le TLR2, récepteur impliqué dans la reconnaissance de pathogènes et l'induction de la réponse immune dont l'expression est diminuée dans la dermatite atopique, est diminué dans le modèle, ce qui tend à montrer l'incapacité de la peau à se défendre, avec un déficit de cet élément important de l'immunité innée.

Ainsi, dans cette phase précoce d'induction du modèle, les signaux moléculaires liés à la réponse immune semblent se mettre en place pour induire le shift Th2 caractéristique de la dermatite atopique.

**Tableau 12: Niveau d'expression génique des marqueurs dans le modèle d'épidermes « immunocompétents » colonisés par S aureus**

| *(T1, Phase précoce, 16h post colonisation)* | | | | |
|---|---|---|---|---|
| | KLK5 | hBD2 | TLR2 | TSLP |
| Contrôle | 1 | 1 | 1 | 1 |
| S. aureus + THP1 | 1.425 | 1.469 | 0.308 | 1.299 |

### - T2 : Phase modélisant la Dermatite Atopique établie

Après 16 heures de co-culture puis 24 heures d'incubation, la réponse des épidermes à la colonisation par le *S*. *aureus* reproduit les caractéristiques principales de la physiopathologie de la dermatite atopique (tableau 13) :
Une altération de la barrière, représentée par l'inhibition de la claudine-1, protéine constitutive des jonctions serrées ;
Une inhibition des mécanismes de défense liés à l'immunité innée : hBD2 et TLR2 ;
Une surexpression de la TSLP, cytokine clé de la physiopathologie de la dermatite atopique, impliquée dans l'induction d'une réponse inflammatoire de type Th2.

De plus, une nette diminution des marqueurs des cellules souches (Kératine 19, Intégrines α6 et β1) a été observée, de façon comparable aux résultats obtenus dans le modèle Th2 décrit plus haut.

**Tableau 13 : Niveau d'expression génique des marqueurs dans le modèle d'épidermes « immunocompétents » colonisés par S aureus**

| *(T2, Phase tardive, 16*+*24h post colonisation)* | | | | | | | |
|---|---|---|---|---|---|---|---|
| | CLDN1 | KRT19 | ITGA6 | ITGB1 | hBD2 | TLR2 | TSLP |
| Contrôle | 1 | 1 | 1 | 1 | 1 | 1 | 1 |
| S. aureus + THP1 | 0,576 | 0,412 | 0,05 | 0,129 | 0,047 | 0,201 | 2,446 |

### 2. Evaluation de l'efficacité biologique des produits

### - Protection du capital en cellules souches

L'application topique du produit P1 a protégé, de façon nettement supérieure au produit P5, les marqueurs de cellules souches de l'inhibition induite dans le modèle (tableau 14).

**Tableau 14 : Niveau d'expression génique des marqueurs des cellules souches dans le modèle d'épidermes « immunocompétents » colonisés par S aureus - Effet des produits**

| *(T2, Phase tardive, 16*+*24h post colonisation)* | | | | |
|---|---|---|---|---|
| | Contrôle | S. aureus + THP1 | P1 | P5 |
| KRT19 | 1 | 0,412 | 3,14356 | 0,615528 |
| ITGA6 | 1 | 0,05 | 0,18585 | 0,43795 |
| ITGB1 | 1 | 0,129 | 0,57663 | 0,651063 |
| Moyenne pool de marqueurs | **1,00** | **0,20** | **1,30** | **0,57** |

### - Modulation d'un marqueur du prurit

Les deux produits testés (P1 et P5) ont inhibé de façon comparable le niveau d'expression de la kallikréine 5 pour restaurer un niveau comparable au contrôle (tableau 15).

Les deux produits modulent l'induction de ce marqueur précoce, impliqué dans le prurit, et qui contribue à l'installation de la dermatite atopique.

**Tableau 15 : Niveau d'expression génique de la Kallikréine 5 dans le modèle d'épidermes « immunocompétents » colonisés par S aureus - Effet des produits**

| *(T1, Phase précoce, 16h post colonisation)* | | | | |
|---|---|---|---|---|
| | Contrôle | S. aureus + THP1 | P1 | P5 |
| KLK5 | 1,00 | 1,43 | 1,05 | 0,92 |

### - Restauration d'un marqueur de défense immune

Les deux produits testés, P1 et P5, ont induit une stimulation du TLR2, récepteur impliqué dans la réponse immune et dont l'expression est altérée dans la dermatite atopique (tableau 16).

Le produit P1 a induit une augmentation supérieure du TLR2, par rapport au produit P5, en faveur d'une efficacité supérieure pour la protection des mécanismes liés aux défenses immunes innées.

**Tableau 16 : Niveau d'expression génique du TLR2 dans le modèle d'épidermes « immunocompétents » colonisés par S aureus - Effet des produits**

| | Contrôle | S. aureus + THP1 | P1 | P5 |
|---|---|---|---|---|
| T1 (16h) | 1,00 | 0,31 | 0,64 | 0,44 |
| T2 (16h + 24h) | 1,00 | 0,20 | 3,04 | 0,71 |

### - Modulation de l'inflammation TH2

Dans la phase précoce du modèle (T1), les deux produits P1 et P5 ont inhibé de façon similaire l'expression de la TSLP induite par le modèle (tableau 17).

Dans la phase tardive modélisant la dermatite atopique installée (T2), seul le produit P1 a inhibé l'expression génique de la TSLP.

Ainsi, *via* son action inhibitrice de la TSLP, le produit P1 pourrait moduler l'induction de l'inflammation TH2.

**Tableau 17 : Niveau d'expression génique de la TSLP dans le modèle d'épidermes « immunocompétents » colonisés par S aureus - Effet des produits**

| | Contrôle | S. aureus + THP1 | P1 | P2 |
|---|---|---|---|---|
| T1 (16h) | 1,00 | 1,30 | 0,92 | 1,04 |
| T2 (16h + 24h) | 1,00 | 2,45 | 1,35 | 2,89 |

### C. Conclusion

Ce modèle unique, utilisant des épidermes reconstruits d'un nourrisson de 1 an en co-culture avec des cellules immunocompétentes, permet de reproduire le rôle du S. aureus dans la physiopathologie de la dermatite atopique.

L'utilisation de ce modèle permet de valoriser comparativement l'activité biologique de produits topiques sur la protection des cellules souches, de la barrière et des défenses immunes, ainsi que sur la modulation du prurit et de l'inflammation TH2.

### IV. Modèle d'étude du Biofilm de S. aureus

L'efficacité biologique des produits P1, P5 et P8 vis-à-vis du biofilm de *S*. *aureus* a été évaluée dans un modèle d'épidermes reconstruits colonisés par *S*. *aureus* et sur lesquels, la formation du biofilm, a été visualisée en microscopie électronique.

L'efficacité biologique des produits P1,P5 ou P8 vis-à-vis du biofilm de *S*. *aureus* a été évaluée dans un modèle d'épidermes reconstruits colonisés par *S*. *aureus* et sur lesquels, la formation du biofilm, a été visualisée en microscopie électronique.

### A. Matériel et méthodes

Les produits à l'essai (P1, P5 ou P8), ou le xylitol à 5% (témoin positif d'inhibition de la formation du biofilm) ont été appliqués topiquement sur des épidermes reconstruits à raison de 30µL/épiderme, après que ceux-ci ont subi une légère abrasion de leur surface.

Après incubation sur la nuit, les produits ont été éliminés par rinçage et un inoculum de 2.10⁶ cfu de *Staphylococcus aureus* (Methicillin-Resistant *S*. *aureus,* MRSA ; ATCC 33591) a été appliqué à la surface des épidermes reconstruits.

La formation du biofilm et l'effet du produit sur celle-ci, a été observée visuellement en microscopie électronique à balayage (MEB) 6 et 24 heures après colonisation.

### B. Résultats

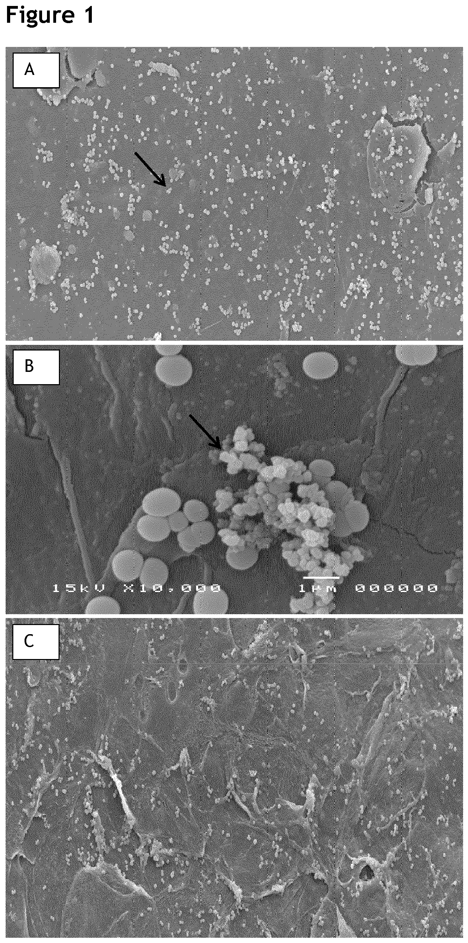

Dès 6 heures après la colonisation par le *S*. *aureus,* les bactéries planctoniques recouvrent la surface des épidermes (figure 1A), et produisent une matrice polysaccharidique caractéristique de l'initiation de la formation du biofilm (figure 1B).

L'application du produit P8 exerce un effet anti-adhésion du *S*. *aureus,* visualisé par la quantité diminuée de bactéries présentes à la surface des épidermes (figure 1C).

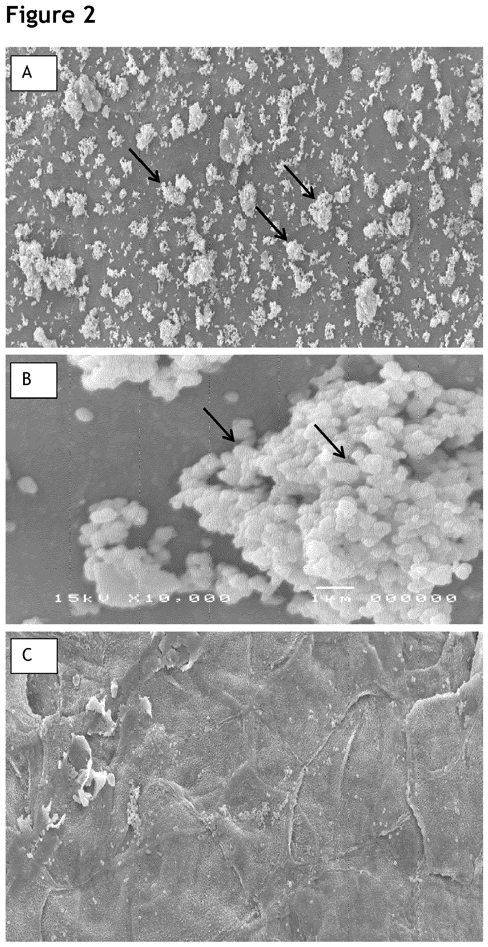

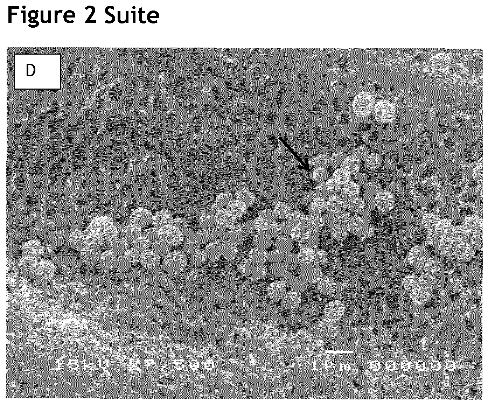

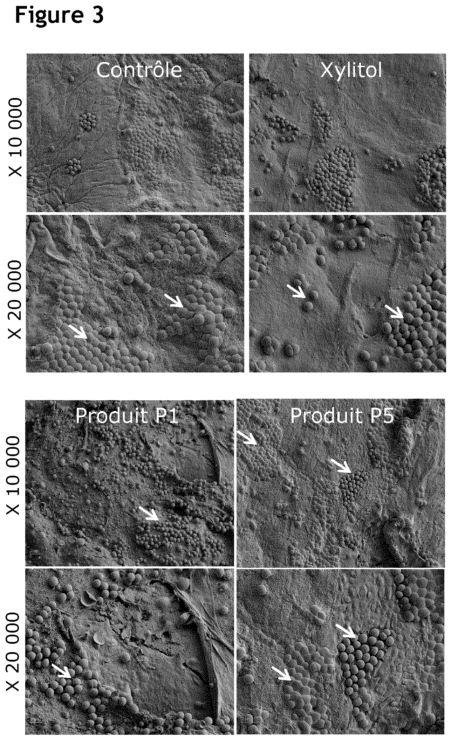

Après 24 heures de colonisation (figures 2 et 3), les bactéries sont observées en nombre à la surface des épidermes.

Dans les conditions contrôles (épidermes non traités), un biofilm dense est visualisé (flèches ; figures 2A et 2B).

Dans les épidermes témoins traitées par le xylitol, les bactéries apparaissent bien individualisées. Dans ces conditions, les bactéries n'ont pas produit de biofilm, ce résultat valide l'essai (figure 3).

En présence des produits P1 (figure 3) et P8 (figures 2C et 2D), on observe à la surface des épidermes des bactéries (coccis) bien individualisées et qui ne sont pas enveloppées dans une matrice exopolymérique comme dans les conditions contrôle non traité. Ces observations suggèrent que l'application topique des produits P1 et P8 ont inhibé la formation du biofilm de S. aureus. Et, particulièrement pour le produit P8, un effet anti-adhésion de *S*. *aureus* est observé (figures 1C, 2C, 2D).

Le produit P5, quant à lui, semble induire une inhibition partielle de la formation du biofilm. En effet, à la surface des épidermes on observe quelques zones où les bactéries apparaissent bien individualisées (absence de biofilm) ; tandis que la majorité de la surface de l'épiderme présente des bactéries recouvertes d'un biofilm.

### C. Conclusion

Ce modèle permet de suivre en microscopie électronique la cinétique de formation du biofilm de *S*. *aureus* à la surface d'épidermes et d'évaluer comparativement l'efficacité de produits topiques à limiter ce biofilm, facteur de virulence et de pathogénèse de la bactérie.

Via ce modèle il est possible de classer des produits selon leur efficacité à inhiber le biofilm de *S*. *aureus* : inhibition totale, avec effet anti-adhésion (ex. produits P1, P8) ou partielle (ex. produit P5).

## Revendications

1. Méthode d'évaluation de l'efficacité *in vitro* d'un actif cosmétique, d'un émollient ou d'une formulation pour prévenir ou traiter les effets de la dermatite atopique affectant la peau d'enfant, ladite méthode comprenant la détermination de l'efficacité dudit actif cosmétique, dudit émollient ou de ladite formulation dans chacun des quatre tests A, B, C et D et ladite méthode étant **caractérisée en ce que** :
• le test A comprend les étapes suivantes :
a) obtenir un modèle de peau reconstruite à partir d'un échantillon cutané provenant d'un enfant ;
b) mettre en contact ledit actif cosmétique, ledit émollient ou ladite formulation avec le modèle de peau reconstruite de l'étape a) ;
c) mettre en contact le modèle de peau reconstruite de l'étape b) avec une solution comprenant de acide poly(déoxyinosinique-déoxycytidylique) et de l'interleukine 1 alpha (IL1α) ;
d) mesurer le niveau d'expression d'au moins un marqueur biologique dans le modèle de peau de l'étape c) ; et
e) évaluer l'efficacité dudit actif cosmétique, dudit émollient ou de ladite formulation en fonction du niveau de l'étape d) ;
• le test B comprend les étapes suivantes :
a) obtenir un modèle de peau reconstruite à partir d'un échantillon cutané provenant d'un enfant ;
b) mettre en contact ledit actif cosmétique, ledit émollient ou ladite formulation avec le modèle de peau reconstruite de l'étape a) ;
c) mettre en contact le modèle de peau reconstruite de l'étape b) avec une solution comprenant au moins deux cytokines Th2 ;
d) mesurer le niveau d'expression d'au moins un marqueur biologique dans le modèle de peau de l'étape c) ; et
e) évaluer l'efficacité dudit actif cosmétique, dudit émollient ou de ladite formulation en fonction du niveau de l'étape d) ;
• le test C comprend les étapes suivantes :
a) obtenir un modèle de peau reconstruite à partir d'un échantillon cutané provenant d'un enfant ;
b) cultiver le modèle de peau reconstruite de l'étape a) en présence de monocytes THP-1 ;
c) mettre en contact ledit actif cosmétique, ledit émollient ou ladite formulation avec le modèle de peau reconstruite de l'étape b) ;
d) induire une altération de la fonction barrière dans le modèle de peau reconstruite de l'étape c) ;
e) mettre en contact le modèle de peau reconstruite de l'étape d) avec au moins une bactérie pathogène ;
f) mesurer le niveau d'expression et/ou d'activation d'au moins un marqueur biologique dans le modèle de peau de l'étape e) ; et
g) évaluer l'efficacité dudit actif cosmétique, dudit émollient ou de ladite formulation en fonction du niveau de l'étape f) ; et
• le test D comprend les étapes suivantes :
a) obtenir un modèle de peau reconstruite à partir d'un échantillon cutané provenant d'un enfant ;
b) mettre en contact ledit actif cosmétique, ledit émollient ou ladite formulation avec le modèle de peau reconstruite de l'étape a) ;
c) induire une altération de la fonction barrière dans le modèle de peau reconstruite de l'étape b) ;
d) mettre en contact le modèle de peau reconstruite de l'étape c) avec au moins une bactérie pathogène ;
e) mesurer le niveau d'expression et/ou d'activation d'au moins un marqueur biologique dans le modèle de peau de l'étape d) ; et
f) évaluer l'efficacité dudit actif cosmétique, dudit émollient ou de ladite formulation en fonction du niveau de l'étape e) ;
dans laquelle ledit marqueur est choisi parmi :
• les marqueurs des activités inhibitrices de la physiologie bactérienne, dans lesquels l'activité inhibitrice de de la physiologie bactérienne est choisie parmi l'inhibition de la prolifération bactérienne et l'inhibition de la formation du biofilm ;
• les marqueurs de l'immunité, ledit marqueur de l'immunité étant HBD2 ou TLR2 ;
• les marqueurs de l'inflammation, ledit marqueur de l'inflammation étant CCL2, CXCL1, CCL7, IL6, IL18, CCL3, CCL5, CCL7, KLK5 ou TSLP ;
• les marqueurs de la fonction barrière, ledit marqueur de la fonction barrière étant FLG, KRT1, KRT10, SCEL, BARX2, LOR, IVL, TGM1,DSG1, CDSN, CLDN1, CASP14, SMPD1, GBA, LASS6, NMF ou les céramides ; et
• les marqueurs préférentiellement exprimés dans les cellules souches, ledit marqueur préférentiellement exprimé dans les cellules souches étant KRT15, KRT19, NOTCH1, BIRC5, ITGA6, ITGB1 ou ITGB4 ;
et dans laquelle l'échantillon cutané provient d'un donneur choisi dans le groupe constitué des nouveau-nés, dont l'âge est compris entre 0 et 1 mois, des nourrissons, dont l'âge est compris entre 1 mois et 2 ans, et des enfants dont l'âge est compris entre 2 ans et 16 ans, et préférentiellement d'un donneur choisi dans le groupe constitué des nouveau-nés et des nourrissons.

2. Méthode d'évaluation de l'efficacité *in vitro* d'un actif cosmétique, d'un émollient ou d'une formulation dans la prévention ou la réduction des effets de la dermatite atopique de la peau d'enfant, ladite méthode comprenant la détermination de l'efficacité dudit actif cosmétique, dudit émollient ou de ladite formulation sur ladite peau atopique d'enfant dans chacun des quatre tests A, B, C et D, ladite méthode étant **caractérisée en ce que** :
• le test A comprend les étapes suivantes :
a) obtenir un modèle de peau reconstruite à partir d'un échantillon cutané provenant d'un enfant ;
b) mettre en contact le modèle de peau reconstruite de l'étape a) avec une solution comprenant de l'acide poly(déoxyinosinique-déoxycytidylique) (poly(dldC))et de l'interleukine 1 alpha (IL1α) ;
c) mettre en contact ledit actif cosmétique, ledit émollient ou ladite formulation avec le modèle de peau reconstruite de l'étape b) ;
d) mesurer le niveau d'expression d'au moins un marqueur biologique dans le modèle de peau de l'étape c) ; et
e) évaluer l'efficacité dudit actif cosmétique, dudit émollient ou de ladite formulation en fonction du niveau de l'étape d) ;
• le test B comprend les étapes suivantes :
a) obtenir un modèle de peau reconstruite à partir d'un échantillon cutané provenant d'un enfant ;
b) mettre en contact le modèle de peau reconstruite de l'étape a) avec une solution comprenant au moins deux cytokines Th2 ;
c) mettre en contact ledit actif cosmétique, ledit émollient ou ladite formulation avec le modèle de peau reconstruite de l'étape b) ;
d) mesurer le niveau d'expression d'au moins un marqueur biologique dans le modèle de peau de l'étape c) ; et
e) évaluer l'efficacité dudit actif cosmétique, dudit émollient ou de ladite formulation en fonction du niveau de l'étape d) ;
• le test C comprend les étapes suivantes :
a) obtenir un modèle de peau reconstruite à partir d'un échantillon cutané provenant d'un enfant ;
b) cultiver le modèle de peau reconstruite de l'étape a) en présence de monocytes THP-1 ;
c) induire une altération de la fonction barrière dans le modèle de peau reconstruite de l'étape b) ;
d) mettre en contact le modèle de peau reconstruite de l'étape c) avec au moins une bactérie pathogène ;
e) mettre en contact ledit actif cosmétique, ledit émollient ou ladite formulation avec le modèle de peau reconstruite de l'étape d) ;
f) mesurer le niveau d'expression et/ou d'activation d'au moins un marqueur biologique dans le modèle de peau de l'étape e) ; et
g) évaluer l'efficacité dudit actif cosmétique, dudit émollient ou de ladite formulation en fonction du niveau de l'étape f) ; et
• le test D comprend les étapes suivantes :
a) obtenir un modèle de peau reconstruite à partir d'un échantillon cutané provenant d'un enfant ;
b) induire une altération de la fonction barrière dans le modèle de peau reconstruite de l'étape a) ;
c) mettre en contact le modèle de peau reconstruite de l'étape b) avec au moins une bactérie pathogène ;
d) mettre en contact ledit actif cosmétique, ledit émollient ou ladite formulation avec le modèle de peau reconstruite de l'étape c) ;
e) mesurer le niveau d'expression et/ou d'activation d'au moins un marqueur biologique dans le modèle de peau de l'étape d) ; et
f) évaluer l'efficacité dudit actif cosmétique, dudit émollient ou de ladite formulation en fonction du niveau de l'étape e) ;
dans laquelle ledit marqueur est choisi parmi :
• les marqueurs des activités inhibitrices de la physiologie bactérienne, dans lesquels l'activité inhibitrice de de la physiologie bactérienne est choisie parmi l'inhibition de la prolifération bactérienne et l'inhibition de la formation du biofilm ;
• les marqueurs de l'immunité, ledit marqueur de l'immunité étant HBD2 ou TLR2 ;
• les marqueurs de l'inflammation, ledit marqueur de l'inflammation étant CCL2, CXCL1, CCL7, IL6, IL18, CCL3, CCL5, CCL7, KLK5 ou TSLP ;
• les marqueurs de la fonction barrière, ledit marqueur de la fonction barrière étant FLG, KRT1, KRT10, SCEL, BARX2, LOR, IVL, TGM1,DSG1, CDSN, CLDN1, CASP14, SMPD1, GBA, LASS6, NMF ou les céramides ; et
• les marqueurs préférentiellement exprimés dans les cellules souches, ledit marqueur préférentiellement exprimé dans les cellules souches étant KRT15, KRT19, NOTCH1, BIRC5, ITGA6, ITGB1 ou ITGB4 ;
et dans laquelle l'échantillon cutané provient d'un donneur choisi dans le groupe constitué des nouveau-nés, dont l'âge est compris entre 0 et 1 mois, des nourrissons, dont l'âge est compris entre 1 mois et 2 ans, et des enfants dont l'âge est compris entre 2 ans et 16 ans, et préférentiellement d'un donneur choisi dans le groupe constitué des nouveau-nés et des nourrissons.

3. Méthode d'évaluation de la tolérance d'un actif cosmétique, d'un émollient ou d'une formulation par la peau atopique d'enfant, ladite méthode comprenant la détermination de la tolérance dudit actif cosmétique, dudit émollient ou de ladite formulation par ladite peau atopique d'enfant dans chacun des quatre tests A, B, C et D, ladite méthode étant **caractérisée en ce que** :
• le test A comprend les étapes suivantes :
a) obtenir un modèle de peau reconstruite à partir d'un échantillon cutané provenant d'un enfant ;
b) mettre en contact ledit actif cosmétique, ledit émollient ou ladite formulation avec le modèle de peau reconstruite de l'étape a) ;
c) mettre en contact le modèle de peau reconstruite de l'étape b) avec une solution comprenant de acide poly(déoxyinosinique-déoxycytidylique) et de l'interleukine 1 alpha (IL1α) ;
d) mesurer le niveau d'expression d'au moins un marqueur biologique dans le modèle de peau de l'étape c) ; et
e) déterminer si ledit actif cosmétique, ledit émollient ou ladite formulation est bien tolérée par la peau atopique d'enfant en fonction du niveau de l'étape d) ;
• le test B comprend les étapes suivantes :
a) obtenir un modèle de peau reconstruite à partir d'un échantillon cutané provenant d'un enfant ;
b) mettre en contact ledit actif cosmétique, ledit émollient ou ladite formulation avec le modèle de peau reconstruite de l'étape a) ;
c) mettre en contact le modèle de peau reconstruite de l'étape b) avec une solution comprenant au moins deux cytokines Th2 ;
d) mesurer le niveau d'expression d'au moins un marqueur biologique dans le modèle de peau de l'étape c) ; et
e) déterminer si ledit actif cosmétique, ledit émollient ou ladite formulation est bien tolérée par la peau atopique d'enfant en fonction du niveau de l'étape d) ;
• le test C comprend les étapes suivantes :
a) obtenir un modèle de peau reconstruite à partir d'un échantillon cutané provenant d'un enfant ;
b) cultiver le modèle de peau reconstruite de l'étape a) en présence de monocytes THP-1 ;
c) mettre en contact ledit actif cosmétique, ledit émollient ou ladite formulation avec le modèle de peau reconstruite de l'étape b) ;
d) induire une altération de la fonction barrière dans le modèle de peau reconstruite de l'étape c) ;
e) mettre en contact le modèle de peau reconstruite de l'étape d) avec au moins une bactérie pathogène ;
f) mesurer le niveau d'expression et/ou d'activation d'au moins un marqueur biologique dans le modèle de peau de l'étape e) ; et
g) déterminer si ledit actif cosmétique, ledit émollient ou ladite formulation est bien tolérée par la peau atopique d'enfant en fonction du niveau de l'étape f) ; et
• le test D comprend les étapes suivantes :
a) obtenir un modèle de peau reconstruite à partir d'un échantillon cutané provenant d'un enfant ;
b) mettre en contact ledit actif cosmétique, ledit émollient ou ladite formulation avec le modèle de peau reconstruite de l'étape a) ;
c) induire une altération de la fonction barrière dans le modèle de peau reconstruite de l'étape b) ;
d) mettre en contact le modèle de peau reconstruite de l'étape c) avec au moins une bactérie pathogène ;
e) mesurer le niveau d'expression et/ou d'activation d'au moins un marqueur biologique dans le modèle de peau de l'étape d) ; et
f) déterminer si ledit actif cosmétique, ledit émollient ou ladite formulation est bien tolérée par la peau atopique d'enfant en fonction du niveau de l'étape e) ;
dans laquelle ledit marqueur est choisi parmi :
• les marqueurs des activités inhibitrices de la physiologie bactérienne, dans lesquels l'activité inhibitrice de de la physiologie bactérienne est choisie parmi l'inhibition de la prolifération bactérienne et l'inhibition de la formation du biofilm ;
• les marqueurs de l'immunité, ledit marqueur de l'immunité étant HBD2 ou TLR2 ;
• les marqueurs de l'inflammation, ledit marqueur de l'inflammation étant CCL2, CXCL1, CCL7, IL6, IL18, CCL3, CCL5, CCL7, KLK5 ou TSLP ;
• les marqueurs de la fonction barrière, ledit marqueur de la fonction barrière étant FLG, KRT1, KRT10, SCEL, BARX2, LOR, IVL, TGM1,DSG1, CDSN, CLDN1, CASP14, SMPD1, GBA, LASS6, NMF ou les céramides ; et
• les marqueurs préférentiellement exprimés dans les cellules souches, ledit marqueur préférentiellement exprimé dans les cellules souches étant KRT15, KRT19, NOTCH1, BIRC5, ITGA6, ITGB1 ou ITGB4 ;
et dans laquelle l'échantillon cutané provient d'un donneur choisi dans le groupe constitué des nouveau-nés, dont l'âge est compris entre 0 et 1 mois, des nourrissons, dont l'âge est compris entre 1 mois et 2 ans, et des enfants dont l'âge est compris entre 2 ans et 16 ans, et préférentiellement d'un donneur choisi dans le groupe constitué des nouveau-nés et des nourrissons.

4. Méthode d'identification d'un actif cosmétique, d'un émollient ou d'une formulation pour la prévention des effets de la dermatite atopique de la peau d'enfant, ladite méthode comprenant la détermination de l'efficacité dudit actif cosmétique, dudit émollient ou de ladite formulation pour la prévention des effets de la dermatite atopique de la peau d'enfant dans chacun des quatre tests A, B, C et D, ladite méthode étant **caractérisée en ce que** :
• le test A comprend les étapes suivantes :
a) obtenir un modèle de peau reconstruite à partir d'un échantillon cutané provenant d'un enfant ;
b) mettre en contact ledit actif cosmétique, ledit émollient ou ladite formulation avec le modèle de peau reconstruite de l'étape a) ;
c) mettre en contact le modèle de peau reconstruite de l'étape b) avec une solution comprenant de acide poly(déoxyinosinique-déoxycytidylique) et de l'interleukine 1 alpha (IL1α) ;
d) mesurer le niveau d'expression d'au moins un marqueur biologique dans le modèle de peau de l'étape c) ; et
e) déterminer si ledit actif cosmétique, ledit émollient ou ladite formulation candidat est un actif cosmétique, un émollient ou une formulation pour la prévention des effets de la dermatite atopique sur la peau d'enfant en fonction du niveau de l'étape d) ;
• le test B comprend les étapes suivantes :
a) obtenir un modèle de peau reconstruite à partir d'un échantillon cutané provenant d'un enfant ;
b) mettre en contact ledit actif cosmétique, ledit émollient ou ladite formulation avec le modèle de peau reconstruite de l'étape a) ;
c) mettre en contact le modèle de peau reconstruite de l'étape b) avec une solution comprenant au moins deux cytokines Th2 ;
d) mesurer le niveau d'expression d'au moins un marqueur biologique dans le modèle de peau de l'étape c) ; et
e) déterminer si ledit actif cosmétique, ledit émollient ou ladite formulation candidat est un actif cosmétique, un émollient ou une formulation pour la prévention des effets de la dermatite atopique sur la peau d'enfant en fonction du niveau de l'étape d) ;
• le test C comprend les étapes suivantes :
a) obtenir un modèle de peau reconstruite à partir d'un échantillon cutané provenant d'un enfant ;
b) cultiver le modèle de peau reconstruite de l'étape a) en présence de monocytes THP-1 ;
c) mettre en contact ledit actif cosmétique, ledit émollient ou ladite formulation avec le modèle de peau reconstruite de l'étape b) ;
d) induire une altération de la fonction barrière dans le modèle de peau reconstruite de l'étape c) ;
e) mettre en contact le modèle de peau reconstruite de l'étape d) avec au moins une bactérie pathogène ;
f) mesurer le niveau d'expression et/ou d'activation d'au moins un marqueur biologique dans le modèle de peau de l'étape e) ; et
g) déterminer si ledit actif cosmétique, ledit émollient ou ladite formulation candidat est un actif cosmétique, un émollient ou une formulation pour la prévention des effets de la dermatite atopique sur la peau d'enfant en fonction du niveau de l'étape f) ; et
• le test D comprend les étapes suivantes :
a) obtenir un modèle de peau reconstruite à partir d'un échantillon cutané provenant d'un enfant ;
b) mettre en contact ledit actif cosmétique, ledit émollient ou ladite formulation avec le modèle de peau reconstruite de l'étape a) ;
c) induire une altération de la fonction barrière dans le modèle de peau reconstruite de l'étape b) ;
d) mettre en contact le modèle de peau reconstruite de l'étape c) avec au moins une bactérie pathogène ;
e) mesurer le niveau d'expression et/ou d'activation d'au moins un marqueur biologique dans le modèle de peau de l'étape d) ; et
f) déterminer si ledit actif cosmétique, ledit émollient ou ladite formulation candidat est un actif cosmétique, un émollient ou une formulation pour la prévention des effets de la dermatite atopique sur la peau d'enfant en fonction du niveau de l'étape e) ;
dans laquelle ledit marqueur est choisi parmi :
• les marqueurs des activités inhibitrices de la physiologie bactérienne, dans lesquels l'activité inhibitrice de de la physiologie bactérienne est choisie parmi l'inhibition de la prolifération bactérienne et l'inhibition de la formation du biofilm ;
• les marqueurs de l'immunité, ledit marqueur de l'immunité étant HBD2 ou TLR2 ;
• les marqueurs de l'inflammation, ledit marqueur de l'inflammation étant CCL2, CXCL1, CCL7, IL6, IL18, CCL3, CCL5, CCL7, KLK5 ou TSLP ;
• les marqueurs de la fonction barrière, ledit marqueur de la fonction barrière étant FLG, KRT1, KRT10, SCEL, BARX2, LOR, IVL, TGM1,DSG1, CDSN, CLDN1, CASP14, SMPD1, GBA, LASS6, NMF ou les céramides ; et
• les marqueurs préférentiellement exprimés dans les cellules souches, ledit marqueur préférentiellement exprimé dans les cellules souches étant KRT15, KRT19, NOTCH1, BIRC5, ITGA6, ITGB1 ou ITGB4 ;
et dans laquelle l'échantillon cutané provient d'un donneur choisi dans le groupe constitué des nouveau-nés, dont l'âge est compris entre 0 et 1 mois, des nourrissons, dont l'âge est compris entre 1 mois et 2 ans, et des enfants dont l'âge est compris entre 2 ans et 16 ans, et préférentiellement d'un donneur choisi dans le groupe constitué des nouveau-nés et des nourrissons.

5. Méthode d'identification d'un actif cosmétique, d'un émollient ou d'une formulation pour la réduction des effets de la dermatite atopique affectant la peau d'enfant, ladite méthode comprenant la détermination de l'efficacité dudit actif cosmétique, dudit émollient ou de ladite formulation pour la réduction des effets de la dermatite atopique de la peau d'enfant dans chacun des quatre tests A, B, C et D, ladite méthode étant **caractérisée en ce que** :
• le test A comprend les étapes suivantes :
a) obtenir un modèle de peau reconstruite à partir d'un échantillon cutané provenant d'un enfant ;
b) mettre en contact le modèle de peau reconstruite de l'étape a) avec une solution comprenant de acide poly(déoxyinosinique-déoxycytidylique) et de l'interleukine 1 alpha (IL1α) ;
c) mettre en contact ledit actif cosmétique, ledit émollient ou ladite formulation avec le modèle de peau reconstruite de l'étape b) ;
d) mesurer le niveau d'expression d'au moins un marqueur biologique dans le modèle de peau de l'étape c) ; et
e) déterminer si ledit actif cosmétique, ledit émollient ou ladite formulation candidat est un actif cosmétique, un émollient ou une formulation pour la réduction des effets de la dermatite atopique sur la peau d'enfant en fonction du niveau de l'étape d) ;
• le test B comprend les étapes suivantes :
a) obtenir un modèle de peau reconstruite à partir d'un échantillon cutané provenant d'un enfant ;
b) mettre en contact le modèle de peau reconstruite de l'étape a) avec une solution comprenant au moins deux cytokines Th2 ;
c) mettre en contact ledit actif cosmétique, ledit émollient ou ladite formulation avec le modèle de peau reconstruite de l'étape b) ;
d) mesurer le niveau d'expression d'au moins un marqueur biologique dans le modèle de peau de l'étape c) ; et
e) déterminer si ledit actif cosmétique, ledit émollient ou ladite formulation candidat est un actif cosmétique, un émollient ou une formulation pour la réduction des effets de la dermatite atopique sur la peau d'enfant en fonction du niveau de l'étape d) ;
• le test C comprend les étapes suivantes :
a) obtenir un modèle de peau reconstruite à partir d'un échantillon cutané provenant d'un enfant ;
b) cultiver le modèle de peau reconstruite de l'étape a) en présence de monocytes THP-1 ;
c) induire une altération de la fonction barrière dans le modèle de peau reconstruite de l'étape b) ;
d) mettre en contact le modèle de peau reconstruite de l'étape c) avec au moins une bactérie pathogène ;
e) mettre en contact ledit actif cosmétique, ledit émollient ou ladite formulation avec le modèle de peau reconstruite de l'étape d) ;
f) mesurer le niveau d'expression et/ou d'activation d'au moins un marqueur biologique dans le modèle de peau de l'étape e) ; et
g) déterminer si ledit actif cosmétique, ledit émollient ou ladite formulation candidat est un actif cosmétique, un émollient ou une formulation pour la réduction des effets de la dermatite atopique sur la peau d'enfant en fonction du niveau de l'étape f) ; et
• le test D comprend les étapes suivantes :
a) obtenir un modèle de peau reconstruite à partir d'un échantillon cutané provenant d'un enfant ;
b) induire une altération de la fonction barrière dans le modèle de peau reconstruite de l'étape a) ;
c) mettre en contact le modèle de peau reconstruite de l'étape b) avec au moins une bactérie pathogène ;
d) mettre en contact ledit actif cosmétique, ledit émollient ou ladite formulation avec le modèle de peau reconstruite de l'étape c) ;
e) mesurer le niveau d'expression et/ou d'activation d'au moins un marqueur biologique dans le modèle de peau de l'étape d) ; et
f) déterminer si ledit actif cosmétique, ledit émollient ou ladite formulation candidat est un actif cosmétique, un émollient ou une formulation pour la réduction des effets de la dermatite atopique sur la peau d'enfant en fonction du niveau de l'étape e) ;
dans laquelle ledit marqueur est choisi parmi :
• les marqueurs des activités inhibitrices de la physiologie bactérienne, dans lesquels l'activité inhibitrice de de la physiologie bactérienne est choisie parmi l'inhibition de la prolifération bactérienne et l'inhibition de la formation du biofilm ;
• les marqueurs de l'immunité, ledit marqueur de l'immunité étant HBD2 ou TLR2 ;
• les marqueurs de l'inflammation, ledit marqueur de l'inflammation étant CCL2, CXCL1, CCL7, IL6, IL18, CCL3, CCL5, CCL7, KLK5 ou TSLP ;
• les marqueurs de la fonction barrière, ledit marqueur de la fonction barrière étant FLG, KRT1, KRT10, SCEL, BARX2, LOR, IVL, TGM1,DSG1, CDSN, CLDN1, CASP14, SMPD1, GBA, LASS6, NMF ou les céramides ; et
• les marqueurs préférentiellement exprimés dans les cellules souches, ledit marqueur préférentiellement exprimé dans les cellules souches étant KRT15, KRT19, NOTCH1, BIRC5, ITGA6, ITGB1 ou ITGB4 ;
et dans laquelle l'échantillon cutané provient d'un donneur choisi dans le groupe constitué des nouveau-nés, dont l'âge est compris entre 0 et 1 mois, des nourrissons, dont l'âge est compris entre 1 mois et 2 ans, et des enfants dont l'âge est compris entre 2 ans et 16 ans, et préférentiellement d'un donneur choisi dans le groupe constitué des nouveau-nés et des nourrissons.

6. Méthode selon l'une quelconque des revendications 1 à 5, **caractérisée en ce que** les au moins deux cytokines Th2 sont choisies dans le groupe constitué par IL-4, IL-5, IL-10, IL-13, IL-22, IL-31, TSLP1 et TNFα.

7. Méthode selon l'une quelconque des revendications 1 à 6, **caractérisée en ce que** la bactérie pathogène est *Staphylococcus aureus.*

8. Méthode selon l'une quelconque des revendications 1 à 7, **caractérisée en ce que** l'étape d) des tests A et B, l'étape f) du test C, et l'étape e) du test D comprennent la mesure d'une combinaison de marqueurs biologiques, ladite combinaison comprenant au moins deux marqueurs, lesdits marqueurs étant choisis dans au moins deux catégories différentes de marqueurs telles que définies dans les revendications 1 à 5.

9. Méthode selon l'une quelconque des revendications 1 à 8, **caractérisée en ce que** l'échantillon cutané provient d'un donneur choisi dans le groupe constitué des nouveau-nés et des nourrissons.

10. Méthode selon l'une quelconque des revendications 1 à 9, **caractérisée en ce que** l'échantillon cutané provient d'une peau présentant un phototype I, II, III, IV, V ou VI.

11. Méthode selon l'une quelconque des revendications 1 à 10, **caractérisée en ce que** le modèle de peau reconstruite est choisi parmi les cultures de cellules cutanées en suspension, les cultures de cellules cutanées en monocouche, les cultures de cellules cutanées en bicouche, les cultures de peaux reconstruites et les cultures de muqueuses reconstruites.

12. Méthode selon la revendication 11, **caractérisée en ce que** les cellules dudit modèle proviennent d'un explant de tissu cutané ou de cellules souches différenciées en cellules cutanées.

13. Méthode selon l'une quelconque des revendications 1 à 12, **caractérisée en ce que** ledit modèle comprend au moins des fibroblastes ou des kératinocytes.

## Patentansprüche

1. Methode zur Bewertung der Wirksamkeit *in vitro* eines kosmetischen Wirkstoffs, eines Erweichungsmittels oder einer Formulierung zur Vorbeugung oder Behandlung der Auswirkungen der atopischen Dermatitis, die die Kinderhaut schädigt, wobei die Methode die Bestimmung der Wirksamkeit des kosmetischen Wirkstoffs, des Erweichungsmittels oder der Formulierung in jedem der vier Tests A, B, C und D umfasst und die Methode **dadurch gekennzeichnet ist, dass**:
• der Test A die folgenden Schritte umfasst:
a) Erhalten eines rekonstruierten Hautmodells aus einer Hautprobe eines Kindes;
b) Inkontaktversetzen des kosmetischen Wirkstoffs, des Erweichungsmittels oder der Formulierung mit dem rekonstruierten Hautmodell von Schritt a);
c) Inkontaktversetzen des rekonstruierten Hautmodells von Schritt b) mit einer Lösung, die Poly(deoxyinosine-deoxycytidyl)-Säure und Interleukin 1 Alpha (IL1α) umfasst;
d) Messen des Expressionsniveaus mindestens eines biologischen Markers in dem Hautmodell von Schritt c); und
e) Bewerten der Wirksamkeit des kosmetischen Wirkstoffs, des Erweichungsmittels oder der Formulierung in Abhängigkeit des Niveaus von Schritt d);
• der Test B die folgenden Schritte umfasst:
a) Erhalten eines rekonstruierten Hautmodells aus einer Hautprobe eines Kindes;
b) Inkontaktversetzen des kosmetischen Wirkstoffs, des Erweichungsmittels oder der Formulierung mit dem rekonstruierten Hautmodell von Schritt a);
c) Inkontaktversetzen des rekonstruierten Hautmodells von Schritt b) mit einer Lösung, die mindestens zwei Zytokine Th2 umfasst;
d) Messen des Expressionsniveaus mindestens eines biologischen Markers in dem Hautmodell von Schritt c); und
e) Bewerten der Wirksamkeit des kosmetischen Wirkstoffs, des Erweichungsmittels oder der Formulierung in Abhängigkeit des Niveaus von Schritt d);
• der Test C die folgenden Schritte umfasst:
a) Erhalten eines rekonstruierten Hautmodells aus einer Hautprobe eines Kindes;
b) Kultivieren des rekonstruierten Hautmodells von Schritt a) in Anwesenheit von Monozyten THP-1;
c) Inkontaktversetzen des kosmetischen Wirkstoffs, des Erweichungsmittels oder der Formulierung mit dem rekonstruierten Hautmodell von Schritt b);
d) Herbeiführen einer Minderung der Barrierefunktion im rekonstruierten Hautmodell von Schritt c);
e) Inkontaktversetzen des rekonstruierten Hautmodells von Schritt d) mit mindestens einer krankmachenden Bakterie;
f) Messen des Expressionsniveaus und/oder der Aktivierung mindestens eines biologischen Markers im Hautmodell von Schritt e); und
g) Bewerten der Wirksamkeit des kosmetischen Wirkstoffs, des Erweichungsmittels oder der Formulierung in Abhängigkeit des Niveaus von Schritt f); und
• der Test D die folgenden Schritte umfasst:
a) Erhalten eines rekonstruierten Hautmodells aus einer Hautprobe eines Kindes;
b) Inkontaktversetzen des kosmetischen Wirkstoffs, des Erweichungsmittels oder der Formulierung mit dem rekonstruierten Hautmodell von Schritt a);
c) Herbeiführen einer Minderung der Barrierefunktion im rekonstruierten Hautmodell von Schritt b);
d) Inkontaktversetzen des rekonstruierten Hautmodells von Schritt c) mit mindestens einer krankmachenden Bakterie;
e) Messen des Expressionsniveaus und/oder der Aktivierung mindestens eines biologischen Markers im Hautmodell von Schritt d); und
f) Bewerten der Wirksamkeit des kosmetischen Wirkstoffs, des Erweichungsmittels oder der Formulierung in Abhängigkeit des Niveaus von Schritt e).
wobei der Marker ausgewählt ist aus:
• den Markern der inhibitorischen Aktivitäten der bakteriellen Physiologie, wobei die inhibitorische Aktivität der bakteriellen Physiologie aus der Inhibition der Weiterverbreitung der Bakterien und der Inhibition der Bildung des Biofilms ausgewählt ist;
• den Markern der Immunität, wobei der Marker der Immunität HBD2 oder TLR2 ist;
• den Entzündungsmarkern, wobei der Entzündungsmarker CCL2, CXCL1, CCL7, IL6, IL18, CCL3, CCL5, CCL7, KLK5 oder TSLP ist,
• den Markern der Barrierefunktion, wobei der Marker der Barrierefunktion FLG, KRT1, KRT10, SCEL, BARX2, LOR, IVL, TGM1, DSG1, CDSN, CLDN1, CASP14, SMPD1, GBA, LASS6, NMF oder die Ceramide sind, und
• den Markern, die vorzugsweise in den Stammzellen exprimiert sind, wobei der vorzugsweise in den Stammzellen exprimierte Marker KRT15, KRT19, NOTCH1, BIRC5, ITGA6, ITGB1 oder ITGB4 ist;
und wobei die Hautprobe von einem Spender stammt, der aus der Gruppe ausgewählt ist, die von den Neugeborenen gebildet ist, deren Alter zwischen 0 und 1 Monat liegt, den Säuglingen, deren Alter zwischen 1 Monat und 2 Jahren liegt, und den Kindern, deren Alter zwischen 2 Jahren und 16 Jahren liegt, und vorzugsweise von einem Spender, der aus der Gruppe ausgewählt ist, die von den Neugeborenen und den Säuglingen gebildet ist.

2. Methode zur Bewertung der Wirksamkeit *in vitro* eines kosmetischen Wirkstoffs, eines Erweichungsmittels oder einer Formulierung zur Vorbeugung oder Reduzierung der Auswirkungen der atopischen Dermatitis der Kinderhaut, wobei die Methode die Bestimmung der Wirksamkeit des kosmetischen Wirkstoffs, des Erweichungsmittels oder der Formulierung auf die atopische Kinderhaut in jedem der vier Tests A, B, C und D umfasst, wobei die Methode **dadurch gekennzeichnet ist, dass**:
• der Test A die folgenden Schritte umfasst:
a) Erhalten eines rekonstruierten Hautmodells aus einer Hautprobe eines Kindes;
b) Inkontaktversetzen des rekonstruierten Hautmodells von Schritt a) mit einer Lösung, die die Poly(deoxyinosine-deoxycytidyl)-Säure und Interleukin 1 Alpha (IL1α) umfasst;
c) Inkontaktversetzen des kosmetischen Wirkstoffs, des Erweichungsmittels oder der Formulierung mit dem rekonstruierten Hautmodell von Schritt b);
d) Messen des Expressionsniveaus mindestens eines biologischen Markers im Hautmodell von Schritt c); und
e) Bewerten der Wirksamkeit des kosmetischen Wirkstoffs, des Erweichungsmittels oder der Formulierung in Abhängigkeit des Niveaus von Schritt d);
• der Test B die folgenden Schritte umfasst:
a) Erhalten eines rekonstruierten Hautmodells aus einer Hautprobe eines Kindes;
b) Inkontaktversetzen des rekonstruierten Hautmodells von Schritt a) mit einer Lösung, die mindestens zwei Zytokine Th2 umfasst;
c) Inkontaktversetzen des kosmetischen Wirkstoffs, des Erweichungsmittels oder der Formulierung mit dem rekonstruierten Hautmodell von Schritt b);
d) Messen des Expressionsniveaus mindestens eines biologischen Markers im Hautmodell von Schritt c); und
e) Bewerten der Wirksamkeit des kosmetischen Wirkstoffs, des Erweichungsmittels oder der Formulierung in Abhängigkeit des Niveaus von Schritt d);
• der Test C die folgenden Schritte umfasst:
a) Erhalten eines rekonstruierten Hautmodells aus einer Hautprobe eines Kindes;
b) Kultivieren des rekonstruierten Hautmodells von Schritt a) in Anwesenheit von Monozyten THP-1;
c) Herbeiführen einer Minderung der Barrierefunktion im rekonstruierten Hautmodell von Schritt b);
d) Inkontaktversetzen des rekonstruierten Hautmodells von Schritt c) mit mindestens einer krankmachenden Bakterie;
e) Inkontaktversetzen des kosmetischen Wirkstoffs, des Erweichungsmittels oder der Formulierung mit dem rekonstruierten Hautmodell von Schritt d);
f) Messen des Expressionsniveaus und/oder der Aktivierung mindestens eines biologischen Markers im Hautmodell von Schritt e); und
g) Bewerten der Wirksamkeit des kosmetischen Wirkstoffs, des Erweichungsmittels oder der Formulierung in Abhängigkeit des Niveaus von Schritt f); und
• der Test D die folgenden Schritte umfasst:
a) Erhalten eines rekonstruierten Hautmodells aus einer Hautprobe eines Kindes;
b) Herbeiführen einer Minderung der Barrierefunktion im rekonstruierten Hautmodell von Schritt a);
c) Inkontaktversetzen des rekonstruierten Hautmodells von Schritt b) mit mindestens einer krankmachenden Bakterie;
d) Inkontaktversetzen des kosmetischen Wirkstoffs, des Erweichungsmittels oder der Formulierung mit dem rekonstruierten Hautmodell von Schritt c);
e) Messen des Expressionsniveaus und/oder der Aktivierung mindestens eines biologischen Markers im Hautmodell von Schritt d); und
f) Bewerten der Wirksamkeit des kosmetischen Wirkstoffs, des Erweichungsmittels oder der Formulierung in Abhängigkeit des Niveaus von Schritt e);
wobei der Marker ausgewählt ist aus:
• den Markern der inhibitorischen Aktivitäten der bakteriellen Physiologie, wobei die inhibitorische Aktivität der bakteriellen Physiologie aus der Inhibition der Weiterverbreitung der Bakterien und der Inhibition der Bildung des Biofilms ausgewählt ist;
• den Markern der Immunität, wobei der Marker der Immunität HBD2 oder TLR2 ist;
• den Entzündungsmarkern, wobei der Entzündungsmarker CCL2, CXCL1, CCL7, IL6, IL18, CCL3, CCL5, CCL7, KLK5 oder TSLP ist,
• den Markern der Barrierefunktion, wobei der Marker der Barrierefunktion FLG, KRT1, KRT10, SCEL, BARX2, LOR, IVL, TGM1, DSG1, CDSN, CLDN1, CASP14, SMPD1, GBA, LASS6, NMF oder die Ceramide sind, und
• den Markern, die vorzugsweise in den Stammzellen exprimiert sind, wobei der vorzugsweise in den Stammzellen exprimierte Marker KRT15, KRT19, NOTCH1, BIRC5, ITGA6, ITGB1 oder ITGB4 ist;
und wobei die Hautprobe von einem Spender stammt, der aus der Gruppe ausgewählt ist, die von den Neugeborenen gebildet ist, deren Alter zwischen 0 und 1 Monat liegt, den Säuglingen, deren Alter zwischen 1 Monat und 2 Jahren liegt, und den Kindern, deren Alter zwischen 2 Jahren und 16 Jahren liegt, und vorzugsweise von einem Spender, der aus der Gruppe ausgewählt ist, die von den Neugeborenen und den Säuglingen gebildet ist.

3. Methode zur Bewertung der Verträglichkeit eines kosmetischen Wirkstoffs, eines Erweichungsmittels oder einer Formulierung durch die atopische Kinderhaut, wobei die Methode die Bestimmung der Verträglichkeit des kosmetischen Wirkstoffs, des Erweichungsmittels oder der Formulierung durch die atopische Kinderhaut in jedem der vier Tests A, B, C und D umfasst, wobei die Methode **dadurch gekennzeichnet ist, dass**:
• der Test A die folgenden Schritte umfasst:
a) Erhalten eines rekonstruierten Hautmodells aus einer Hautprobe eines Kindes;
b) Inkontaktversetzen des kosmetischen Wirkstoffs, des Erweichungsmittels oder der Formulierung mit dem rekonstruierten Hautmodell von Schritt a);
c) Inkontaktversetzen des rekonstruierten Hautmodells von Schritt b) mit einer Lösung, die Poly(deoxyinosine-deoxycytidyl)-Säure und Interleukin 1 Alpha (IL1α) umfasst;
d) Messen des Expressionsniveaus mindestens eines biologischen Markers im Hautmodell von Schritt c); und
e) Bestimmen, ob der kosmetische Wirkstoff, das Erweichungsmittel oder die Formulierung von der atopische Kinderhaut in Abhängigkeit des Niveaus von Schritt d) gut vertragen wird;
• der Test B die folgenden Schritte umfasst:
a) Erhalten eines rekonstruierten Hautmodells aus einer Hautprobe eines Kindes;
b) Inkontaktversetzen des kosmetischen Wirkstoffs, des Erweichungsmittels oder der Formulierung mit dem rekonstruierten Hautmodell von Schritt a);
c) Inkontaktversetzen des rekonstruierten Hautmodells von Schritt b) mit einer Lösung, die mindestens zwei Zytokine Th2 umfasst;
d) Messen des Expressionsniveaus mindestens eines biologischen Markers im Hautmodell von Schritt c); und
e) Bestimmen, ob der kosmetische Wirkstoff, das Erweichungsmittel oder die Formulierung von der atopische Kinderhaut in Abhängigkeit des Niveaus von Schritt d) gut vertragen wird;
• der Test C die folgenden Schritte umfasst:
a) Erhalten eines rekonstruierten Hautmodells aus einer Hautprobe eines Kindes;
b) Kultivieren des rekonstruierten Hautmodells von Schritt a) in Anwesenheit von Monozyten THP-1;
c) Inkontaktversetzen des kosmetischen Wirkstoffs, des Erweichungsmittels oder der Formulierung mit dem rekonstruierten Hautmodell von Schritt b);
d) Herbeiführen einer Minderung der Barrierefunktion im rekonstruierten Hautmodell von Schritt c);
e) Inkontaktversetzen des rekonstruierten Hautmodells von Schritt d) mit mindestens einer krankmachenden Bakterie;
f) Messen des Expressionsniveaus und/oder der Aktivierung mindestens eines biologischen Markers im Hautmodell von Schritt e); und
g) Bestimmen, ob der kosmetische Wirkstoff, das Erweichungsmittel oder die Formulierung von der atopischen Kinderhaut in Abhängigkeit des Niveaus von Schritt f) gut vertragen wird; und
• der Test D die folgenden Schritte umfasst:
a) Erhalten eines rekonstruierten Hautmodells aus einer Hautprobe eines Kindes;
b) Inkontaktversetzen des kosmetischen Wirkstoffs, des Erweichungsmittels oder der Formulierung mit dem rekonstruierten Hautmodell von Schritt a);
c) Herbeiführen einer Minderung der Barrierefunktion im rekonstruierten Hautmodell von Schritt b);
d) Inkontaktversetzen des rekonstruierten Hautmodells von Schritt c) mit mindestens einer krankmachenden Bakterie;
e) Messen des Expressionsniveaus und/oder der Aktivierung mindestens eines biologischen Markers im Hautmodell von Schritt d); und
f) Bestimmen, ob der kosmetische Wirkstoff, das Erweichungsmittel oder die Formulierung von der atopischen Kinderhaut in Abhängigkeit des Niveaus von Schritt e) gut vertragen wird;
wobei der Marker ausgewählt ist aus:
• den Markern der inhibitorischen Aktivitäten der bakteriellen Physiologie, wobei die inhibitorische Aktivität der bakteriellen Physiologie aus der Inhibition der Weiterverbreitung der Bakterien und der Inhibition der Bildung des Biofilms ausgewählt ist;
• den Markern der Immunität, wobei der Marker der Immunität HBD2 oder TLR2 ist;
• den Entzündungsmarkern, wobei der Entzündungsmarker CCL2, CXCL1, CCL7, IL6, IL18, CCL3, CCL5, CCL7, KLK5 oder TSLP ist,
• den Markern der Barrierefunktion, wobei der Marker der Barrierefunktion FLG, KRT1, KRT10, SCEL, BARX2, LOR, IVL, TGM1, DSG1, CDSN, CLDN1, CASP14, SMPD1, GBA, LASS6, NMF oder die Ceramide sind, und
• den Markern, die vorzugsweise in den Stammzellen exprimiert sind, wobei der vorzugsweise in den Stammzellen exprimierte Marker KRT15, KRT19, NOTCH1, BIRC5, ITGA6, ITGB1 oder ITGB4 ist;
und wobei die Hautprobe von einem Spender stammt, der aus der Gruppe ausgewählt ist, die von den Neugeborenen gebildet ist, deren Alter zwischen 0 und 1 Monat liegt, den Säuglingen, deren Alter zwischen 1 Monat und 2 Jahren liegt, und den Kindern, deren Alter zwischen 2 Jahren und 16 Jahren liegt, und vorzugsweise von einem Spender, der aus der Gruppe ausgewählt ist, die von den Neugeborenen und den Säuglingen gebildet ist.

4. Methode zur Identifizierung eines kosmetischen Wirkstoffs, eines Erweichungsmittels oder einer Formulierung zur Vorbeugung gegen die Auswirkungen der atopischen Dermatitis der Kinderhaut, wobei die Methode die Bestimmung der Wirksamkeit des kosmetischen Wirkstoffs, des Erweichungsmittels oder der Formulierung zur Vorbeugung gegen die Auswirkungen der atopischen Dermatitis der Kinderhaut in jedem der vier Tests A, B, C und D umfasst, wobei die Methode **dadurch gekennzeichnet ist, dass**:
• der Test A die folgenden Schritte umfasst:
a) Erhalten eines rekonstruierten Hautmodells aus einer Hautprobe eines Kindes;
b) Inkontaktversetzen des kosmetischen Wirkstoffs, des Erweichungsmittels oder der Formulierung mit dem rekonstruierten Hautmodell von Schritt a);
c) Inkontaktversetzen des rekonstruierten Hautmodells von Schritt b) mit einer Lösung, die Poly(deoxyinosine-deoxycytidyl)-Säure und Interleukin 1 Alpha (IL1α) umfasst;
d) Messen des Expressionsniveaus mindestens eines biologischen Markers im Hautmodell von Schritt c); und
e) Bestimmen, ob der kosmetische Wirkstoff, das Erweichungsmittel oder die Formulierung als Kandidat ein kosmetischer Wirkstoff, ein Erweichungsmittel oder eine Formulierung zur Vorbeugung gegen die Auswirkungen der atopischen Dermatitis auf die Kinderhaut in Abhängigkeit des Niveaus von Schritt d) ist;
• der Test B die folgenden Schritte umfasst:
a) Erhalten eines rekonstruierten Hautmodells aus einer Hautprobe eines Kindes;
b) Inkontaktversetzen des kosmetischen Wirkstoffs, des Erweichungsmittels oder der Formulierung mit dem rekonstruierten Hautmodell von Schritt a);
c) Inkontaktversetzen des rekonstruierten Hautmodells von Schritt b) mit einer Lösung, die mindestens zwei Zytokine Th2 umfasst;
d) Messen des Expressionsniveaus mindestens eines biologischen Markers im Hautmodell von Schritt c); und
e) Bestimmen, ob der kosmetische Wirkstoff, das Erweichungsmittel oder die Formulierung als Kandidat ein kosmetischer Wirkstoff, ein Erweichungsmittel oder eine Formulierung zur Vorbeugung gegen die Auswirkungen der atopischen Dermatitis auf die Kinderhaut in Abhängigkeit des Niveaus von Schritt d) ist;
• der Test C die folgenden Schritte umfasst:
a) Erhalten eines rekonstruierten Hautmodells aus einer Hautprobe eines Kindes;
b) Kultivieren des rekonstruierten Hautmodells von Schritt a) in Anwesenheit von Monozyten THP-1;
c) Inkontaktversetzen des kosmetischen Wirkstoffs, des Erweichungsmittels oder der Formulierung mit dem rekonstruierten Hautmodell von Schritt b);
d) Herbeiführen einer Minderung der Barrierefunktion im rekonstruierten Hautmodell von Schritt c);
e) Inkontaktversetzen des rekonstruierten Hautmodells von Schritt d) mit mindestens einer krankmachenden Bakterie;
f) Messen des Expressionsniveaus und/oder der Aktivierung mindestens eines biologischen Markers im Hautmodell von Schritt e); und
g) Bestimmen, ob der kosmetische Wirkstoff, das Erweichungsmittel oder die Formulierung als Kandidat ein kosmetischer Wirkstoff, ein Erweichungsmittel oder eine Formulierung zur Vorbeugung gegen die Auswirkungen der atopischen Dermatitis auf die Kinderhaut in Abhängigkeit des Niveaus von Schritt f) ist; und
• der Test D die folgenden Schritte umfasst:
a) Erhalten eines rekonstruierten Hautmodells aus einer Hautprobe eines Kindes;
b) Inkontaktversetzen des kosmetischen Wirkstoffs, des Erweichungsmittels oder der Formulierung mit dem rekonstruierten Hautmodell von Schritt a);
c) Herbeiführen einer Minderung der Barrierefunktion im rekonstruierten Hautmodell von Schritt b);
d) Inkontaktversetzen des rekonstruierten Hautmodells von Schritt c) mit mindestens einer krankmachenden Bakterie;
e) Messen des Expressionsniveaus und/oder der Aktivierung mindestens eines biologischen Markers im Hautmodell von Schritt d); und
f) Bestimmen, ob der kosmetische Wirkstoff, das Erweichungsmittel oder die Formulierung als Kandidat ein kosmetischer Wirkstoff, ein Erweichungsmittel oder eine Formulierung zur Vorbeugung gegen die Auswirkungen der atopischen Dermatitis auf die Kinderhaut in Abhängigkeit des Niveaus von Schritt e) ist,
wobei der Marker ausgewählt ist aus:
• den Markern der inhibitorischen Aktivitäten der bakteriellen Physiologie, wobei die inhibitorische Aktivität der bakteriellen Physiologie aus der Inhibition der Weiterverbreitung der Bakterien und der Inhibition der Bildung des Biofilms ausgewählt ist;
• den Markern der Immunität, wobei der Marker der Immunität HBD2 oder TLR2 ist;
• den Entzündungsmarkern, wobei der Entzündungsmarker CCL2, CXCL1, CCL7, IL6, IL18, CCL3, CCL5, CCL7, KLK5 oder TSLP ist,
• den Markern der Barrierefunktion, wobei der Marker der Barrierefunktion FLG, KRT1, KRT10, SCEL, BARX2, LOR, IVL, TGM1, DSG1, CDSN, CLDN1, CASP14, SMPD1, GBA, LASS6, NMF oder die Ceramide sind, und
• den Markern, die vorzugsweise in den Stammzellen exprimiert sind, wobei der vorzugsweise in den Stammzellen exprimierte Marker KRT15, KRT19, NOTCH1, BIRC5, ITGA6, ITGB1 oder ITGB4 ist;
und wobei die Hautprobe von einem Spender stammt, der aus der Gruppe ausgewählt ist, die von den Neugeborenen gebildet ist, deren Alter zwischen 0 und 1 Monat liegt, den Säuglingen, deren Alter zwischen 1 Monat und 2 Jahren liegt, und den Kindern, deren Alter zwischen 2 Jahren und 16 Jahren liegt, und vorzugsweise von einem Spender, der aus der Gruppe ausgewählt ist, die von den Neugeborenen und den Säuglingen gebildet ist.

5. Methode zur Identifizierung eines kosmetischen Wirkstoffs, eines Erweichungsmittels oder einer Formulierung zur Reduzierung der Auswirkungen der atopischen Dermatitis, die die Kinderhaut schädigt, wobei die Methode die Bestimmung der Wirksamkeit des kosmetischen Wirkstoffs, des Erweichungsmittels oder der Formulierung zur Reduzierung der Auswirkungen der atopischen Dermatitis der Kinderhaut in jedem der vier Tests A, B, C und D umfasst, wobei die Methode **dadurch gekennzeichnet ist, dass**:
• der Test A die folgenden Schritte umfasst:
a) Erhalten eines rekonstruierten Hautmodells aus einer Hautprobe eines Kindes;
b) Inkontaktversetzen des rekonstruierten Hautmodells von Schritt a) mit einer Lösung, die Poly(deoxyinosine-deoxycytidyl)-Säure und Interleukin 1 Alpha (IL1α) umfasst;
c) Inkontaktversetzen des kosmetischen Wirkstoffs, des Erweichungsmittels oder der Formulierung mit dem rekonstruierten Hautmodell von Schritt b);
d) Messen des Expressionsniveaus mindestens eines biologischen Markers im Hautmodell von Schritt c); und
e) Bestimmen, ob der kosmetische Wirkstoff, das Erweichungsmittel oder die Formulierung als Kandidat ein kosmetischer Wirkstoff, ein Erweichungsmittel oder eine Formulierung zur Reduzierung der Auswirkungen der atopischen Dermatitis auf die Kinderhaut in Abhängigkeit des Niveaus von Schritt d) ist;
• der Test B die folgenden Schritte umfasst:
a) Erhalten eines rekonstruierten Hautmodells aus einer Hautprobe eines Kindes;
b) Inkontaktversetzen des rekonstruierten Hautmodells von Schritt a) mit einer Lösung, die mindestens zwei Zytokine Th2 umfasst;
c) Inkontaktversetzen des kosmetischen Wirkstoffs, des Erweichungsmittels oder der Formulierung mit dem rekonstruierten Hautmodell von Schritt b);
d) Messen des Expressionsniveaus mindestens eines biologischen Markers im Hautmodell von Schritt c); und
e) Bestimmen, ob der kosmetische Wirkstoff, das Erweichungsmittel oder die Formulierung als Kandidat ein kosmetischer Wirkstoff, ein Erweichungsmittel oder eine Formulierung zur Reduzierung der Auswirkungen der atopischen Dermatitis auf die Kinderhaut in Abhängigkeit des Niveaus von Schritt d) ist;
• der Test C die folgenden Schritte umfasst:
a) Erhalten eines rekonstruierten Hautmodells aus einer Hautprobe eines Kindes;
b) Kultivieren des rekonstruierten Hautmodells von Schritt a) in Anwesenheit von Monozyten THP-1;
c) Herbeiführen einer Minderung der Barrierefunktion im rekonstruierten Hautmodell von Schritt b);
d) Inkontaktversetzen des rekonstruierten Hautmodells von Schritt c) mit mindestens einer krankmachenden Bakterie;
e) Inkontaktversetzen des kosmetischen Wirkstoffs, des Erweichungsmittels oder der Formulierung mit dem rekonstruierten Hautmodell von Schritt d);
f) Messen des Expressionsniveaus und/oder der Aktivierung mindestens eines biologischen Markers im Hautmodell von Schritt e); und
g) Bestimmen, ob der kosmetische Wirkstoff, das Erweichungsmittel oder die Formulierung als Kandidat ein kosmetischer Wirkstoff, ein Erweichungsmittel oder eine Formulierung zur Reduzierung der Auswirkungen der atopischen Dermatitis auf die Kinderhaut in Abhängigkeit des Niveaus von Schritt f) ist; und
• der Test D die folgenden Schritte umfasst:
a) Erhalten eines rekonstruierten Hautmodells aus einer Hautprobe eines Kindes;
b) Herbeiführen einer Minderung der Barrierefunktion im rekonstruierten Hautmodell von Schritt a);
c) Inkontaktversetzen des rekonstruierten Hautmodells von Schritt b) mit mindestens einer krankmachenden Bakterie;
d) Inkontaktversetzen des kosmetischen Wirkstoffs, des Erweichungsmittels oder der Formulierung mit dem rekonstruierten Hautmodell von Schritt c);
e) Messen des Expressionsniveaus und/oder der Aktivierung mindestens eines biologischen Markers im Hautmodell von Schritt d); und
f) Bestimmen, ob der kosmetische Wirkstoff, das Erweichungsmittel oder die Formulierung als Kandidat ein kosmetischer Wirkstoff, ein Erweichungsmittel oder eine Formulierung zur Reduzierung der Auswirkungen der atopischen Dermatitis auf die Kinderhaut in Abhängigkeit des Niveaus von Schritt e) ist;
wobei der Marker ausgewählt ist aus:
• den Markern der inhibitorischen Aktivitäten der bakteriellen Physiologie, wobei die inhibitorische Aktivität der bakteriellen Physiologie aus der Inhibition der Weiterverbreitung der Bakterien und der Inhibition der Bildung des Biofilms ausgewählt ist;
• den Markern der Immunität, wobei der Marker der Immunität HBD2 oder TLR2 ist;
• den Entzündungsmarkern, wobei der Entzündungsmarker CCL2, CXCL1, CCL7, IL6, IL18, CCL3, CCL5, CCL7, KLK5 oder TSLP ist,
• den Markern der Barrierefunktion, wobei der Marker der Barrierefunktion FLG, KRT1, KRT10, SCEL, BARX2, LOR, IVL, TGM1, DSG1, CDSN, CLDN1, CASP14, SMPD1, GBA, LASS6, NMF oder die Ceramide sind, und
• den Markern, die vorzugsweise in den Stammzellen exprimiert sind, wobei der vorzugsweise in den Stammzellen exprimierte Marker KRT15, KRT19, NOTCH1, BIRC5, ITGA6, ITGB1 oder ITGB4 ist;
und wobei die Hautprobe von einem Spender stammt, der aus der Gruppe ausgewählt ist, die von den Neugeborenen gebildet ist, deren Alter zwischen 0 und 1 Monat liegt, den Säuglingen, deren Alter zwischen 1 Monat und 2 Jahren liegt, und den Kindern, deren Alter zwischen 2 Jahren und 16 Jahren liegt, und vorzugsweise von einem Spender, der aus der Gruppe ausgewählt ist, die von den Neugeborenen und den Säuglingen gebildet ist.

6. Methode nach einem der Ansprüche 1 bis 5, **dadurch gekennzeichnet, dass** die mindestens zwei Zytokine Th2 aus der Gruppe ausgewählt sind, die von IL-4, IL-5, IL-10, IL-13, IL-22, IL-31, TSLP1 und TNFa gebildet ist.

7. Methode nach einem der Ansprüche 1 bis 6, **dadurch gekennzeichnet, dass** die krankmachende Bakterie *Staphylococcus aureus* ist.

8. Methode nach einem der Ansprüche 1 bis 7, **dadurch gekennzeichnet, dass** der Schritt d) der Tests A und B, der Schritt f) des Tests C und der Schritt e) des Tests D das Messen einer Kombination biologischer Marker umfasst, wobei die Kombination mindestens zwei Marker umfasst, wobei die Marker aus mindestens zwei unterschiedlichen Markerkategorien wie in den Ansprüchen 1 bis 5 definiert ausgewählt sind.

9. Methode nach einem der Ansprüche 1 bis 8, **dadurch gekennzeichnet, dass** die Hautprobe von einem Spender stammt, der aus der Gruppe ausgewählt ist, die von den Neugeborenen und den Säuglingen gebildet ist.

10. Methode nach einem der Ansprüche 1 bis 9, **dadurch gekennzeichnet, dass** die Hautprobe von einer Haut stammt, die einen Phototyp I, II, III, IV, V oder VI aufweist.

11. Methode nach einem der Ansprüche 1 bis 10, **dadurch gekennzeichnet, dass** das rekonstruierte Hautmodell aus den Hautzellenkulturen in Suspension, den einschichtigen Hautzellenkulturen, den zweischichtigen Hautzellenkulturen, den Kulturen rekonstruierter Haut und den Kulturen rekonstruierter Schleimhaut ausgewählt ist.

12. Methode nach Anspruch 11, **dadurch gekennzeichnet, dass** die Zellen des Modells von einem Hautgewebeexplantat oder von in Hautzellen differenzierten Stammzellen stammen.

13. Methode nach einem der Ansprüche 1 bis 12, **dadurch gekennzeichnet, dass** das Modell mindestens Fibroblasten oder Keratinozyten umfasst.

## Claims

1. A method for assessing the *in vitro* efficacy of a cosmetic active agent, an emollient or a formulation to prevent or treat the effects of atopic dermatitis affecting the skin of a child, said method comprising the determination of the efficacy of said cosmetic active agent, said emollient or said formulation in each of four tests A, B, C and D and said method being **characterized in that**:
• test A comprises the following steps:
a) obtaining a reconstructed skin model from a skin sample originating from a child;
b) contacting said cosmetic active agent, said emollient or said formulation with the reconstructed skin model of step a);
c) contacting the reconstructed skin model of step b) with a solution comprising poly(deoxyinosinic-deoxycytidylic) acid and interleukin 1 alpha (IL1α);
d) measuring the expression level of at least one biological marker in the skin model of step c); and
e) assessing the efficacy of said cosmetic active agent, said emollient or said formulation as a function of the level of step d);
• test B comprises the following steps:
a) obtaining a reconstructed skin model from a skin sample originating from a child;
b) contacting said cosmetic active agent, said emollient or said formulation with the reconstructed skin model of step a);
c) contacting the reconstructed skin model of step b) with a solution comprising at least two Th2 cytokines;
d) measuring the expression level of at least one biological marker in the skin model of step c); and
e) assessing the efficacy of said cosmetic active agent, said emollient or said formulation as a function of the level of step d);
• test C comprises the following steps:
a) obtaining a reconstructed skin model from a skin sample originating from a child;
b) culturing the reconstructed skin model of step a) in the presence of THP-1 monocytes;
c) contacting said cosmetic active agent, said emollient or said formulation with the reconstructed skin model of step b);
d) inducing an alteration in the barrier function of the reconstructed skin model of step c);
e) contacting the reconstructed skin model of step d) with at least one pathogenic bacterium;
f) measuring the expression and/or activation level of at least one biological marker in the skin model of step e); and
g) assessing the efficacy of said cosmetic active agent, said emollient or said formulation as a function of the level of step f); and
• test D comprises the following steps:
a) obtaining a reconstructed skin model from a skin sample originating from a child;
b) contacting said cosmetic active agent, said emollient or said formulation with the reconstructed skin model of step a);
c) inducing an alteration in the barrier function of the reconstructed skin model of step b);
d) contacting the reconstructed skin model of step c) with at least one pathogenic bacterium;
e) measuring the expression and/or activation level of at least one biological marker in the skin model of step d); and
f) assessing the efficacy of said cosmetic active agent, said emollient or said formulation as a function of the level of step e);
wherein said marker is chosen from:
• markers of activities inhibiting bacterial physiology, wherein the bacterial physiological inhibiting activity is chosen from inhibiting bacterial proliferation and inhibiting biofilm formation;
• immunity markers, said immunity marker being HBD2 or TLR2;
• inflammation markers, said inflammation marker being CCL2, CXCL1, CCL7, IL6, IL18, CCL3, CCL5, CCL7, KLK5 or TSLP;
• barrier function markers, said barrier function marker being FLG, KRT1, KRT10, SCEL, BARX2, LOR, IVL, TGM1,DSG1, CDSN, CLDN1, CASP14, SMPD1, GBA, LASS6, NMF or ceramides; and
• markers preferentially expressed in stem cells, said marker preferentially expressed in stem cells being KRT15, KRT19, NOTCH1, BIRC5, ITGA6, ITGB1 or ITGB4;
and wherein the skin sample originates from a donor selected from the group consisting of neonates, whose age is comprised between 0 and 1 month, infants, whose age is comprised between 1 month and 2 years, and children, whose age is comprised between 2 years and 16 years, and preferentially from a donor selected from the group consisting of neonates and infants.

2. A method for assessing the in vitro efficacy of a cosmetic active agent, an emollient or a formulation to prevent or to reduce the effects of child skin atopic dermatitis, said method comprising the determination of the efficacy of said cosmetic active agent, said emollient or said formulation on said child atopic skin in each of four tests A, B, C and D, said method being **characterized in that**:
• test A comprises the following steps:
a) obtaining a reconstructed skin model from a skin sample originating from a child;
b) contacting the reconstructed skin model of step a) with a solution comprising poly(deoxyinosinic-deoxycytidylic) acid and interleukin 1 alpha (IL1α);
c) contacting said cosmetic active agent, said emollient or said formulation with the reconstructed skin model of step b);
d) measuring the expression level of at least one biological marker in the skin model of step c); and
e) assessing the efficacy of said cosmetic active agent, said emollient or said formulation as a function of the level of step d);
• test B comprises the following steps:
a) obtaining a reconstructed skin model from a skin sample originating from a child;
b) contacting the reconstructed skin model of step a) with a solution comprising at least two Th2 cytokines;
c) contacting said cosmetic active agent, said emollient or said formulation with the reconstructed skin model of step b);
d) measuring the expression level of at least one biological marker in the skin model of step c); and
e) assessing the efficacy of said cosmetic active agent, said emollient or said formulation as a function of the level of step d);
• test C comprises the following steps:
a) obtaining a reconstructed skin model from a skin sample originating from a child;
b) culturing the reconstructed skin model of step a) in the presence of THP-1 monocytes;
c) inducing an alteration in the barrier function of the reconstructed skin model of step b);
d) contacting the reconstructed skin model of step c) with at least one pathogenic bacterium;
e) contacting said cosmetic active agent, said emollient or said formulation with the reconstructed skin model of step d);
f) measuring the expression and/or activation level of at least one biological marker in the skin model of step e); and
g) assessing the efficacy of said cosmetic active agent, said emollient or said formulation as a function of the level of step f); and
• test D comprises the following steps:
a) obtaining a reconstructed skin model from a skin sample originating from a child;
b) inducing an alteration in the barrier function of the reconstructed skin model of step a);
c) contacting the reconstructed skin model of step b) with at least one pathogenic bacterium;
d) contacting said cosmetic active agent, said emollient or said formulation with the reconstructed skin model of step c);
e) measuring the expression and/or activation level of at least one biological marker in the skin model of step d); and
f) assessing the efficacy of said cosmetic active agent, said emollient or said formulation as a function of the level of step e);
wherein said marker is chosen from:
• markers of activities inhibiting bacterial physiology, wherein the bacterial physiological inhibiting activity is chosen from inhibiting bacterial proliferation and inhibiting biofilm formation;
• immunity markers, said immunity marker being HBD2 or TLR2;
• inflammation markers, said inflammation marker being CCL2, CXCL1, CCL7, IL6, IL18, CCL3, CCL5, CCL7, KLK5 or TSLP;
• barrier function markers, said barrier function marker being FLG, KRT1, KRT10, SCEL, BARX2, LOR, IVL, TGM1,DSG1, CDSN, CLDN1, CASP14, SMPD1, GBA, LASS6, NMF or ceramides; and
• markers preferentially expressed in stem cells, said marker preferentially expressed in stem cells being KRT15, KRT19, NOTCH1, BIRC5, ITGA6, ITGB1 or ITGB4;
and wherein the skin sample originates from a donor selected from the group consisting of neonates, whose age is comprised between 0 and 1 month, infants, whose age is comprised between 1 month and 2 years, and children, whose age is comprised between 2 years and 16 years, and preferentially from a donor selected from the group consisting of neonates and infants.

3. Method for assessing the tolerance of a cosmetic active agent, an emollient or a formulation by child atopic skin, said method comprising the determination of the tolerance of said cosmetic active agent, said emollient or said formulation by said child atopic skin in each of four tests A, B, C and D, said method being **characterized in that**:
• test A comprises the following steps:
a) obtaining a reconstructed skin model from a skin sample originating from a child;
b) contacting said cosmetic active agent, said emollient or said formulation with the reconstructed skin model of step a);
c) contacting the reconstructed skin model of step b) with a solution comprising poly(deoxyinosinic-deoxycytidylic) acid and interleukin 1 alpha (IL1α);
d) measuring the expression level of at least one biological marker in the skin model of step c); and
e) determining if said cosmetic active agent, said emollient or said formulation is well tolerated by the atopic skin of a child as a function of the level of step d);
• test B comprises the following steps:
a) obtaining a reconstructed skin model from a skin sample originating from a child;
b) contacting said cosmetic active agent, said emollient or said formulation with the reconstructed skin model of step a);
c) contacting the reconstructed skin model of step b) with a solution comprising at least two Th2 cytokines;
d) measuring the expression level of at least one biological marker in the skin model of step c); and
e) determining if said cosmetic active agent, said emollient or said formulation is well tolerated by child atopic skin as a function of the level of step d);
• test C comprises the following steps:
a) obtaining a reconstructed skin model from a skin sample originating from a child;
b) culturing the reconstructed skin model of step a) in the presence of THP-1 monocytes;
c) contacting said cosmetic active agent, said emollient or said formulation with the reconstructed skin model of step b);
d) inducing an alteration in the barrier function of the reconstructed skin model of step c);
e) contacting the reconstructed skin model of step d) with at least one pathogenic bacterium;
f) measuring the expression and/or activation level of at least one biological marker in the skin model of step e); and
g) determining if said cosmetic active agent, said emollient or said formulation is well tolerated by child atopic skin as a function of the level of step f);
• test D comprises the following steps:
a) obtaining a reconstructed skin model from a skin sample originating from a child;
b) contacting said cosmetic active agent, said emollient or said formulation with the reconstructed skin model of step a);
c) inducing an alteration in the barrier function of the reconstructed skin model of step b);
d) contacting the reconstructed skin model of step c) with at least one pathogenic bacterium;
e) measuring the expression and/or activation level of at least one biological marker in the skin model of step d); and
f) determining if said cosmetic active agent, said emollient or said formulation is well tolerated by the atopic skin of a child as a function of the level of step e);
wherein said marker is chosen from:
• markers of activities inhibiting bacterial physiology, wherein the bacterial physiological inhibiting activity is chosen from inhibiting bacterial proliferation and inhibiting biofilm formation;
• immunity markers, said immunity marker being HBD2 or TLR2;
• inflammation markers, said inflammation marker being CCL2, CXCL1, CCL7, IL6, IL18, CCL3, CCL5, CCL7, KLK5 or TSLP;
• barrier function markers, said barrier function marker being FLG, KRT1, KRT10, SCEL, BARX2, LOR, IVL, TGM1,DSG1, CDSN, CLDN1, CASP14, SMPD1, GBA, LASS6, NMF or ceramides; and
• markers preferentially expressed in stem cells, said marker preferentially expressed in stem cells being KRT15, KRT19, NOTCH1, BIRC5, ITGA6, ITGB1 or ITGB4;
and wherein the skin sample originates from a donor selected from the group consisting of neonates, whose age is comprised between 0 and 1 month, infants, whose age is comprised between 1 month and 2 years, and children, whose age is comprised between 2 years and 16 years, and preferentially from a donor selected from the group consisting of neonates and infants.

4. Method for identifying a cosmetic active agent, an emollient or a formulation for the prevention of the effects of atopic dermatitis of child skin, said method comprising the determination of the efficacy of said cosmetic active agent, said emollient or said formulation in each of four tests A, B, C and D, said method, being **characterized in that**:
• test A comprises the following steps:
a) obtaining a reconstructed skin model from a skin sample originating from a child;
b) contacting said cosmetic active agent, said emollient or said formulation with the reconstructed skin model of step a);
c) contacting the reconstructed skin model of step b) with a solution comprising poly(deoxyinosinic-deoxycytidylic) acid and interleukin 1 alpha (IL1α);
d) measuring the expression level of at least one biological marker in the skin model of step c); and
e) determining if said cosmetic active agent, said emollient or said candidate formulation is a cosmetic active agent, an emollient or a formulation for preventing the effects of atopic dermatitis on child skin as a function of the level in step d);
• test B comprises the following steps:
a) obtaining a reconstructed skin model from a skin sample originating from a child;
b) contacting said cosmetic active agent, said emollient or said formulation with the reconstructed skin model of step a);
c) contacting the reconstructed skin model of step b) with a solution comprising at least two Th2 cytokines;
d) measuring the expression level of at least one biological marker in the skin model of step c); and
e) determining if said cosmetic active agent, said emollient or said candidate formulation is a cosmetic active agent, an emollient or a formulation for preventing the effects of atopic dermatitis on the skin of a child as a function of the level in step d);
• test C comprises the following steps:
a) obtaining a reconstructed skin model from a skin sample originating from a child;
b) culturing the reconstructed skin model of step a) in the presence of THP-1 monocytes;
c) contacting said cosmetic active agent, said emollient or said formulation with the reconstructed skin model of step b);
d) inducing an alteration in the barrier function of the reconstructed skin model of step c);
e) contacting the reconstructed skin model of step d) with at least one pathogenic bacterium;
f) measuring the expression and/or activation level of at least one biological marker in the skin model of step e); and
g) determining if said cosmetic active agent, said emollient or said candidate formulation is a cosmetic active agent, an emollient or a formulation for preventing the effects of atopic dermatitis on child skin as a function of the level in step f); and
• test D comprises the following steps:
a) obtaining a reconstructed skin model from a skin sample originating from a child;
b) contacting said cosmetic active agent, said emollient or said formulation with the reconstructed skin model of step a);
c) inducing an alteration in the barrier function of the reconstructed skin model of step b);
d) contacting the reconstructed skin model of step c) with at least one pathogenic bacterium;
e) measuring the expression and/or activation level of at least one biological marker in the skin model of step d); and
f) determining if said cosmetic active agent, said emollient or said candidate formulation is a cosmetic active agent, an emollient or a formulation for preventing the effects of atopic dermatitis on child skin as a function of the level in step e);
wherein said marker is chosen from:
• markers of activities inhibiting bacterial physiology, wherein the bacterial physiological inhibiting activity is chosen from inhibiting bacterial proliferation and inhibiting biofilm formation;
• immunity markers, said immunity marker being HBD2 or TLR2;
• inflammation markers, said inflammation marker being CCL2, CXCL1, CCL7, IL6, IL18, CCL3, CCL5, CCL7, KLK5 or TSLP;
• barrier function markers, said barrier function marker being FLG, KRT1, KRT10, SCEL, BARX2, LOR, IVL, TGM1,DSG1, CDSN, CLDN1, CASP14, SMPD1, GBA, LASS6, NMF or ceramides; and
• markers preferentially expressed in stem cells, said marker preferentially expressed in stem cells being KRT15, KRT19, NOTCH1, BIRC5, ITGA6, ITGB1 or ITGB4;
and wherein the skin sample originates from a donor selected from the group consisting of neonates, whose age is comprised between 0 and 1 month, infants, whose age is comprised between 1 month and 2 years, and children, whose age is comprised between 2 years and 16 years, and preferentially from a donor selected from the group consisting of neonates and infants.

5. Method for identifying a cosmetic active agent, an emollient or a formulation for the reduction of the effects of atopic dermatitis affecting the skin of a child, said method comprising the determination of the efficacy of said cosmetic active agent, said emollient or said formulation for the reduction of the effects of atopic dermatitis on the skin of a child in each of four tests A, B, C and D, said method being **characterized in that**:
• test A comprises the following steps:
a) obtaining a reconstructed skin model from a skin sample originating from a child;
b) contacting the reconstructed skin model of step a) with a solution comprising poly(deoxyinosinic-deoxycytidylic) acid and interleukin 1 alpha (IL1α);
c) contacting said cosmetic active agent, said emollient or said formulation with the reconstructed skin model of step b);
d) measuring the expression level of at least one biological marker in the skin model of step c); and
e) determining if said cosmetic active agent, said emollient or said candidate formulation is a cosmetic active agent, an emollient or a formulation for reducing the effects of atopic dermatitis on child skin as a function of the level in step d);
• test B comprises the following steps:
a) obtaining a reconstructed skin model from a skin sample originating from a child;
b) contacting the reconstructed skin model of step a) with a solution comprising at least two Th2 cytokines;
c) contacting said cosmetic active agent, said emollient or said formulation with the reconstructed skin model of step b);
d) measuring the expression level of at least one biological marker in the skin model of step c); and
e) determining if said cosmetic active agent, said emollient or said candidate formulation is a cosmetic active agent, an emollient or a formulation for reducing the effects of atopic dermatitis on child skin as a function of the level in step d);
• test C comprises the following steps:
a) obtaining a reconstructed skin model from a skin sample originating from a child;
b) culturing the reconstructed skin model of step a) in the presence of THP-1 monocytes;
c) inducing an alteration in the barrier function of the reconstructed skin model of step b);
d) contacting the reconstructed skin model of step c) with at least one pathogenic bacterium;
e) contacting said cosmetic active agent, said emollient or said formulation with the reconstructed skin model of step d);
f) measuring the expression and/or activation level of at least one biological marker in the skin model of step e); and
g) determining if said cosmetic active agent, said emollient or said candidate formulation is a cosmetic active agent, an emollient or a formulation for reducing the effects of atopic dermatitis on child skin as a function of the level in step f); and
• test D comprises the following steps:
a) obtaining a reconstructed skin model from a skin sample originating from a child;
b) inducing an alteration in the barrier function of the reconstructed skin model of step a);
c) contacting the reconstructed skin model of step b) with at least one pathogenic bacterium;
d) contacting said cosmetic active agent, said emollient or said formulation with the reconstructed skin model of step c);
e) measuring the expression and/or activation level of at least one biological marker in the skin model of step d); and
f) determining if said cosmetic active agent, said emollient or said candidate formulation is a cosmetic active agent, an emollient or a formulation for reducing the effects of atopic dermatitis on child skin as a function of the level in step e);
wherein said marker is chosen from:
• markers of activities inhibiting bacterial physiology, wherein the bacterial physiological inhibiting activity is chosen from inhibiting bacterial proliferation and inhibiting biofilm formation;
• immunity markers, said immunity marker being HBD2 or TLR2;
• inflammation markers, said inflammation marker being CCL2, CXCL1, CCL7, IL6, IL18, CCL3, CCL5, CCL7, KLK5 or TSLP;
• barrier function markers, said barrier function marker being FLG, KRT1, KRT10, SCEL, BARX2, LOR, IVL, TGM1,DSG1, CDSN, CLDN1, CASP14, SMPD1, GBA, LASS6, NMF or ceramides; and
• markers preferentially expressed in stem cells, said marker preferentially expressed in stem cells being KRT15, KRT19, NOTCH1, BIRC5, ITGA6, ITGB1 or ITGB4;
and wherein the skin sample originates from a donor selected from the group consisting of neonates, whose age is comprised between 0 and 1 month, infants, whose age is comprised between 1 month and 2 years, and children, whose age is comprised between 2 years and 16 years, and preferentially from a donor selected from the group consisting of neonates and infants.

6. Method according to any one of claims 1 to 5, **characterized in that** the at least two Th2 cytokines are chosen from the group consisting of IL-4, IL-5, IL-10, IL-13, IL-22, IL-31, TSLP1 and TNFα.

7. Method according to any one of claims 1 to 6, **characterized in that** the pathogenic bacterium is *Staphylococcus aureus.*

8. Method according to any one of claims 1 to 7, **characterized in that** step d) of tests A and B, step f) of test C, and step e) of test D comprise the measurement of a combination of biological markers, said combination comprising at least two markers, said markers being chosen in at least two different marker categories, such as defined in claims 1 to 5.

9. Method according to any one of claims 1 to 8, **characterized in that** the skin sample originates from a donor selected from the group consisting of neonates and infants.

10. Method according to any one of claims 1 to 9, **characterized in that** the skin sample originates from skin with a phototype I, II, III, IV, V or VI.

11. Method according to any one of claims 1 to 10, **characterized in that** the reconstructed skin model is chosen from skin cell cultures in suspension, skin cell cultures in a monolayer, skin cell cultures in a bilayer, reconstructed skin cultures and reconstructed mucosa cultures.

12. Method according to claim 11, **characterized in that** the cells of said model originate from a cutaneous tissue explant or from stem cells differentiated into cutaneous cells.

13. Method according to any one of claims 1 to 12, **characterized in that** said model comprises at least fibroblasts or keratinocytes.
